(19) Europäisches Patentamt
European Patent Office
Office européen des brevets

(11) Veröffentlichungsnummer: **0 610 733 A1**

(12) **EUROPÄISCHE PATENTANMELDUNG**

(21) Anmeldenummer: **94101222.1**

(22) Anmeldetag: **27.01.94**

(51) Int. Cl.5: **C07D 249/14**, C07D 401/12, C07D 403/12, A01N 43/653

(30) Priorität: **09.02.93 DE 4303676**

(43) Veröffentlichungstag der Anmeldung: **17.08.94 Patentblatt 94/33**

(84) Benannte Vertragsstaaten: **BE CH DE DK ES FR GB IT LI NL**

(71) Anmelder: **BAYER AG**

**D-51368 Leverkusen (DE)**

(72) Erfinder: **Linker, Karl-Heinz**
**Albert-Schweitzer-Strasse 3**
**D-51377 Leverkusen (DE)**
Erfinder: **Findeisen, Kurt, Prof. Dr.**
**Dünfelder Strasse 28**
**D-51375 Leverkusen (DE)**
Erfinder: **Haas, Wilhelm, Dr.**
**Schürgespfad 19**
**D-50259 Pulheim (DE)**
Erfinder: **Schallner, Otto, Dr.**
**Noldeweg 22**
**D-40789 Monheim (DE)**
Erfinder: **Dollinger, Markus, Dr.**
**Hüschelrath 7**
**D-42799 Leichlingen (DE)**
Erfinder: **Santel, Hans-Joachim, Dr.**
**Grünstrasse 9a**
**D-51371 Leverkusen (DE)**

(54) **1-Aryltriazolin(thi)one.**

(57) Die Erfindung betrifft neue 1-Aryltriazolin(thi)one der allgemeinen Formel (I)

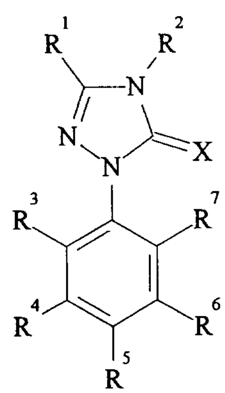

(I)

in welcher

| | |
|---|---|
| $R^1$ | für Nitro oder für einen Rest $-NR^8R^9$ steht, |
| $R^2$ | für Wasserstoff, Amino, Cyano oder für einen der Reste $-R^{10}$, $-O-R^{10}$, $-S-R^{10}$, $-NR^{10}R^{11}$, $-N(R^{11})-CO-R^{10}$ oder $-N=CR^{10}R^{11}$ steht, |
| $R^3$, $R^6$ und $R^7$ | unabhängig voneinander jeweils für Wasserstoff, Halogen oder Nitro stehen, |
| $R^4$ | für Wasserstoff, Halogen, Cyano, Nitro, oder für einen der Reste $-R^{12}$, $-O-R^{12}$, $-S-R^{12}$, $-S(O)-R^{12}$, $-SO_2-R^{12}$, $-SO_2-OR^{12}$, $-SO_2-NR^{11}R^{12}$, $-CO-OR^{12}$, $-CO-NR^{11}R^{12}$, $-O-SO_2-R^{12}$, $-N(R^{11})-SO_2-R^{12}$, $-NR^{11}R^{12}$, $-NH-P(O)(R^{11})(OR^{12})$ oder $-NH-P(O)(OR^{11})(OR^{12})$ steht, |
| $R^5$ | für Nitro, Cyano, Halogen oder Halogenalkyl steht und |

EP 0 610 733 A1

| | |
|---|---|
| X | für Sauerstoff oder Schwefel steht, wobei |
| $R^8$ und $R^9$ | unabhängig voneinander jeweils für Wasserstoff oder für jeweils gegebenenfalls substituiertes Alkyl, Alkenyl, Alkinyl, Alkoxy, Cycloalkyl, Aryl oder Heterocyclyl stehen oder gemeinsam mit dem Stickstoffatom, an welches sie gebunden sind, für einen gegebenenfalls substituierten Heterocyclus stehen, |
| $R^{10}$ | für jeweils gegebenenfalls substituiertes Alkyl, Alkenyl, Alkinyl, Cycloalkyl, Aryl, Arylalkyl oder Heterocyclyl steht, |
| $R^{11}$ | für Wasserstoff oder für jeweils gegebenenfalls substituiertes Alkyl, Alkenyl, Alkinyl, Cycloalkyl, Arylalkyl oder Aryl steht und |
| $R^{12}$ | für Wasserstoff oder für jeweils gegebenenfalls substituiertes Alkyl, Alkenyl, Alkinyl, Cycloalkyl, Aryl oder Heterocyclyl steht, |

mehrere Verfahren zu ihrer Herstellung, mehrere neue Zwischenprodukte, sowie ihre Verwendung als Herbizide.

Die Erfindung betrifft neue 1-Aryltriazolin(thi)one, mehrere Verfahren zu ihrer Herstellung, mehrere neue Zwischenprodukte sowie ihre Verwendung als Herbizide.

Es ist bekannt, daß bestimmte 1-Aryltriazolin(thi)one wie beispielsweise die Verbindung 3-Methyl-4-propargyl-1-(2,5-difluor-4-cyano-phenyl)-1,2,4-triazolin-5-on herbizide Eigenschaften besitzen (vergl. z.B. DE 38 39 480).

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue 1-Aryltriazolin(thi)one der allgemeinen Formel (I),

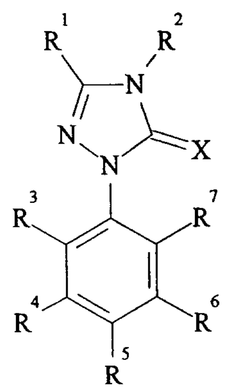

(I)

in welcher

| | |
|---|---|
| $R^1$ | für Nitro oder für einen Rest $-NR^8R^9$ steht, |
| $R^2$ | für Wasserstoff, Amino, Cyano oder für einen der Reste $-R^{10}$, $-O-R^{10}$, $-S-R^{10}$, $-NR^{10}R^{11}$, $-N(R^{11})-CO-R^{10}$ oder $-N=CR^{10}R^{11}$ steht, |
| $R^3$, $R^6$ und $R^7$ | unabhängig voneinander jeweils für Wasserstoff, Halogen oder Nitro stehen, |
| $R^4$ | für Wasserstoff, Halogen, Cyano, Nitro, oder für einen der Reste $-R^{12}$, $-O-R^{12}$, $-S-R^{12}$, $-S(O)-R^{12}$, $-SO_2-R^{12}$, $-SO_2-OR^{12}$, $-SO_2-NR^{11}R^{12}$, $-CO-OR^{12}$, $-CO-NR^{11}R^{12}$, $-O-SO_2-R^{12}$, $-N(R^{11})-SO_2-R^{12}$, $-NR^{11}R^{12}$, $-NH-P(O)(R^{11})(OR^{12})$ oder $-NH-P(O)(OR^{11})(OR^{12})$ steht, |
| $R^5$ | für Nitro, Cyano, Halogen oder Halogenalkyl steht und |
| X | für Sauerstoff oder Schwefel steht, wobei |
| $R^8$ und $R^9$ | unabhängig voneinander jeweils für Wasserstoff oder für jeweils gegebenenfalls substituiertes Alkyl, Alkenyl, Alkinyl, Alkoxy, Cycloalkyl, Aryl oder Heterocyclyl stehen oder gemeinsam mit dem Stickstoffatom, an welches sie gebunden sind, für einen gegebenenfalls substituierten Heterocyclus stehen, |
| $R^{10}$ | für jeweils gegebenenfalls substituiertes Alkyl, Alkenyl, Alkinyl, Cycloalkyl, Aryl, Arylalkyl oder Heterocyclyl steht, |
| $R^{11}$ | für Wasserstoff oder für jeweils gegebenenfalls substituiertes Alkyl, Alkenyl, Alkinyl, Cycloalkyl, Arylalkyl oder Aryl steht und |
| $R^{12}$ | für Wasserstoff oder für jeweils gegebenenfalls substituiertes Alkyl, Alkenyl, Alkinyl, Cycloalkyl, Aryl oder Heterocyclyl steht, |

gefunden.

Die Verbindungen der Formel (I) können gegebenenfalls in Abhängigkeit von der Art der Substituenten als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen 1-Aryltriazolin(thi)one der allgemeinen Formel (I),

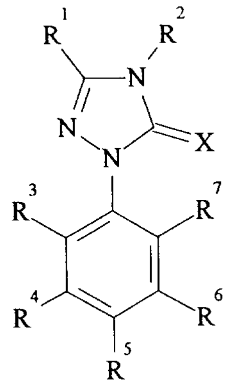

(I)

in welcher

| | |
|---|---|
| $R^1$ | für Nitro oder für einen Rest $-NR^8R^9$ steht, |
| $R^2$ | für Wasserstoff, Amino, Cyano oder für einen der Reste $-R^{10}$, $-O-R^{10}$, $-S-R^{10}$, $-NR^{10}R^{11}$, $-N(R^{11})-CO-R^{10}$ oder $-N=CR^{10}R^{11}$ steht, |
| $R^3$, $R^6$ und $R^7$ | unabhängig voneinander jeweils für Wasserstoff, Halogen oder Nitro stehen, |
| $R^4$ | für Wasserstoff, Halogen, Cyano, Nitro, oder für einen der Reste $-R^{12}$, $-O-R^{12}$, $-S-R^{12}$, $-S(O)-R^{12}$, $-SO_2-R^{12}$, $-SO_2-OR^{12}$, $-SO_2-NR^{11}R^{12}$, $-CO-OR^{12}$, $-CO-NR^{11}R^{12}$, $-O-SO_2-R^{12}$, $-N(R^{11})-SO_2-R^{12}$, $-NR^{11}R^{12}$, $-NH-P(O)(R^{11})(OR^{12})$ oder $-NH-P(O)(OR^{11})(OR^{12})$ steht, |
| $R^5$ | für Nitro, Cyano, Halogen oder Halogenalkyl steht und |
| X | für Sauerstoff oder Schwefel steht, wobei |
| $R^8$ und $R^9$ | unabhängig voneinander jeweils für Wasserstoff oder für jeweils gegebenenfalls substituiertes Alkyl, Alkenyl, Alkinyl, Alkoxy, Cycloalkyl, Aryl oder Heterocyclyl stehen oder gemeinsam mit dem Stickstoffatom, an welches sie gebunden sind, für einen gegebenenfalls substituierten Heterocyclus stehen, |
| $R^{10}$ | für jeweils gegebenenfalls substituiertes Alkyl, Alkenyl, Alkinyl, Cycloalkyl, Aryl, Arylalkyl oder Heterocyclyl steht, |
| $R^{11}$ | für Wasserstoff oder für jeweils gegebenenfalls substituiertes Alkyl, Alkenyl, Alkinyl, Cycloalkyl, Arylalkyl oder Aryl steht und |
| $R^{12}$ | für Wasserstoff oder für jeweils gegebenenfalls substituiertes Alkyl, Alkenyl, Alkinyl, Cycloalkyl, Aryl oder Heterocyclyl steht, |

erhält, wenn man

a) 1H-Triazolinone der Formel (II),

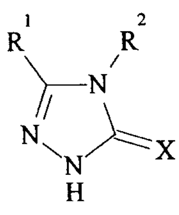

(II)

in welcher

$R^1$, $R^2$ und X die oben angegebenen Bedeutungen haben,

mit Halogenbenzol-Derivaten der Formel (III),

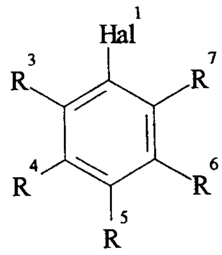

(III)

in welcher

$R^3$, $R^4$, $R^5$, $R^6$ und $R^7$ die oben angegebenen Bedeutungen haben und

$Hal^1$ für Halogen steht,

gegebenenfalls in Gegenwart eines Verdünnungsmittels und gegebenenfalls in Gegenwart eines Reaktionshilfsmittels umsetzt, oder wenn man

b) substituierte Triazolinone der Formel (Ia),

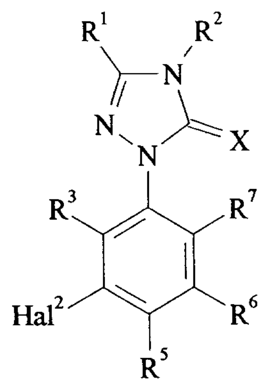

(Ia)

in welcher

$R^1$, $R^2$, $R^3$, $R^5$, $R^6$, $R^7$ und X die oben angegebenen Bedeutungen haben und

$Hal^2$ für Halogen steht,

mit Nukleophilen der Formel (IV),

$H\text{-}R^{13}$ (IV)

in welcher

| | |
|---|---|
| $R^{13}$ | für einen Rest der Formel $-O-R^{12}$, $-S-R^{12}$ oder $-NR^{11}R^{12}$ steht, wobei |
| $R^{11}$ und $R^{12}$ | die oben angegebenen Bedeutungen haben, |

gegebenenfalls in Gegenwart eines Verdünnungsmittels und gegebenenfalls in Gegenwart eines Reaktionshilfsmittels umsetzt, oder wenn man

c) substituierte Triazolinone der Formel (Ib),

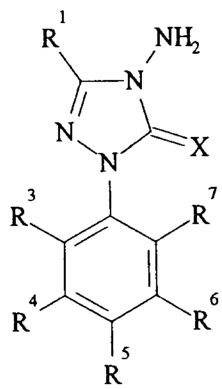

(Ib)

in welcher

$R^1$, $R^3$, $R^4$, $R^5$, $R^6$, $R^7$ und X die oben angegebenen Bedeutungen haben,

mit Natriumnitrit in Gegenwart einer Säure und gegebenenfalls in Gegenwart eines Verdünnungsmittels umsetzt, oder wenn man

d) substituierte Triazolinone der Formel (Ic),

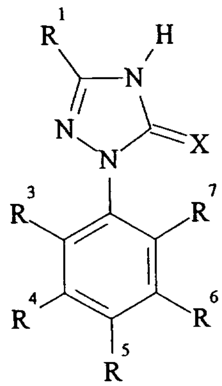

(Ic)

in welcher

$R^1$, $R^3$, $R^4$, $R^5$, $R^6$, $R^7$ und X die oben angegebenen Bedeutungen haben,

mit Alkylierungsmitteln der Formel (V),

$R^{2-1}$-$E^1$ (V)

in welcher

$R^{2-1}$ für jeweils gegebenenfalls substituiertes Alkyl, Alkenyl, Alkinyl oder Cycloalkyl steht und

$E^1$ für eine elektronenanziehende Abgangsgruppe steht,

gegebenenfalls in Gegenwart eines Verdünnungsmittels und gegebenenfalls in Gegenwart eines Reaktionshilfsmittels umsetzt, oder wenn man

d) Chlor-formamidinium-Hydrochloride der Formel (VI),

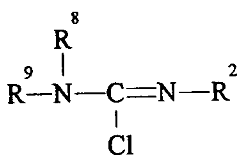

x HCl (VI)

in welcher

$R^2$, $R^8$ und $R^9$ die oben angegebenen Bedeutungen haben,

zunächst in einer ersten Stufe mit Arylhydrazinen der Formel (VII),

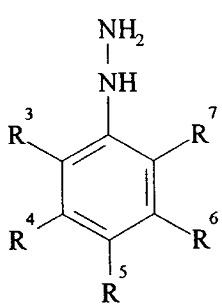

(VII)

in welcher

$R^3$, $R^4$, $R^5$, $R^6$ und $R^7$ die oben angegebenen Bedeutungen haben,

gegebenenfalls in Gegenwart eines Verdünnungsmittels umsetzt und in einer anschließenden zweiten Stufe die so erhältlichen Zwischenprodukte der Formel (VIII),

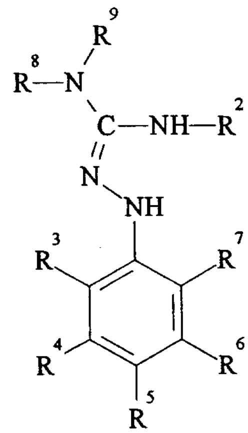

(VIII)

in welcher

$R^2$, $R^3$, $R^4$, $R^5$, $R^6$, $R^7$, $R^8$ und $R^9$ die oben angegebenen Bedeutungen haben,

mit Phosgen oder Thiophosgen gegebenenfalls in Gegenwart eines Verdünnungsmittels und gegebenenfalls in Gegenwart eines Reaktionshilfsmittels cyclisiert.

Schließlich wurde gefunden, daß die neuen 1-Aryltriazolin(thi)one der allgemeinen Formel (I) herbizide Eigenschaften besitzen.

Überraschenderweise zeigen die erfindungsgemäßen 1-Aryltriazolin(thi)one der allgemeinen Formel (I) eine erheblich bessere herbizide Wirksamkeit gegenüber Problemunkräutern bei vergleichbarer Verträglichkeit gegenüber Nutzpflanzen im Vergleich zu den aus dem Stand der Technik bekannten substituierten Triazolinonen, wie beispielsweise die Verbindung 3-Methyl-4-propargyl-1-(2,5-difluor-4-cyano-phenyl)-1,2,4-triazolin-5-on, welche chemisch und wirkungsmäßig naheliegende Verbindungen sind.

Die erfindungsgemäßen 1-Aryltriazolin(thi)one sind durch die Formel (I) allgemein definiert. Bevorzugt sind Verbindungen der Formel (I), bei welchen

$R^1$ für Nitro oder für einen Rest $-NR^8R^9$ steht,

$R^2$ für Wasserstoff, Amino, Cyano oder für einen der Reste $-R^{10}$, $-O-R^{10}$, $-S-R^{10}$, $-NR^{10}R^{11}$, $-N(R^{11})-CO-R^{10}$ oder $-N=CR^{10}R^{11}$ steht,

$R^3$, $R^6$ und $R^7$ unabhängig voneinander jeweils für Wasserstoff, Fluor, Chlor, Brom, Iod oder Nitro stehen,

$R^4$ für Wasserstoff, Fluor, Chlor, Brom, Iod, Cyano, Nitro, oder für einen der Reste $-R^{12}$, $-O-R^{12}$, $-S-R^{12}$, $-S(O)-R^{12}$, $-SO_2-R^{12}$, $-SO_2-OR^{12}$, $-SO_2-NR^{11}R^{12}$, $-CO-OR^{12}$, $-CO-NR^{11}R^{12}$, $-O-SO_2-R^{12}$, $-N(R^{11})-SO_2-R^{12}$, $-NR^{11}R^{12}$, $-NH-P(O)(R^{11})(OR^{12})$ oder $-NH-P(O)-(OR^{11})(OR^{12})$ steht,

$R^5$ für Nitro, Cyano, Fluor, Chlor, Brom, Iod oder für geradkettiges oder verzweigtes Halogenalkyl mit 1 bis 6 Kohlenstoffatomen und 1 bis 13 gleichen oder verschiedenen Halogenatomen steht und

X für Sauerstoff oder Schwefel steht, wobei

$R^8$ und $R^9$ unabhängig voneinander jeweils für Wasserstoff stehen;

$R^8$ und $R^9$ außerdem für jeweils gegebenenfalls einfach oder mehrfach, gleich oder verschieden substituiertes, geradkettiges oder verzweigtes Alkyl oder Alkoxy mit jeweils 1 bis 14 Kohlenstoffatomen stehen, wobei als Substituenten jeweils infrage kommen:
Halogen - insbesondere Fluor, Chlor, Brom und/oder Iod -, Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl oder Alkylsulfonylaminocarbonyl mit jeweils 1 bis 8 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/ oder Schwefel - steht;

$R^8$ und $R^9$ außerdem für jeweils gegebenenfalls einfach oder mehrfach, gleich oder verschieden

durch Halogen - insbesondere Fluor, Chlor, Brom und/oder Iod - substituiertes Alkenyl oder Alkinyl mit jeweils 2 bis 8 Kohlenstoffatomen stehen;

$R^8$ und $R^9$ außerdem für gegebenenfalls einfach oder mehrfach, gleich oder verschieden durch Halogen - insbesondere Fluor, Chlor, Brom und/oder Iod - und/oder geradkettiges oder verzweigtes Alkyl mit 1 bis 4 Kohlenstoffatomen substituiertes Cycloalkyl mit 3 bis 7 Kohlenstoffatomen stehen;

$R^8$ und $R^9$ außerdem für jeweils gegebenenfalls im Arylteil einfach oder mehrfach, gleich oder verschieden substituiertes Arylalkyl oder Aryl mit jeweils 6 bis 10 Kohlenstoffatomen im Arylteil und gegebenenfalls 1 bis 4 Kohlenstoffatomen im geradkettigen oder verzweigten Alkylteil stehen, oder für einen gegebenenfalls einfach oder mehrfach, gleich oder verschieden substituierten und/oder benzannellierten, gesättigten oder ungesättigten, fünf- bis siebengliedrigen Heterocyclylrest mit 1 bis 3 gleichen oder verschiedenen Heteroatomen - insbesondere Stickstoff, Sauerstoff und/oder Schwefel - stehen, wobei als Aryl- bzw. Heterocyclylsubstituenten jeweils infrage kommen:

Halogen, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Alkoxycarbonyl oder Alkoximinoalkyl mit jeweils 1 bis 6 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen substituiertes Phenyl;

$R^8$ und $R^9$ außerdem gemeinsam mit dem Stickstoffatom, an welches sie gebunden sind, für einen gegebenenfalls einfach oder mehrfach, gleich oder verschieden substituierten und/oder benzannellierten, gesättigten oder ungesättigten, fünf- bis siebengliedrigen Heterocyclylrest mit 1 bis 3 gleichen oder verschiedenen Heteroatomen - insbesondere Stickstoff, Sauerstoff und/oder Schwefel - stehen, wobei als Heterocyclylsubstituenten infrage kommen:

Halogen, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Alkoxycarbonyl oder Alkoximinoalkyl mit jeweils 1 bis 6 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen substituiertes Phenyl;

$R^{10}$ für gegebenenfalls einfach oder mehrfach, gleich oder verschieden substituiertes, geradkettiges oder verzweigtes Alkyl mit 1 bis 14 Kohlenstoffatomen steht, wobei als Substituenten infrage kommen:

Halogen - insbesondere Fluor, Chlor, Brom und/oder Iod -, Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl oder Alkylsulfonylaminocarbonyl mit jeweils 1 bis 8 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/ oder Schwefel - steht;

$R^{10}$ außerdem für jeweils gegebenenfalls einfach oder mehrfach, gleich oder verschieden durch Halogen - insbesondere Fluor, Chlor, Brom und/oder Iod - substituiertes Alkenyl oder Alkinyl mit jeweils 2 bis 8 Kohlenstoffatomen steht;

$R^{10}$ außerdem für gegebenenfalls einfach oder mehrfach, gleich oder verschieden durch Halogen - insbesondere Fluor, Chlor, Brom und/oder Iod - und/oder geradkettiges oder verzweigtes Alkyl mit 1 bis 4 Kohlenstoffatomen substituiertes Cycloalkyl mit 3 bis 7 Kohlenstoffatomen steht;

$R^{10}$ außerdem für jeweils gegebenenfalls im Arylteil einfach oder mehrfach, gleich oder verschieden substituiertes Arylalkyl oder Aryl mit jeweils 6 bis 10 Kohlenstoffatomen im Arylteil und gegebenenfalls 1 bis 4 Kohlenstoffatomen im geradkettigen oder verzweigten Alkylteil steht, oder für einen gegebenenfalls einfach oder mehrfach, gleich oder verschieden substituierten und/oder benzannellierten, gesättigten oder ungesättigten, fünf- bis siebengliedrigen Heterocyclylrest mit 1 bis 3 gleichen oder verschiedenen Heteroatomen - insbesondere Stickstoff, Sauerstoff und/oder Schwefel - steht, wobei als Aryl- bzw. Heterocyclylsubstituenten jeweils infrage kommen:

Halogen, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Alkoxycarbonyl oder Alkoximinoalkyl mit jeweils 1 bis 6 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen substituiertes Phenyl;

$R^{11}$ für Wasserstoff steht;

$R^{11}$ außerdem für gegebenenfalls einfach oder mehrfach, gleich oder verschieden substituiertes, geradkettiges oder verzweigtes Alkyl mit 1 bis 14 Kohlenstoffatomen steht, wobei als Substituenten infrage kommen:

Halogen - insbesondere Fluor, Chlor, Brom und/oder Iod -, Cyano, Carboxyl, Carbamoyl, jeweils geradkettiges oder verzweigtes Alkoxy, Alkoxyalkoxy, Alkylthio, Alkylsulfinyl, Alkylsulfonyl Alkoxycarbonyl, N-Alkylaminocarbonyl, N,N-Dialkylaminocarbonyl oder Alkylsulfonylaminocarbonyl mit jeweils 1 bis 8 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/ oder Schwefel - steht;

$R^{11}$ außerdem für jeweils gegebenenfalls einfach oder mehrfach, gleich oder verschieden durch Halogen - insbesondere Fluor, Chlor, Brom und/oder Iod - substituiertes Alkenyl oder Alkinyl mit jeweils 2 bis 8 Kohlenstoffatomen steht;

$R^{11}$ außerdem für gegebenenfalls einfach oder mehrfach, gleich oder verschieden durch Halogen - insbesondere Fluor, Chlor, Brom und/oder Iod - und/oder geradkettiges oder verzweigtes Alkyl mit 1 bis 4 Kohlenstoffatomen substituiertes Cycloalkyl mit 3 bis 7 Kohlenstoffatomen steht;

$R^{11}$ außerdem für jeweils gegebenenfalls im Arylteil einfach oder mehrfach, gleich oder verschieden substituiertes Arylalkyl oder Aryl mit jeweils 6 bis 10 Kohlenstoffatomen im Arylteil und gegebenenfalls 1 bis 4 Kohlenstoffatomen im geradkettigen oder verzweigten Alkylteil steht, wobei als Arylsubstituenten jeweils infrage kommen:

Halogen, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Alkoxycarbonyl oder Alkoximinoalkyl mit jeweils 1 bis 6 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen substituiertes

Phenyl;

$R^{12}$ für Wasserstoff steht;

$R^{12}$ außerdem für gegebenenfalls einfach oder mehrfach, gleich oder verschieden substituiertes, geradkettiges oder verzweigtes Alkyl mit 1 bis 14 Kohlenstoffatomen steht, wobei als Substituenten infrage kommen:
Halogen - insbesondere Fluor, Chlor, Brom und/oder Iod -, Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl oder Alkylsulfonylaminocarbonyl mit jeweils 1 bis 8 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/ oder Schwefel - steht;

$R^{12}$ außerdem für jeweils gegebenenfalls einfach oder mehrfach, gleich oder verschieden durch Halogen - insbesondere Fluor, Chlor, Brom und/oder Iod - substituiertes Alkenyl oder Alkinyl mit jeweils 2 bis 8 Kohlenstoffatomen steht;

$R^{12}$ außerdem für gegebenenfalls einfach oder mehrfach, gleich oder verschieden durch Halogen - insbesondere Fluor, Chlor, Brom und/oder Iod - und/oder geradkettiges oder verzweigtes Alkyl mit 1 bis 4 Kohlenstoffatomen substituiertes Cycloalkyl mit 3 bis 7 Kohlenstoffatomen steht;

$R^{12}$ außerdem für jeweils gegebenenfalls im Arylteil einfach oder mehrfach, gleich oder verschieden substituiertes Arylalkyl oder Alkyl mit jeweils 6 bis 10 Kohlenstoffatomen im Arylteil und gegebenenfalls 1 bis 4 Kohlenstoffatomen im geradkettigen oder verzweigten Alkylteil steht, oder für einen gegebenenfalls einfach oder mehrfach, gleich oder verschieden substituierten und/oder benzannellierten, gesättigten oder ungesättigten, fünf- bis siebengliedrigen Heterocyclylrest mit 1 bis 3 gleichen oder verschiedenen Heteroatomen - insbesondere Stickstoff, Sauerstoff und/oder Schwefel - steht, wobei als Aryl- bzw. Heterocyclylsubstituenten jeweils infrage kommen:
Halogen, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Alkoxycarbonyl oder Alkoximinoalkyl mit jeweils 1 bis 6 Kohlenstoffatomen in den einzelnen Alkylteilen, sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen substituiertes Phenyl.

Besonders bevorzugt sind Verbindungen der Formel (I), bei welchen

$R^1$ für Nitro oder für einen Rest $-NR^8R^9$ steht,

$R^2$ für Wasserstoff, Amino, Cyano oder für einen der Reste $-R^{10}$, $-O-R^{10}$, $-S-R^{10}$, $-NR^{10}R^{11}$, $-N(R^{11})-CO-R^{10}$ oder $-N=CR^{10}R^{11}$ steht,

$R^3$, $R^6$ und $R^7$ unabhängig voneinander jeweils für Wasserstoff, Fluor, Chlor, Brom oder Nitro stehen,

$R^4$ für Wasserstoff, Fluor, Chlor, Brom, Cyano, Nitro, oder für einen der Reste $-R^{12}$, $-O-R^{12}$, $-S-R^{12}$, $-S(O)-R^{12}$, $-SO_2-R^{12}$, $-SO_2-OR^{12}$, $-SO_2-NR^{11}R^{12}$, $-CO-OR^{12}$, $-CO-NR^{11}R^{12}$, $-O-SO_2-R^{12}$, $N(R^{11})-SO_2-R^{12}$, $-NR^{11}R^{12}$, $-NH-P(O)(R^{11})(OR^{12})$ oder $-NH-P(O)(OR^{11})(OR^{12})$ steht,

$R^5$ für Nitro, Cyano, Fluor, Chlor, Brom oder für geradkettiges oder verzweigtes Halogenalkyl mit 1 bis 4 Kohlenstoffatomen und 1 bis 9 gleichen oder verschiedenen Halogenatomen steht und

X für Sauerstoff oder Schwefel steht, wobei

$R^8$ und $R^9$ unabhängig voneinander jeweils für Wasserstoff stehen;

$R^8$ und $R^9$ außerdem für jeweils gegebenenfalls einfach oder zweifach, gleich oder verschieden substituiertes, geradkettiges oder verzweigtes Alkyl oder Alkoxy mit jeweils 1 bis 12 Kohlenstoffatomen stehen, wobei als Substituenten jeweils infrage kommen:
Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl oder Alkylsulfonylaminocarbonyl mit jeweils 1 bis 6 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/oder Schwefel - steht;

$R^8$ und $R^9$ außerdem für jeweils geradkettiges oder verzweigtes Halogenalkyl oder Halogenalkoxy mit jeweils 1 bis 6 Kohlenstoffatomen und 1 bis 13 gleichen oder verschiedenen Halogenatomen - insbesondere Fluor, Chlor und/oder Brom - stehen,

$R^8$ und $R^9$ außerdem für jeweils gegebenenfalls einfach bis fünffach, gleich oder verschieden durch Halogen - insbesondere Fluor, Chlor und/oder Brom - substituiertes Alkenyl oder Alkinyl mit jeweils 2 bis 8 Kohlenstoffatomen stehen;

$R^8$ und $R^9$ außerdem für gegebenenfalls einfach bis fünffach, gleich oder verschieden durch Halogen - insbesondere Fluor, Chlor und/oder Brom - und/oder geradkettiges oder verzweigtes Alkyl mit 1 bis 3 Kohlenstoffatomen substituiertes Cycloalkyl mit 3 bis 6 Kohlenstoffatomen stehen;

$R^8$ und $R^9$ außerdem für jeweils gegebenenfalls im Phenylteil einfach bis fünffach, gleich oder verschieden substituiertes Phenylalkyl oder Phenyl mit gegebenenfalls 1 bis 4 Kohlenstoffatomen im geradkettigen oder verzweigten Alkylteil stehen, oder für einen gegebenenfalls einfach bis dreifach, gleich oder verschieden substituierten und/oder benzannellierten, gesättigten oder ungesättigten, fünf- bis siebengliedrigen Heterocyclylrest mit 1 bis 3 gleichen oder verschiedenen Heteroatomen - insbesondere Stickstoff, Sauerstoff und/oder Schwefel - stehen, wobei als Phenyl- bzw. Heterocyclylsubstituenten jeweils infrage kommen:

Fluor, Chlor, Brom, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach bis fünffach, gleich oder verschieden durch Fluor, Chlor, Brom und/oder geradkettiges oder verzweigtes Alkyl oder Alkoxy mit jeweils 1 bis 4 Kohlenstoffatomen und/oder geradkettiges oder verzweigtes Halogenalkyl oder Halogenalkoxy mit jeweils 1 bis 4 Kohlenstoffatomen und 1 bis 9 gleichen oder verschiedenen Halogenatomen substituiertes Phenyl;

$R^8$ und $R^9$ außerdem gemeinsam mit dem Stickstoffatom, an welches sie gebunden sind, für einen gegebenenfalls einfach bis dreifach, gleich oder verschieden substituierten und/oder benzannellierten, gesättigten oder ungesättigten, fünf- bis siebengliedrigen Heterocyclylrest mit 1 bis 3 gleichen oder verschiedenen Heteroatomen - insbesondere Stickstoff, Sauerstoff und/oder Schwefel - stehen, wobei als Heterocyclylsubstituenten infrage kommen:

Fluor, Chlor, Brom, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach bis fünffach, gleich oder verschieden durch Fluor, Chlor, Brom und/oder geradkettiges oder verzweigtes Alkyl oder Alkoxy mit jeweils 1 bis 4 Kohlenstoffatomen und/oder geradkettiges oder verzweigtes Halogenalkyl oder Halogenalkoxy mit jeweils 1 bis 4 Kohlenstoffatomen und 1 bis 9 gleichen oder verschiedenen Halogenatomen substituiertes Phenyl;

$R^{10}$ für gegebenenfalls einfach oder zweifach, gleich oder verschieden substituiertes, geradkettiges oder verzweigtes Alkyl mit 1 bis 12 Kohlenstoffatomen steht, wobei als Substituenten infrage kommen:

Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl oder Alkylsulfonylaminocarbonyl mit jeweils 1 bis 6 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/oder Schwefel - steht;

$R^{10}$ außerdem für geradkettiges oder verzweigtes Halogenalkyl mit 1 bis 6 Kohlenstoffatomen und 1 bis 13 gleichen oder verschiedenen Halogenatomen - insbesondere Fluor, Chlor und/oder Brom - steht,

$R^{10}$ außerdem für jeweils gegebenenfalls einfach bis dreifach, gleich oder verschieden durch Halogen - insbesondere Fluor, Chlor und/oder Brom - substituiertes Alkenyl oder Alkinyl mit jeweils 2 bis 6 Kohlenstoffatomen steht;

$R^{10}$ außerdem für gegebenenfalls einfach bis dreifach, gleich oder verschieden durch Halogen - insbesondere Fluor, Chlor und/oder Brom - und/oder geradkettiges oder verzweigtes Alkyl mit 1 bis 3 Kohlenstoffatomen substituiertes Cycloalkyl mit 3 bis 7 Kohlenstoffatomen steht;

$R^{10}$ außerdem für jeweils gegebenenfalls im Phenylteil einfach bis fünffach, gleich oder verschieden substituiertes Phenylalkyl oder Phenyl mit gegebenenfalls 1 bis 3 Kohlenstoffatomen im geradkettigen oder verzweigten Alkylteil steht, oder für einen gegebenenfalls einfach bis dreifach, gleich oder verschieden substituierten und/oder benzannellierten, gesättigten oder ungesättigten, fünf- bis siebengliedrigen Heterocyclylrest mit 1 bis 3 gleichen oder verschiedenen Heteroatomen - insbesondere Stickstoff, Sauerstoff und/oder Schwefel - steht, wobei als Phenyl- bzw. Heterocyclylsubstituenten jeweils infrage kommen:

Fluor, Chlor, Brom, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach bis fünffach, gleich oder verschieden durch Fluor, Chlor, Brom und/oder geradkettiges oder verzweigtes Alkyl oder Alkoxy mit jeweils 1 bis 4 Kohlenstoffatomen und/oder geradkettiges oder verzweigtes Halogenalkyl oder Halogenalkoxy mit jeweils 1 bis 4 Kohlenstoffatomen und 1 bis 9 gleichen oder verschiedenen Halogenatomen substituiertes Phenyl;

$R^{11}$ für Wasserstoff steht;

für gegebenenfalls einfach oder zweifach, gleich oder verschieden substituiertes, geradkettiges oder verzweigtes Alkyl mit 1 bis 12 Kohlenstoffatomen steht, wobei als Substituenten infrage kommen:

Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkyaminocarbonyl, N,N-Dialkylaminocarbonyl oder Alkylsulfonylaminocarbonyl mit jeweils 1 bis 6 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/oder Schwefel - steht;

$R^{11}$ außerdem für geradkettiges oder verzweigtes Halogenalkyl mit 1 bis 6 Kohlenstoffatomen und 1 bis 13 gleichen oder verschiedenen Halogenatomen - insbesondere Fluor, Chlor und/oder Brom - steht,

$R^{11}$ außerdem für jeweils gegebenenfalls einfach bis dreifach, gleich oder verschieden durch Halogen - insbesondere Fluor, Chlor und/oder Brom - substituiertes Alkenyl oder Alkinyl mit jeweils 2 bis 6 Kohlenstoffatomen steht;

$R^{11}$ außerdem für gegebenenfalls einfach bis dreifach, gleich oder verschieden durch Halogen - insbesondere Fluor, Chlor und/oder Brom - und/oder geradkettiges oder verzweigtes Alkyl mit 1 bis 3 Kohlenstoffatomen substituiertes Cycloalkyl mit 3 bis 7 Kohlenstoffatomen steht;

$R^{11}$ außerdem für jeweils gegebenenfalls im Phenylteil einfach bis fünffach, gleich oder

verschieden substituiertes Phenylalkyl oder Phenyl mit gegebenenfalls 1 bis 3 Kohlenstoffatomen im geradkettigen oder verzweigten Alkylteil steht, wobei als Phenylsubstituenten jeweils infrage kommen:

Fluor, Chlor, Brom, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach bis fünffach, gleich oder verschieden durch Fluor, Chlor, Brom und/oder geradkettiges oder verzweigtes Alkyl oder Alkoxy mit jeweils 1 bis 4 Kohlenstoffatomen und/oder geradkettiges oder verzweigtes Halogenalkyl oder Halogenalkoxy mit jeweils 1 bis 4 Kohlenstoffatomen und 1 bis 9 gleichen oder verschiedenen Halogenatomen substituiertes Phenyl;

$R^{12}$ für Wasserstoff steht;

$R^{12}$ für gegebenenfalls einfach oder zweifach, gleich oder verschieden substituiertes, geradkettiges oder verzweigtes Alkyl mit 1 bis 12 Kohlenstoffatomen steht, wobei als Substituenten infrage kommen:

Cyano, Carboxyl, Carbamoyl, jeweils geradkettiges oder verzweigtes Alkoxy, Alkoxyalkoxy, Alkylthio, Alkylsulfinyl Alkylsulfonyl, Alkoxycarbonyl, N-Alkylaminocarbonyl, N,N-Dialkylaminocarbonyl oder Alkylsulfonylaminocarbonyl mit jeweils 1 bis 6 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/oder Schwefel - steht;

$R^{12}$ außerdem für geradkettiges oder verzweigtes Halogenalkyl mit 1 bis 6 Kohlenstoffatomen und 1 bis 13 gleichen oder verschiedenen Halogenatomen - insbesondere Fluor, Chlor und/oder Brom - steht,

$R^{12}$ außerdem für jeweils gegebenenfalls einfach bis dreifach, gleich oder verschieden durch Halogen - insbesondere Fluor, Chlor und/oder Brom - substituiertes Alkenyl oder Alkinyl mit jeweils 2 bis 6 Kohlenstoffatomen steht;

$R^{12}$ außerdem für gegebenenfalls einfach bis dreifach, gleich oder verschieden durch Halogen - insbesondere Fluor, Chlor und/oder Brom - und/oder geradkettiges oder verzweigtes Alkyl mit 1 bis 3 Kohlenstoffatomen substituiertes Cycloalkyl mit 3 bis 7 Kohlenstoffatomen steht;

$R^{12}$ außerdem für jeweils gegebenenfalls im Phenylteil einfach bis fünffach, gleich oder verschieden substituiertes Phenylalkyl oder Phenyl mit gegebenenfalls 1 bis 3 Kohlenstoffatomen im geradkettigen oder verzweigten Alkylteil steht oder für einen gegebenenfalls einfach bis dreifach, gleich oder verschieden substituierten und/oder benzannellierten, gesättigten oder ungesättigten, fünf- bis siebengliedrigen Heterocyclylrest mit 1 bis 3 gleichen oder verschiedenen Heteroatomen - insbesondere Stickstoff, Sauerstoff und/oder Schwefel - steht, wobei als Phenyl- bzw. Heterocyclylsubstituenten jeweils infrage kommen:

Fluor, Chlor, Brom, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach bis fünffach, gleich oder verschieden durch Fluor, Chlor, Brom und/oder geradkettiges oder verzweigtes Alkyl oder Alkoxy mit jeweils 1 bis 4 Kohlenstoffatomen und/oder geradkettiges oder verzweigtes Halogenalkyl oder Halogenalkoxy mit jeweils 1 bis 4 Kohlenstoffatomen und 1 bis 9 gleichen oder verschiedenen Halogenatomen substituiertes Phenyl.

Ganz besonders bevorzugt sind Verbindungen der Formel (I), bei welchen

$R^1$ für Nitro oder für einen Rest $-NR^8R^9$ steht,

$R^2$ für Wasserstoff, Amino, Cyano oder für einen der Reste $-R^{10}$, $-O-R^{10}$, $-S-R^{10}$, $-NR^{10}R^{11}$, $-N(R^{11})-CO-R^{10}$ oder $-N=CR^{10}R^{11}$ steht,

$R^3$, $R^6$ und $R^7$ unabhängig voneinander jeweils für Wasserstoff, Fluor, Chlor, Brom oder Nitro stehen,

$R^4$ für Wasserstoff, Fluor, Chlor, Brom, Cyano, Nitro, oder für einen der Reste $-R^{12}$, $-O-R^{12}$, $-S-R^{12}$, $-S(O)-R^{12}$, $-SO_2-R^{12}$, $-SO_2-OR^{12}$, $SO_2-NR^{11}R^{12}$, $-CO-OR^{12}$, $-CO-NR^{11}R^{12}$, $-O-SO_2-R^{12}$, $-N(R^{11})-SO_2-R^{12}$, $-NR^{11}R^{12}$, $-NH-P(O)(R^{11})(OR^{12})$ oder $-NH-P(O)(OR^{11})-(OR^{12})$ steht,

$R^5$ für Nitro, Cyano, Fluor, Chlor, Brom oder für geradkettiges oder verzweigtes Halogenalkyl mit 1 bis 3 Kohlenstoffatomen und 1 bis 7 gleichen oder verschiedenen Halogenatomen steht und

X für Sauerstoff oder Schwefel steht, wobei

$R^8$ und $R^9$ unabhängig voneinander jeweils für Wasserstoff stehen;

$R^8$ und $R^9$ außerdem für jeweils gegebenenfalls einfach substituiertes, geradkettiges oder verzweigtes Alkyl oder Alkoxy mit jeweils 1 bis 8 Kohlenstoffatomen stehen, wobei als Substituenten jeweils infrage kommen:
Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl oder Alkylsulfonylaminocarbonyl mit jeweils 1 bis 4 Kohlenstoffatomen in den einzelnen Alkylteilen oder Heterocyclyl, wobei als Heterocyclylrest ein fünf- oder sechsgliedriger, gegebenenfalls benzannellierter, gesättigter oder aromatischer Heterocyclus mit 1 bis 3 gleichen oder verschiedenen Heteroatomen - insbesondere Stickstoff, Sauerstoff und/oder Schwefel - steht;

$R^8$ und $R^9$ außerdem für jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen - insbesondere Fluor, Chlor und/oder Brom - stehen,

$R^8$ und $R^9$ außerdem für jeweils gegebenenfalls einfach bis dreifach, gleich oder verschieden durch Halogen - insbesondere Fluor, Chlor und/oder Brom - substituiertes Alkenyl oder Alkinyl mit jeweils 2 bis 6 Kohlenstoffatomen stehen;

$R^8$ und $R^9$ außerdem für gegebenenfalls einfach bis dreifach, gleich oder verschieden durch Halogen - insbesondere Fluor, Chlor und/oder Brom - und/oder geradkettiges oder verzweigtes Alkyl mit 1 bis 3 Kohlenstoffatomen substituiertes Cycloalkyl mit 3, 5 oder 6 Kohlenstoffatomen stehen;

$R^8$ und $R^9$ außerdem für jeweils gegebenenfalls im Phenylteil einfach bis dreifach, gleich oder verschieden substituiertes Phenylalkyl oder Phenyl mit gegebenenfalls 1 bis 3 Kohlenstoffatomen im geradkettigen oder verzweigten Alkylteil stehen, oder für einen gegebenenfalls einfach bis dreifach, gleich oder verschieden substituierten und/oder benzannellierten, gesättigten oder aromatischen, fünf- oder sechsgliedrigen Heterocyclylrest mit 1 bis 3 gleichen oder verschiedenen Heteroatomen - insbesondere Stickstoff, Sauerstoff und/oder Schwefel - stehen, wobei als Phenyl- bzw. Heterocyclylsubstituenten jeweils infrage kommen:
Fluor, Chlor, Brom, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 3 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 3 Kohlenstoffatomen und 1 bis 7 gleichen oder verschiedenen Halogenatomen, jeweils geradkettiges oder verzweigtes Alkoxycarbonyl oder Alkoximinoalkyl mit jeweils 1 bis 3 Kohlenstoffatomen in den einzelnen Alkylteilen sowie gegebenenfalls einfach bis dreifach, gleich oder verschieden durch Fluor, Chlor, Brom und/oder geradkettiges oder verzweigtes Alkyl oder Alkoxy mit jeweils 1 bis 3 Kohlenstoffatomen und/oder geradkettiges oder verzweigtes Halogenalkyl oder Halogenalkoxy mit jeweils 1 bis 3 Kohlenstoffatomen und 1 bis 7 gleichen oder verschiedenen Halogenatomen substituiertes Phenyl;

$R^8$ und $R^9$ außerdem gemeinsam mit dem Stickstoffatom, an welches sie gebunden sind, für einen gegebenenfalls einfach oder zweifach, gleich oder verschieden substituierten und/oder benzannellierten, gesättigten oder aromatischen, fünf- oder sechsgliedrigen Heterocyclylrest mit 1 bis 3 gleichen oder verschiedenen Heteroatomen - insbesondere Stickstoff, Sauerstoff und/oder Schwefel - stehen, wobei als Heterocyclylsubstituenten infrage kommen:

Fluor, Chlor, Brom, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 3 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 3 Kohlenstoffatomen und 1 bis 7 gleichen oder verschiedenen Halogenatomen, jeweils geradkettiges oder verzweigtes Alkoxycarbonyl oder Alkoximinoalkyl mit jeweils 1 bis 3 Kohlenstoffatomen in den einzelnen Alkylteilen sowie gegebenenfalls einfach bis dreifach, gleich oder verschieden durch Fluor, Chlor, Brom und/oder geradkettiges oder verzweigtes Alkyl oder Alkoxy mit jeweils 1 bis 3 Kohlenstoffatomen und/oder geradkettiges oder verzweigtes Halogenalkyl oder Halogenalkoxy mit jeweils 1 bis 3 Kohlenstoffatomen und 1 bis 7 gleichen oder verschiedenen Halogenatomen substituiertes Phenyl;

$R^{10}$ für gegebenenfalls einfach substituiertes, geradkettiges oder verzweigtes Alkyl mit 1 bis 8 Kohlenstoffatomen steht, wobei als Substituenten infrage kommen:
Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl oder Alkylsulfonylaminocarbonyl mit jeweils 1 bis 4 Kohlenstoffatomen in den einzelnen Alkylteilen oder Heterocyclyl, wobei als Heterocyclylrest ein fünf- oder sechsgliedriger, gegebenenfalls benzannellierter, gesättigter oder aromatischer Heterocyclus mit 1 bis 3 gleichen oder verschiedenen Heteroatomen - insbesondere Stickstoff, Sauerstoff und/oder Schwefel - steht;

$R^{10}$ außerdem für geradkettiges oder verzweigtes Halogenalkyl mit 1 bis 4 Kohlenstoffatomen und 1 bis 9 gleichen oder verschiedenen Halogenatomen - insbesondere Fluor, Chlor und/oder Brom - steht,

$R^{10}$ außerdem für jeweils gegebenenfalls einfach bis dreifach, gleich oder verschieden durch Halogen - insbesondere Fluor, Chlor und/oder Brom - substituiertes Alkenyl oder Alkinyl mit jeweils 2 bis 6 Kohlenstoffatomen steht;

$R^{10}$ außerdem für gegebenenfalls einfach bis dreifach, gleich oder verschieden durch Halogen - insbesondere Fluor, Chlor und/oder Brom - und/oder geradkettiges oder verzweigtes Alkyl mit 1 bis 3 Kohlenstoffatomen substituiertes Cycloalkyl mit 3, 5 oder 6 Kohlenstoffatomen steht;

$R^{10}$ außerdem für jeweils gegebenenfalls im Phenylteil einfach bis dreifach, gleich oder verschieden substituiertes Phenylalkyl oder Phenyl mit gegebenenfalls 1 bis 3 Kohlenstoffatomen im geradkettigen oder verzweigten Alkylteil steht, oder für einen gegebenenfalls einfach bis dreifach, gleich oder verschieden substituierten und/oder benzannellierten, gesättigten oder aromatischen, fünf- oder sechsgliedrigen Heterocyclylrest mit 1 bis 3 gleichen oder verschiedenen Heteroatomen - insbesondere Stickstoff, Sauerstoff und/oder Schwefel - steht, wobei als Phenyl- bzw. Heterocyclylsubstituenten jeweils infrage kommen:
Fluor, Chlor, Brom, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 3 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 3 Kohlenstoffatomen und 1 bis 7 gleichen oder verschiedenen Halogenatomen, jeweils geradkettiges oder verzweigtes Alkoxycarbonyl oder Alkoximinoalkyl mit jeweils 1 bis 3 Kohlenstoffatomen in den einzelnen Alkylteilen sowie gegebenenfalls einfach bis dreifach, gleich oder verschieden durch Fluor, Chlor, Brom und/oder geradkettiges oder verzweigtes Alkyl oder Alkoxy mit jeweils 1 bis 3 Kohlenstoffatomen und/oder geradkettiges oder verzweigtes Halogenalkyl oder Halogenalkoxy mit jeweils 1 bis 3 Kohlenstoffatomen und 1 bis 7 gleichen oder verschiedenen Halogenatomen substituiertes Phenyl;

$R^{11}$ für Wasserstoff steht;

$R^{11}$ für gegebenenfalls einfach substituiertes, geradkettiges oder verzweigtes Alkyl mit 1 bis 8 Kohlenstoffatomen steht, wobei als Substituenten infrage kommen:
Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl oder Alkylsulfonylaminocarbonyl mit jeweils 1 bis 4 Kohlenstoffatomen in den einzelnen Alkylteilen oder Heterocyclyl, wobei als Heterocyclylrest ein

fünf- oder sechsgliedriger, gegebenenfalls benzannellierter, gesättigter oder aromatischer Heterocyclus mit 1 bis 3 gleichen oder verschiedenen Heteroatomen - insbesondere Stickstoff, Sauerstoff und/oder Schwefel - steht;

$R^{11}$ außerdem für geradkettiges oder verzweigtes Halogenalkyl mit 1 bis 4 Kohlenstoffatomen und 1 bis 9 gleichen oder verschiedenen Halogenatomen - insbesondere Fluor, Chlor und/oder Brom - steht,

$R^{11}$ außerdem für jeweils gegebenenfalls einfach bis dreifach, gleich oder verschieden durch Halogen - insbesondere Fluor, Chlor und/oder Brom - substituiertes Alkenyl oder Alkinyl mit jeweils 2 bis 6 Kohlenstoffatomen steht;

$R^{11}$ außerdem für gegebenenfalls einfach bis dreifach, gleich oder verschieden durch Halogen - insbesondere Fluor, Chlor und/oder Brom - und/oder geradkettiges oder verzweigtes Alkyl mit 1 bis 3 Kohlenstoffatomen substituiertes Cycloalkyl mit 3, 5 oder 6 Kohlenstoffatomen steht;

$R^{11}$ außerdem für jeweils gegebenenfalls im Phenylteil einfach bis dreifach, gleich oder verschieden substituiertes Phenylalkyl oder Phenyl mit gegebenenfalls 1 bis 3 Kohlenstoffatomen im geradkettigen oder verzweigten Alkylteil steht, wobei als Phenylsubstituenten jeweils infrage kommen:

Fluor, Chlor, Brom, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 3 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 3 Kohlenstoffatomen und 1 bis 7 gleichen oder verschiedenen Halogenatomen, jeweils geradkettiges oder verzweigtes Alkoxycarbonyl oder Alkoximinoalkyl mit jeweils 1 bis 3 Kohlenstoffatomen in den einzelnen Alkylteilen sowie gegebenenfalls einfach bis dreifach, gleich oder verschieden durch Fluor, Chlor, Brom und/oder geradkettiges oder verzweigtes Alkyl oder Alkoxy mit jeweils 1 bis 3 Kohlenstoffatomen und/oder geradkettiges oder verzweigtes Halogenalkyl oder Halogenalkoxy mit jeweils 1 bis 3 Kohlenstoffatomen und 1 bis 7 gleichen oder verschiedenen Halogenatomen substituiertes Phenyl;

$R^{12}$ für Wasserstoff steht;

$R^{12}$ für gegebenenfalls einfach substituiertes, geradkettiges oder verzweigtes Alkyl mit 1 bis 8 Kohlenstoffatomen steht wobei als Substituenten infrage kommen:

Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl oder Alkylsulfonylaminocarbonyl mit jeweils 1 bis 4 Kohlenstoffatomen in den einzelnen Alkylteilen oder Heterocyclyl, wobei als Heterocyclylrest ein fünf- oder sechsgliedriger, gegebenenfalls benzannellierter, gesättigter oder aromatischer Heterocyclus mit 1 bis 3 gleichen oder verschiedenen Heteroatomen - insbesondere Stickstoff, Sauerstoff und/oder Schwefel - steht;

$R^{12}$ außerdem für geradkettiges oder verzweigtes Halogenalkyl mit 1 bis 4 Kohlenstoffatomen und 1 bis 9 gleichen oder verschiedenen Halogenatomen - insbesondere Fluor, Chlor und/oder Brom - steht,

$R^{12}$ außerdem für jeweils gegebenenfalls einfach bis dreifach, gleich oder verschieden durch Halogen - insbesondere Fluor, Chlor und/oder Brom - substituiertes Alkenyl oder Alkinyl mit jeweils 2 bis 6 Kohlenstoffatomen steht;

$R^{12}$ außerdem für gegebenenfalls einfach bis dreifach, gleich oder verschieden durch Halogen - insbesondere Fluor, Chlor und/oder Brom - und/oder geradkettiges oder verzweigtes Alkyl mit 1 bis 3 Kohlenstoffatomen substituiertes Cycloalkyl mit 3, 5 oder 6 Kohlenstoffatomen steht;

$R^{12}$ außerdem für jeweils gegebenenfalls im Phenylteil einfach bis dreifach, gleich oder verschieden substituiertes Phenylalkyl oder Phenyl mit gegebenenfalls 1 bis 3 Kohlenstoffatomen im geradkettigen oder verzweigten Alkylteil steht oder für einen gegebenenfalls einfach bis dreifach, gleich oder verschieden substituierten und/oder benzannellierten, gesättigten oder aromatischen, fünf- oder sechsgliedrigen Heterocyclylrest mit 1 bis 3 gleichen oder verschiedenen Heteroatomen - insbesondere Stickstoff, Sauerstoff und/oder Schwefel - steht, wobei als Phenyl- bzw. Heterocyclylsubstituenten jeweils infrage kommen:

Fluor, Chlor, Brom, Cyano, Nitro, Amino, N-Acetylamino, jeweils geradkettiges oder

verzweigtes Alkyl, Alkoxy, Alkylthio, Alkylsulfinyl oder Alkylsulfonyl mit jeweils 1 bis 3 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 3 Kohlenstoffatomen und 1 bis 7 gleichen oder verschiedenen Halogenatomen, jeweils geradkettiges oder verzweigtes Alkoxycarbonyl oder Alkoximinoalkyl mit jeweils 1 bis 3 Kohlenstoffatomen in den einzelnen Alkylteilen sowie gegebenenfalls einfach bis dreifach, gleich oder verschieden durch Fluor, Chlor, Brom und/oder geradkettiges oder verzweigtes Alkyl oder Alkoxy mit jeweils 1 bis 3 Kohlenstoffatomen und/oder geradkettiges oder verzweigtes Halogenalkyl oder Halogenalkoxy mit jeweils 1 bis 3 Kohlenstoffatomen und 1 bis 7 gleichen oder verschiedenen Halogenatomen substituiertes Phenyl;

Im einzelnen seien außer den bei den Herstellungsbeispielen aufgeführten Verbindungen die folgenden substituierten Triazolinone der allgemeinen Formel (I) genannt:

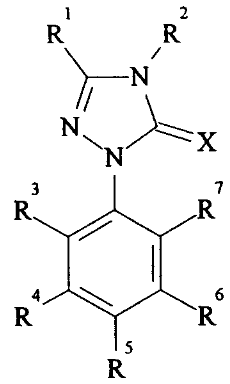

(I)

**Tabelle 1**

| $R^1$ | $R^2$ | $R^3$ | $R^4$ | $R^5$ | $R^6$ | $R^7$ | X |
|---|---|---|---|---|---|---|---|
| $NH_2$ | $CH_3$ | H | $-O-C_2H_5$ | CN | H | Cl | O |
| $NH_2$ | $CH_3$ | H | $-S-CH_3$ | CN | H | Cl | O |
| $NH_2$ | $CH_3$ | H | $-S-C_2H_5$ | CN | H | F | O |
| $NH_2$ | $CH_3$ | H | $-NH-SO_2-CH_3$ | CN | H | F | O |
| $NH_2$ | $CH_3$ | H | $-O-CH_2-C\equiv CH$ | CN | H | F | O |
| $NH_2$ | $CH_3$ | Cl | H | $CF_3$ | H | Cl | O |
| $NH_2$ | $CH_3$ | H | $-O-CH_2-CF_3$ | CN | H | F | O |
| $NH_2$ | $CH_3$ | H | $-O-CH_2-CH(CH_2F)_2$ | CN | H | F | O |
| $NH_2$ | $CH_3$ | H | $-O-CH_2-CH_2-O-C_2H_5$ | CN | H | F | O |
| $NH_2$ | $CH_3$ | H | $-S-CH_2-COOCH_3$ | CN | H | F | O |
| $NH_2$ | $CH_3$ | H | $-N(CH_3)_2$ | CN | H | F | O |
| $NO_2$ | $CH_3$ | H | F | CN | H | F | O |
| $NO_2$ | $CH_3$ | H | F | CN | H | Cl | O |
| $NO_2$ | $CH_3$ | H | $-O-CH(CH_3)-C\equiv CH$ | CN | H | F | O |
| $NO_2$ | $CH_3$ | H | $-S-CH_3$ | CN | H | F | O |
| $NO_2$ | $CH_3$ | H | $-NH-SO_2-C_2H_5$ | CN | H | F | O |
| $NO_2$ | $CH_3$ | H | $-O-SO_2-CH_3$ | CN | H | F | O |
| $NO_2$ | $CH_3$ | H | $-O-CH_2-C_6H_5$ | CN | H | F | O |
| $CH_3-NH-$ | H | H | F | $NO_2$ | H | F | O |
| $CH_3-NH-$ | H | H | F | $NO_2$ | H | F | S |
| $CH_3-NH-$ | H | H | F | $NO_2$ | H | Cl | O |

| $R^1$ | $R^2$ | $R^3$ | $R^4$ | $R^5$ | $R^6$ | $R^7$ | X |
|---|---|---|---|---|---|---|---|
| $CH_3$-NH- | H | Cl | H | $CF_3$ | H | Cl | O |
| $CH_3$-NH- | H | H | F | CN | H | F | O |
| $CH_3$-NH- | H | H | F | CN | H | Cl | O |
| $CH_3$-NH- | H | H | F | CN | H | Cl | S |
| $CH_3$-NH- | H | H | F | CN | H | F | S |
| $CH_3$-NH- | H | Cl | H | $CF_3$ | H | Cl | S |
| $CH_3$-NH- | H | H | CN | CN | H | H | O |
| $CH_3$-NH- | H | H | -O-$C_2H_5$ | CN | H | F | O |
| $CH_3$-NH- | H | H | -S-$CH_3$ | CN | H | F | O |
| $CH_3$-NH- | H | H | -O-$CH_3$ | CN | H | F | O |
| $CH_3$-NH- | H | H | -O-$CH_2$-C≡CH | CN | H | F | O |
| $CH_3$-NH- | H | H | -S-$CH_2$-C≡CH | CN | H | F | O |
| $CH_3$-NH- | H | H | -NH-$SO_2$-$CH_3$ | Cl | H | Cl | O |
| $CH_3$-NH- | H | H | -$NH_2$ | Cl | H | Cl | O |
| $CH_3$-NH- | H | H | -O-$SO_2$-$CH_3$ | Cl | H | Cl | O |
| $CH_3$-NH- | H | H | 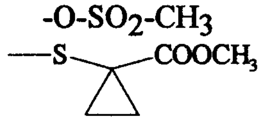 | Cl | H | Cl | O |
| $CH_3$-NH- | H | H | 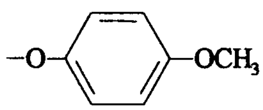 | Cl | H | Cl | O |
| $CH_3$-NH- | H | H | -NH-$CH_3$ | CN | H | Cl | O |
| $CH_3$-NH- | $NH_2$ | H | CN | CN | H | H | O |
| $CH_3$-NH- | $NH_2$ | H | -O-$C_2H_5$ | CN | H | F | O |
| $CH_3$-NH- | $NH_2$ | H | -S-$CH_3$ | CN | H | F | O |
| $CH_3$-NH- | $NH_2$ | H | -NH-$CH_3$ | CN | H | F | O |
| $CH_3$-NH- | $NH_2$ | H | -O-$SO_2$-$CH_3$ | CN | H | F | O |
| $CH_3$-NH- | $NH_2$ | H | -NH-$SO_2$-$CH_3$ | CN | H | F | O |
| $(CH_3)_2N$- | $CH_3$ | H | F | $NO_2$ | H | F | O |
| $(CH_3)_2N$- | $CH_3$ | H | F | $NO_2$ | H | Cl | O |
| $(CH_3)_2N$- | $CH_3$ | H | F | CN | H | H | O |
| $(CH_3)_2N$- | $CH_3$ | F | H | CN | H | H | O |

| $R^1$ | $R^2$ | $R^3$ | $R^4$ | $R^5$ | $R^6$ | $R^7$ | X |
|---|---|---|---|---|---|---|---|
| $(CH_3)_2N$- | $CH_3$ | Cl | H | $CF_3$ | H | Cl | O |
| $(CH_3)_2N$- | $CH_3$ | Cl | H | Cl | H | H | O |
| $(CH_3)_2N$- | $CH_3$ | $NO_2$ | H | $CF_3$ | H | $NO_2$ | O |
| $(CH_3)_2N$- | $CH_3$ | Cl | F | $CF_3$ | H | Cl | O |
| $(CH_3)_2N$- | $CH_3$ | H | Cl | CN | Cl | H | O |
| $(CH_3)_2N$- | $CH_3$ | H | CN | CN | H | H | O |
| $(CH_3)_2N$- | $CH_3$ | Cl | F | $NO_2$ | H | H | O |
| $(CH_3)_2N$- | $CH_3$ | Cl | H | $NO_2$ | H | Cl | O |
| $(CH_3)_2N$- | $CH_3$ | Cl | H | $NO_2$ | H | F | O |
| $(CH_3)_2N$- | $CH_3$ | H | F | CN | H | F | O |
| $(CH_3)_2N$- | $CH_3$ | H | F | CN | H | F | S |
| $(CH_3)_2N$- | $CH_3$ | H | F | CN | H | Cl | S |
| $(CH_3)_2N$- | $CH_3$ | H | F | CN | H | Cl | O |
| $(CH_3)_2N$- | $CH_3$ | H | $-NH_2$ | CN | H | Cl | O |
| $(CH_3)_2N$- | $CH_3$ | H | $-NH_2$ | CN | H | F | O |
| $(CH_3)_2N$- | $CH_3$ | H | $-NH_2$ | CN | H | F | S |
| $(CH_3)_2N$- | $CH_3$ | H | $-NH-CH_3$ | CN | H | F | O |
| $(CH_3)_2N$- | $CH_3$ | H | $-NH-CH_2-CH=CH_2$ | CN | H | F | O |
| $(CH_3)_2N$- | $CH_3$ | H | $-NH-s-C_4H_9$ | CN | H | F | O |
| $(CH_3)_2N$- | $CH_3$ | H | $-N(CH_3)_2$ | CN | H | F | O |
| $(CH_3)_2N$- | $CH_3$ | H | $-N(CH_2CN)_2$ | CN | H | F | O |
| $(CH_3)_2N$- | $CH_3$ | H | $-NH-SO_2-CH_3$ | CN | H | F | O |
| $(CH_3)_2N$- | $CH_3$ | H | $-NH-SO_2-CH_3$ | CN | H | Cl | O |
| $(CH_3)_2N$- | $CH_3$ | H | $-N(CH_3)-SO_2-CH_3$ | CN | H | F | O |
| $(CH_3)_2N$- | $CH_3$ | H | $-NH-SO_2-n-C_4H_9$ | CN | H | F | O |
| $(CH_3)_2N$- | $CH_3$ | H | $-O-SO_2-CH_3$ | CN | H | F | O |
| $(CH_3)_2N$- | $CH_3$ | H | $-NH-P(O)(CH_3)(OCH_3)$ | CN | H | F | O |
| $(CH_3)_2N$- | $CH_3$ | H | $-NH-P(O)(OC_2H_5)_2$ | CN | H | F | O |
| $(CH_3)_2N$- | $CH_3$ | H | -OH | CN | H | F | O |
| $(CH_3)_2N$- | $CH_3$ | H | $-O-CH_3$ | CN | H | F | O |

| $R^1$ | $R^2$ | $R^3$ | $R^4$ | $R^5$ | $R^6$ | $R^7$ | X |
|---|---|---|---|---|---|---|---|
| $(CH_3)_2N-$ | $CH_3$ | H | $-O-C_2H_5$ | CN | H | F | O |
| $(CH_3)_2N-$ | $CH_3$ | H | $-O-CH_2-C \equiv CH$ | CN | H | F | O |
| $(CH_3)_2N-$ | $CH_3$ | H | $-S-C_2H_5$ | CN | H | F | O |
| $(CH_3)_2N-$ | $CH_3$ | H | $-O-CH(CH_2F)_2$ | CN | H | F | O |
| $(CH_3)_2N-$ | $CH_3$ | H | $-(O-CH_2-CH_2)_2-OCH_3$ | CN | H | F | O |
| $(CH_3)_2N-$ | $CH_3$ | H | $-O-CH(CH_3)-COOCH_3$ | CN | H | F | O |
| $(CH_3)_2N-$ | $CH_3$ | H | $-O-CH(CH_3)-C \equiv CH$ | CN | H | F | O |
| $(CH_3)_2N-$ | $CH_3$ | H | $-S-CH_2-COOCH_3$ | CN | H | F | O |
| $(CH_3)_2N-$ | $CH_3$ | H | $-CH_2-CN$ | CN | H | F | O |
| $(CH_3)_2N-$ | $CH_3$ | H | 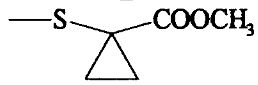 | CN | H | F | O |
| $(CH_3)_2N-$ | $CH_3$ | H | 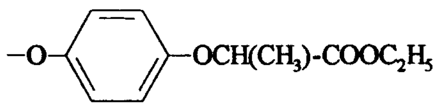 | CN | H | F | O |
| $(CH_3)_2N-$ | $CH_3$ | H | $-O-CH_2-Si(CH_3)_3$ | CN | H | F | O |
| $(CH_3)_2N-$ | $CH_3$ | H | 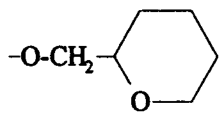 | CN | H | F | O |
| $(CH_3)_2N-$ | $CH_3$ | H | 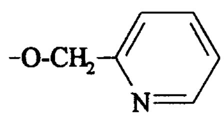 | CN | H | F | O |
| $(CH_3)_2N-$ | $CH_3$ | H | $-O-CHF_2$ | CN | H | F | O |
| $(CH_3)_2N-$ | $CH_3$ | H | $-COOCH_3$ | CN | H | Cl | O |
| $(CH_3)_2N-$ | $CH_3$ | H | $-CO-NH-CH_3$ | CN | H | Cl | O |
| $(CH_3)_2N-$ | $CH_3$ | H | $-CO-N(CH_3)_2$ | CN | H | F | O |
| $(CH_3)_2N-$ | $CH_3$ | H | $-CH_3$ | CN | H | F | O |
| $(CH_3)_2N-$ | $CH_3$ | H | Cl | Cl | H | Cl | O |
| $(CH_3)_2N-$ | $C_2H_5$ | H | F | $NO_2$ | H | F | O |
| $(CH_3)_2N-$ | $C_2H_5$ | H | F | $NO_2$ | H | Cl | O |
| $(CH_3)_2N-$ | $C_2H_5$ | H | F | CN | H | H | O |

| $R^1$ | $R^2$ | $R^3$ | $R^4$ | $R^5$ | $R^6$ | $R^7$ | X |
|---|---|---|---|---|---|---|---|
| $(CH_3)_2N$- | $C_2H_5$ | F | H | CN | H | H | O |
| $(CH_3)_2N$- | $C_2H_5$ | Cl | H | $CF_3$ | H | Cl | O |
| $(CH_3)_2N$- | $C_2H_5$ | Cl | H | Cl | H | H | O |
| $(CH_3)_2N$- | $C_2H_5$ | $NO_2$ | H | $CF_3$ | H | $NO_2$ | O |
| $(CH_3)_2N$- | $C_2H_5$ | Cl | F | $CF_3$ | H | Cl | O |
| $(CH_3)_2N$- | $C_2H_5$ | H | Cl | CN | Cl | H | O |
| $(CH_3)_2N$- | $C_2H_5$ | H | CN | CN | H | H | O |
| $(CH_3)_2N$- | $C_2H_5$ | Cl | F | $NO_2$ | H | H | O |
| $(CH_3)_2N$- | $C_2H_5$ | Cl | H | $NO_2$ | H | Cl | O |
| $(CH_3)_2N$- | $C_2H_5$ | Cl | H | $NO_2$ | H | F | O |
| $(CH_3)_2N$- | $C_2H_5$ | H | F | CN | H | F | O |
| $(CH_3)_2N$- | $C_2H_5$ | H | F | CN | H | F | S |
| $(CH_3)_2N$- | $C_2H_5$ | H | F | CN | H | Cl | S |
| $(CH_3)_2N$- | $C_2H_5$ | H | F | CN | H | Cl | O |
| $(CH_3)_2N$- | $C_2H_5$ | H | $-NH_2$ | CN | H | Cl | O |
| $(CH_3)_2N$- | $C_2H_5$ | H | $-NH_2$ | CN | H | F | O |
| $(CH_3)_2N$- | $C_2H_5$ | H | $-NH_2$ | CN | H | F | S |
| $(CH_3)_2N$- | $C_2H_5$ | H | $-NH-CH_3$ | CN | H | F | O |
| $(CH_3)_2N$- | $C_2H_5$ | H | $-NH-CH_2-CH=CH_2$ | CN | H | F | O |
| $(CH_3)_2N$- | $C_2H_5$ | H | $-NH-s-C_4H_9$ | CN | H | F | O |
| $(CH_3)_2N$- | $C_2H_5$ | H | $-N(CH_3)_2$ | CN | H | F | O |
| $(CH_3)_2N$- | $C_2H_5$ | H | $-N(CH_2CN)_2$ | CN | H | F | O |
| $(CH_3)_2N$- | $C_2H_5$ | H | $-NH-SO_2-CH_3$ | CN | H | F | O |
| $(CH_3)_2N$- | $C_2H_5$ | H | $-NH-SO_2-CH_3$ | CN | H | Cl | O |
| $(CH_3)_2N$- | $C_2H_5$ | H | $-N(CH_3)-SO_2-CH_3$ | CN | H | F | O |
| $(CH_3)_2N$- | $C_2H_5$ | H | $-NH-SO_2-n-C_4H_9$ | CN | H | F | O |
| $(CH_3)_2N$- | $C_2H_5$ | H | $-O-SO_2-CH_3$ | CN | H | F | O |
| $(CH_3)_2N$- | $C_2H_5$ | H | $-NH-P(O)(CH_3)(OCH_3)$ | CN | H | F | O |
| $(CH_3)_2N$- | $C_2H_5$ | H | $-NH-P(O)(OC_2H_5)_2$ | CN | H | F | O |
| $(CH_3)_2N$- | $C_2H_5$ | H | -OH | CN | H | F | O |

| $R^1$ | $R^2$ | $R^3$ | $R^4$ | $R^5$ | $R^6$ | $R^7$ | X |
|---|---|---|---|---|---|---|---|
| $(CH_3)_2N$- | $C_2H_5$ | H | $-O-CH_3$ | CN | H | F | O |
| $(CH_3)_2N$- | $C_2H_5$ | H | $-O-C_2H_5$ | CN | H | F | O |
| $(CH_3)_2N$- | $C_2H_5$ | H | $-O-CH_2-C{\equiv}CH$ | CN | H | F | O |
| $(CH_3)_2N$- | $C_2H_5$ | H | $-S-C_2H_5$ | CN | H | F | O |
| $(CH_3)_2N$- | $C_2H_5$ | H | $-O-CH(CH_2F)_2$ | CN | H | F | O |
| $(CH_3)_2N$- | $C_2H_5$ | H | $-(O-CH_2-CH_2)_2-OCH_3$ | CN | H | F | O |
| $(CH_3)_2N$- | $C_2H_5$ | H | $-O-CH(CH_3)-COOCH_3$ | CN | H | F | O |
| $(CH_3)_2N$- | $C_2H_5$ | H | $-O-CH(CH_3)-C{\equiv}CH$ | CN | H | F | O |
| $(CH_3)_2N$- | $C_2H_5$ | H | $-S-CH_2-COOCH_3$ | CN | H | F | O |
| $(CH_3)_2N$- | $C_2H_5$ | H | $-CH_2-CN$ | CN | H | F | O |
| $(CH_3)_2N$- | $C_2H_5$ | H | 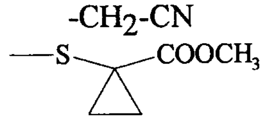 | CN | H | F | O |
| $(CH_3)_2N$- | $C_2H_5$ | H | 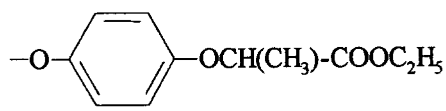 | CN | H | F | O |
| $(CH_3)_2N$- | $C_2H_5$ | H | $-O-CH_2-Si(CH_3)_3$ | CN | H | F | O |
| $(CH_3)_2N$- | $C_2H_5$ | H | 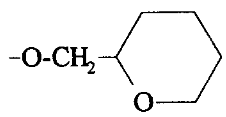 | CN | H | F | O |
| $(CH_3)_2N$- | $C_2H_5$ | H | 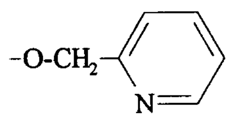 | CN | H | F | O |
| $(CH_3)_2N$- | $C_2H_5$ | H | $-O-CHF_2$ | CN | H | F | O |
| $(CH_3)_2N$- | $C_2H_5$ | H | $-COOCH_3$ | CN | H | Cl | O |
| $(CH_3)_2N$- | $C_2H_5$ | H | $-CO-NH-CH_3$ | CN | H | Cl | O |
| $(CH_3)_2N$- | $C_2H_5$ | H | $-CO-N(CH_3)_2$ | CN | H | F | O |
| $(CH_3)_2N$- | $C_2H_5$ | H | $-CH_3$ | CN | H | F | O |
| $(CH_3)_2N$- | $C_2H_5$ | H | Cl | Cl | H | Cl | O |
| $(CH_3)_2N$- | $i-C_3H_7$ | H | F | $NO_2$ | H | F | O |
| $(CH_3)_2N$- | $i-C_3H_7$ | H | F | $NO_2$ | H | Cl | O |
| $(CH_3)_2N$- | $i-C_3H_7$ | H | F | CN | H | H | O |

| $R^1$ | $R^2$ | $R^3$ | $R^4$ | $R^5$ | $R^6$ | $R^7$ | X |
|---|---|---|---|---|---|---|---|
| $(CH_3)_2N$- | i-$C_3H_7$ | F | H | CN | H | H | O |
| $(CH_3)_2N$- | i-$C_3H_7$ | Cl | H | $CF_3$ | H | Cl | O |
| $(CH_3)_2N$- | i-$C_3H_7$ | Cl | H | Cl | H | H | O |
| $(CH_3)_2N$- | i-$C_3H_7$ | $NO_2$ | H | $CF_3$ | H | $NO_2$ | O |
| $(CH_3)_2N$- | i-$C_3H_7$ | Cl | F | $CF_3$ | H | Cl | O |
| $(CH_3)_2N$- | i-$C_3H_7$ | H | Cl | CN | Cl | H | O |
| $(CH_3)_2N$- | i-$C_3H_7$ | H | CN | CN | H | H | O |
| $(CH_3)_2N$- | i-$C_3H_7$ | Cl | F | $NO_2$ | H | H | O |
| $(CH_3)_2N$- | i-$C_3H_7$ | Cl | H | $NO_2$ | H | Cl | O |
| $(CH_3)_2N$- | i-$C_3H_7$ | Cl | H | $NO_2$ | H | F | O |
| $(CH_3)_2N$- | i-$C_3H_7$ | H | F | CN | H | F | O |
| $(CH_3)_2N$- | i-$C_3H_7$ | H | F | CN | H | F | S |
| $(CH_3)_2N$- | i-$C_3H_7$ | H | F | CN | H | Cl | S |
| $(CH_3)_2N$- | i-$C_3H_7$ | H | F | CN | H | Cl | O |
| $(CH_3)_2N$- | i-$C_3H_7$ | H | $-NH_2$ | CN | H | Cl | O |
| $(CH_3)_2N$- | i-$C_3H_7$ | H | $-NH_2$ | CN | H | F | O |
| $(CH_3)_2N$- | i-$C_3H_7$ | H | $-NH_2$ | CN | H | F | S |
| $(CH_3)_2N$- | i-$C_3H_7$ | H | $-NH-CH_3$ | CN | H | F | O |
| $(CH_3)_2N$- | i-$C_3H_7$ | H | $-NH-CH_2-CH=CH_2$ | CN | H | F | O |
| $(CH_3)_2N$- | i-$C_3H_7$ | H | -NH-s-$C_4H_9$ | CN | H | F | O |
| $(CH_3)_2N$- | i-$C_3H_7$ | H | $-N(CH_3)_2$ | CN | H | F | O |
| $(CH_3)_2N$- | i-$C_3H_7$ | H | $-N(CH_2CN)_2$ | CN | H | F | O |
| $(CH_3)_2N$- | i-$C_3H_7$ | H | $-NH-SO_2-CH_3$ | CN | H | F | O |
| $(CH_3)_2N$- | i-$C_3H_7$ | H | $-NH-SO_2-CH_3$ | CN | H | Cl | O |
| $(CH_3)_2N$- | i-$C_3H_7$ | H | $-N(CH_3)-SO_2-CH_3$ | CN | H | F | O |
| $(CH_3)_2N$- | i-$C_3H_7$ | H | $-NH-SO_2$-n-$C_4H_9$ | CN | H | F | O |
| $(CH_3)_2N$- | i-$C_3H_7$ | H | $-O-SO_2-CH_3$ | CN | H | F | O |
| $(CH_3)_2N$- | i-$C_3H_7$ | H | $-NH-P(O)(CH_3)(OCH_3)$ | CN | H | F | O |
| $(CH_3)_2N$- | i-$C_3H_7$ | H | $-NH-P(O)(OC_2H_5)_2$ | CN | H | F | O |
| $(CH_3)_2N$- | i-$C_3H_7$ | H | -OH | CN | H | F | O |

| $R^1$ | $R^2$ | $R^3$ | $R^4$ | $R^5$ | $R^6$ | $R^7$ | X |
|---|---|---|---|---|---|---|---|
| $(CH_3)_2N$- | i-$C_3H_7$ | H | $-O-CH_3$ | CN | H | F | O |
| $(CH_3)_2N$- | i-$C_3H_7$ | H | $-O-C_2H_5$ | CN | H | F | O |
| $(CH_3)_2N$- | i-$C_3H_7$ | H | $-O-CH_2-C{\equiv}CH$ | CN | H | F | O |
| $(CH_3)_2N$- | i-$C_3H_7$ | H | $-S-C_2H_5$ | CN | H | F | O |
| $(CH_3)_2N$- | i-$C_3H_7$ | H | $-O-CH(CH_2F)_2$ | CN | H | F | O |
| $(CH_3)_2N$- | i-$C_3H_7$ | H | $-(O-CH_2-CH_2)_2-OCH_3$ | CN | H | F | O |
| $(CH_3)_2N$- | i-$C_3H_7$ | H | $-O-CH(CH_3)-COOCH_3$ | CN | H | F | O |
| $(CH_3)_2N$- | i-$C_3H_7$ | H | $-O-CH(CH_3)-C{\equiv}CH$ | CN | H | F | O |
| $(CH_3)_2N$- | i-$C_3H_7$ | H | $-S-CH_2-COOCH_3$ | CN | H | F | O |
| $(CH_3)_2N$- | i-$C_3H_7$ | H | $-CH_2-CN$ | CN | H | F | O |
| $(CH_3)_2N$- | i-$C_3H_7$ | H | 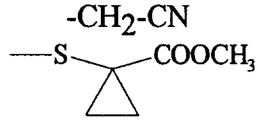 | CN | H | F | O |
| $(CH_3)_2N$- | i-$C_3H_7$ | H | 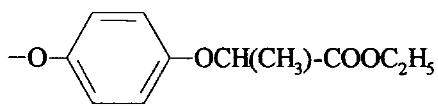 | CN | H | F | O |
| $(CH_3)_2N$- | i-$C_3H_7$ | H | $-O-CH_2-Si(CH_3)_3$ | CN | H | F | O |
| $(CH_3)_2N$- | i-$C_3H_7$ | H | 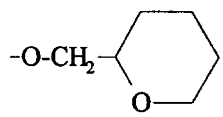 | CN | H | F | O |
| $(CH_3)_2N$- | i-$C_3H_7$ | H | 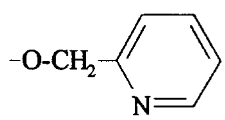 | CN | H | F | O |
| $(CH_3)_2N$- | i-$C_3H_7$ | H | $-O-CHF_2$ | CN | H | F | O |
| $(CH_3)_2N$- | i-$C_3H_7$ | H | $-COOCH_3$ | CN | H | Cl | O |
| $(CH_3)_2N$- | i-$C_3H_7$ | H | $-CO-NH-CH_3$ | CN | H | Cl | O |
| $(CH_3)_2N$- | i-$C_3H_7$ | H | $-CO-N(CH_3)_2$ | CN | H | F | O |
| $(CH_3)_2N$- | i-$C_3H_7$ | H | $-CH_3$ | CN | H | F | O |
| $(CH_3)_2N$- | i-$C_3H_7$ | H | Cl | Cl | H | Cl | O |
| 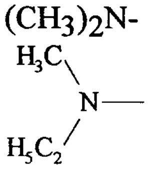 | $CH_3$ | H | F | $NO_2$ | H | F | O |

| $R^1$ | $R^2$ | $R^3$ | $R^4$ | $R^5$ | $R^6$ | $R^7$ | X |
|---|---|---|---|---|---|---|---|
| 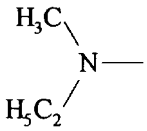 | $CH_3$ | H | F | $NO_2$ | H | Cl | O |
| 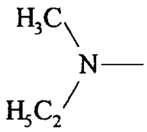 | $CH_3$ | H | F | CN | H | H | O |
| 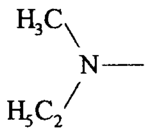 | $CH_3$ | F | H | CN | H | H | O |
| 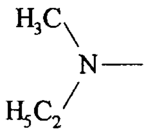 | $CH_3$ | Cl | H | $CF_3$ | H | Cl | O |
| 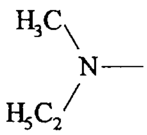 | $CH_3$ | Cl | H | Cl | H | H | O |
| 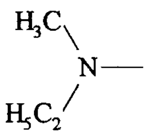 | $CH_3$ | $NO_2$ | H | $CF_3$ | H | $NO_2$ | O |
| 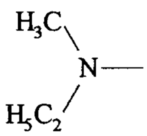 | $CH_3$ | Cl | F | $CF_3$ | H | Cl | O |
| 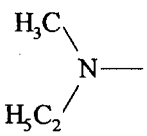 | $CH_3$ | H | Cl | CN | Cl | H | O |
| 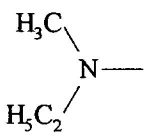 | $CH_3$ | H | CN | CN | H | H | O |
| 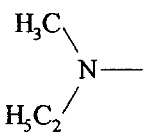 | $CH_3$ | Cl | F | $NO_2$ | H | H | O |

| $R^1$ | $R^2$ | $R^3$ | $R^4$ | $R^5$ | $R^6$ | $R^7$ | X |
|---|---|---|---|---|---|---|---|
| 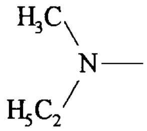 | $CH_3$ | Cl | H | $NO_2$ | H | Cl | O |
| 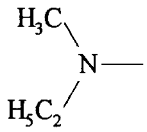 | $CH_3$ | Cl | H | $NO_2$ | H | F | O |
| 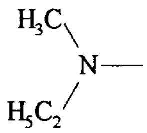 | $CH_3$ | H | F | CN | H | F | O |
| 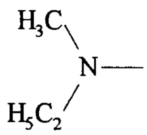 | $CH_3$ | H | F | CN | H | F | S |
| 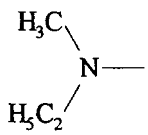 | $CH_3$ | H | F | CN | H | Cl | S |
| 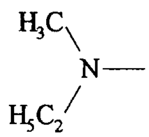 | $CH_3$ | H | F | CN | H | Cl | O |
| 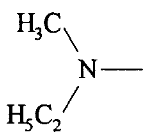 | $CH_3$ | H | $-NH_2$ | CN | H | Cl | O |
| 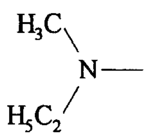 | $CH_3$ | H | $-NH_2$ | CN | H | F | O |
| 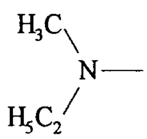 | $CH_3$ | H | $-NH_2$ | CN | H | F | S |
| 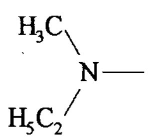 | $CH_3$ | H | $-NH-CH_3$ | CN | H | F | O |

| $R^1$ | $R^2$ | $R^3$ | $R^4$ | $R^5$ | $R^6$ | $R^7$ | X |
|---|---|---|---|---|---|---|---|
| 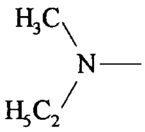 | $CH_3$ | H | $-NH-CH_2-CH=CH_2$ | CN | H | F | O |
| 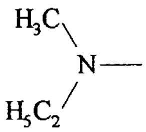 | $CH_3$ | H | $-NH-s-C_4H_9$ | CN | H | F | O |
| 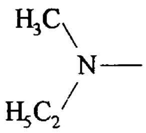 | $CH_3$ | H | $-N(CH_3)_2$ | CN | H | F | O |
| 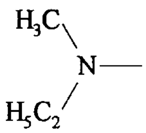 | $CH_3$ | H | $-N(CH_2CN)_2$ | CN | H | F | O |
| 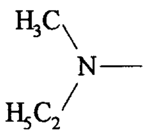 | $CH_3$ | H | $-NH-SO_2-CH_3$ | CN | H | F | O |
| 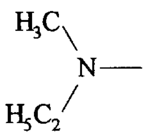 | $CH_3$ | H | $-NH-SO_2-CH_3$ | CN | H | Cl | O |
| 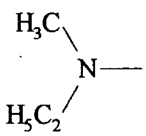 | $CH_3$ | H | $-N(CH_3)-SO_2-CH_3$ | CN | H | F | O |
| 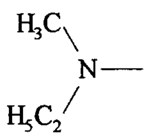 | $CH_3$ | H | $-NH-SO_2-n-C_4H_9$ | CN | H | F | O |
| 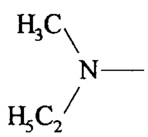 | $CH_3$ | H | $-O-SO_2-CH_3$ | CN | H | F | O |
| 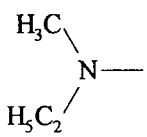 | $CH_3$ | H | $-NH-P(O)(CH_3)(OCH_3)$ | CN | H | F | O |

| $R^1$ | $R^2$ | $R^3$ | $R^4$ | $R^5$ | $R^6$ | $R^7$ | X |
|---|---|---|---|---|---|---|---|
| 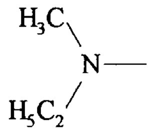 | $CH_3$ | H | $-NH-P(O)(OC_2H_5)_2$ | CN | H | F | O |
| 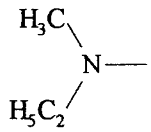 | $CH_3$ | H | -OH | CN | H | F | O |
| 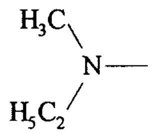 | $CH_3$ | H | $-O-CH_3$ | CN | H | F | O |
| 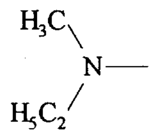 | $CH_3$ | H | $-O-C_2H_5$ | CN | H | F | O |
| 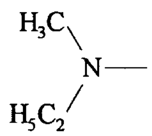 | $CH_3$ | H | $-O-CH_2-C{\equiv}CH$ | CN | H | F | O |
| 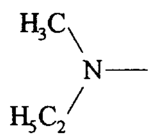 | $CH_3$ | H | $-S-C_2H_5$ | CN | H | F | O |
| 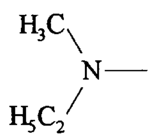 | $CH_3$ | H | $-O-CH(CH_2F)_2$ | CN | H | F | O |
| 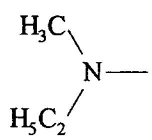 | $CH_3$ | H | $-(O-CH_2-CH_2)_2-OCH_3$ | CN | H | F | O |
| 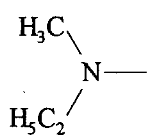 | $CH_3$ | H | $-O-CH(CH_3)-COOCH_3$ | CN | H | F | O |
| 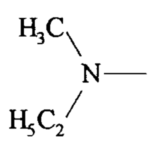 | $CH_3$ | H | $-O-CH(CH_3)-C{\equiv}CH$ | CN | H | F | O |

| $R^1$ | $R^2$ | $R^3$ | $R^4$ | $R^5$ | $R^6$ | $R^7$ | X |
|---|---|---|---|---|---|---|---|
| 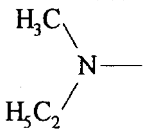 | $CH_3$ | H | $-S-CH_2-COOCH_3$ | CN | H | F | O |
| 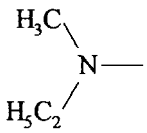 | $CH_3$ | H | $-CH_2-CN$ | CN | H | F | O |
| 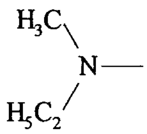 | $CH_3$ | H | 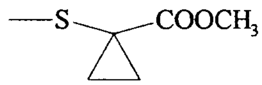 | CN | H | F | O |
| 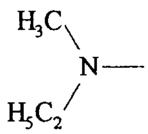 | $CH_3$ | H | 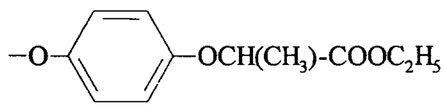 | CN | H | F | O |
| 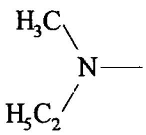 | $CH_3$ | H | $-O-CH_2-Si(CH_3)_3$ | CN | H | F | O |
| 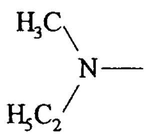 | $CH_3$ | H | 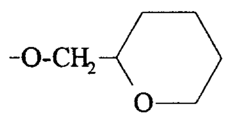 | CN | H | F | O |
| 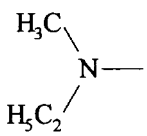 | $CH_3$ | H | 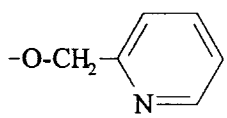 | CN | H | F | O |
| 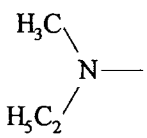 | $CH_3$ | H | $-O-CHF_2$ | CN | H | F | O |
| 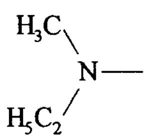 | $CH_3$ | H | $-COOCH_3$ | CN | H | Cl | O |
| 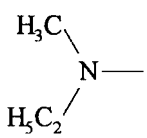 | $CH_3$ | H | $-CO-NH-CH_3$ | CN | H | Cl | O |

| $R^1$ | $R^2$ | $R^3$ | $R^4$ | $R^5$ | $R^6$ | $R^7$ | X |
|---|---|---|---|---|---|---|---|
| 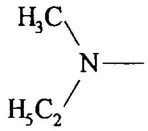 | $CH_3$ | H | $-CO-N(CH_3)_2$ | CN | H | F | O |
| 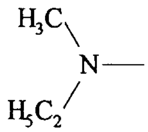 | $CH_3$ | H | $-CH_3$ | CN | H | F | O |
| 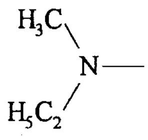 | $CH_3$ | H | Cl | Cl | H | Cl | O |
| 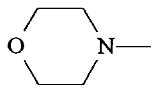 | $CH_3$ | H | F | $NO_2$ | H | F | O |
| 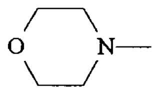 | $CH_3$ | H | F | $NO_2$ | H | Cl | O |
| 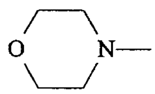 | $CH_3$ | H | F | CN | H | H | O |
| 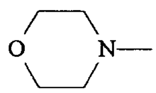 | $CH_3$ | F | H | CN | H | H | O |
| 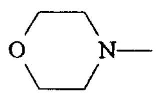 | $CH_3$ | Cl | H | $CF_3$ | H | Cl | O |
| 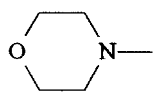 | $CH_3$ | Cl | H | Cl | H | H | O |
| 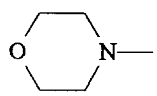 | $CH_3$ | $NO_2$ | H | $CF_3$ | H | $NO_2$ | O |
| 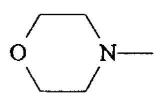 | $CH_3$ | Cl | F | $CF_3$ | H | Cl | O |
| 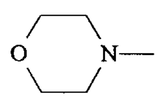 | $CH_3$ | H | Cl | CN | Cl | H | O |

| $R^1$ | $R^2$ | $R^3$ | $R^4$ | $R^5$ | $R^6$ | $R^7$ | X |
|---|---|---|---|---|---|---|---|
| 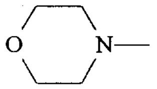 | $CH_3$ | H | CN | CN | H | H | O |
| 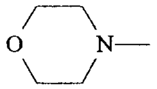 | $CH_3$ | Cl | F | $NO_2$ | H | H | O |
| 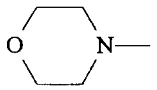 | $CH_3$ | Cl | H | $NO_2$ | H | Cl | O |
| 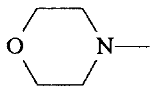 | $CH_3$ | Cl | H | $NO_2$ | H | F | O |
| 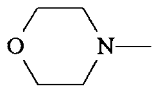 | $CH_3$ | H | F | CN | H | F | O |
| 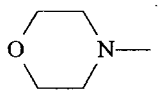 | $CH_3$ | H | F | CN | H | F | S |
| 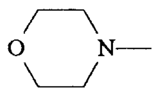 | $CH_3$ | H | F | CN | H | Cl | S |
| 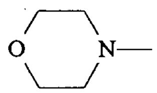 | $CH_3$ | H | F | CN | H | Cl | O |
| 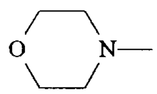 | $CH_3$ | H | $-NH_2$ | CN | H | Cl | O |
| 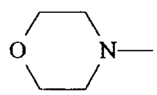 | $CH_3$ | H | $-NH_2$ | CN | H | F | O |
| 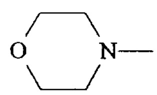 | $CH_3$ | H | $-NH_2$ | CN | H | F | S |
| 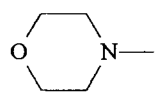 | $CH_3$ | H | $-NH-CH_3$ | CN | H | F | O |
| 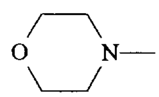 | $CH_3$ | H | $-NH-CH_2-CH=CH_2$ | CN | H | F | O |

| $R^1$ | $R^2$ | $R^3$ | $R^4$ | $R^5$ | $R^6$ | $R^7$ | X |
|---|---|---|---|---|---|---|---|
| 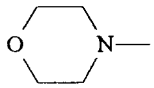 | $CH_3$ | H | $-NH-s-C_4H_9$ | CN | H | F | O |
| 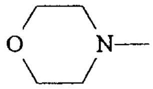 | $CH_3$ | H | $-N(CH_3)_2$ | CN | H | F | O |
| 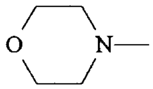 | $CH_3$ | H | $-N(CH_2CN)_2$ | CN | H | F | O |
| 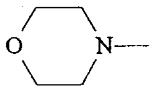 | $CH_3$ | H | $-NH-SO_2-CH_3$ | CN | H | F | O |
| 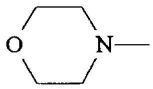 | $CH_3$ | H | $-NH-SO_2-CH_3$ | CN | H | Cl | O |
| 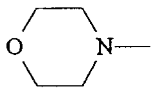 | $CH_3$ | H | $-N(CH_3)-SO_2-CH_3$ | CN | H | F | O |
| 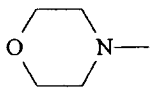 | $CH_3$ | H | $-NH-SO_2-n-C_4H_9$ | CN | H | F | O |
| 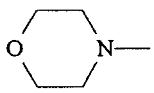 | $CH_3$ | H | $-O-SO_2-CH_3$ | CN | H | F | O |
| 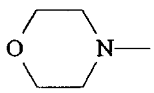 | $CH_3$ | H | $-NH-P(O)(CH_3)(OCH_3)$ | CN | H | F | O |
| 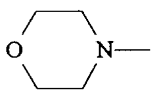 | $CH_3$ | H | $-NH-P(O)(OC_2H_5)_2$ | CN | H | F | O |
| 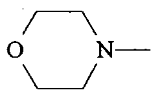 | $CH_3$ | H | -OH | CN | H | F | O |
| 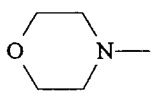 | $CH_3$ | H | $-O-CH_3$ | CN | H | F | O |
| 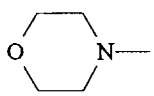 | $CH_3$ | H | $-O-C_2H_5$ | CN | H | F | O |

| $R^1$ | $R^2$ | $R^3$ | $R^4$ | $R^5$ | $R^6$ | $R^7$ | X |
|---|---|---|---|---|---|---|---|
| 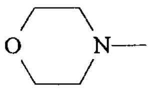 | $CH_3$ | H | $-O-CH_2-C \equiv CH$ | CN | H | F | O |
| 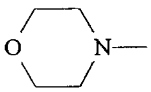 | $CH_3$ | H | $-S-C_2H_5$ | CN | H | F | O |
| 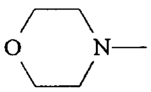 | $CH_3$ | H | $-O-CH(CH_2F)_2$ | CN | H | F | O |
| 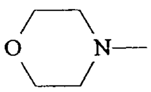 | $CH_3$ | H | $-(O-CH_2-CH_2)_2-OCH_3$ | CN | H | F | O |
| 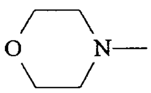 | $CH_3$ | H | $-O-CH(CH_3)-COOCH_3$ | CN | H | F | O |
| 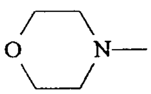 | $CH_3$ | H | $-O-CH(CH_3)-C \equiv CH$ | CN | H | F | O |
| 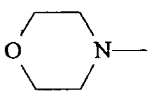 | $CH_3$ | H | $-S-CH_2-COOCH_3$ | CN | H | F | O |
| 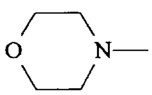 | $CH_3$ | H | $-CH_2-CN$ | CN | H | F | O |
| 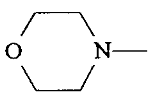 | $CH_3$ | H | 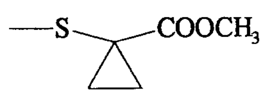 | CN | H | F | O |
| 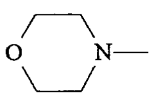 | $CH_3$ | H | 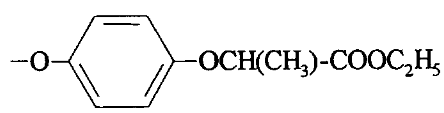 | CN | H | F | O |
| 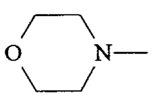 | $CH_3$ | H | $-O-CH_2-Si(CH_3)_3$ | CN | H | F | O |
| 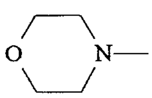 | $CH_3$ | H | 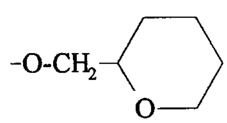 | CN | H | F | O |

| $R^1$ | $R^2$ | $R^3$ | $R^4$ | $R^5$ | $R^6$ | $R^7$ | X |
|---|---|---|---|---|---|---|---|
| 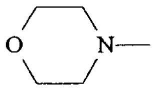 | $CH_3$ | H | 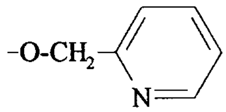 | CN | H | F | O |
| 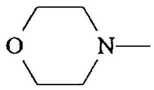 | $CH_3$ | H | $-O-CHF_2$ | CN | H | F | O |
| 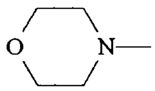 | $CH_3$ | H | $-COOCH_3$ | CN | H | Cl | O |
| 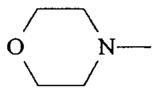 | $CH_3$ | H | $-CO-NH-CH_3$ | CN | H | Cl | O |
| 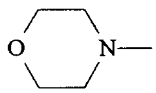 | $CH_3$ | H | $-CO-N(CH_3)_2$ | CN | H | F | O |
| 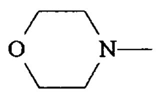 | $CH_3$ | H | $-CH_3$ | CN | H | F | O |
| 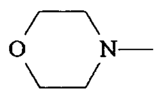 | $CH_3$ | H | Cl | Cl | H | Cl | O |
| $(CH_3)_2N-$ | $NH_2$ | H | F | $NO_2$ | H | F | O |
| $(CH_3)_2N-$ | $NH_2$ | H | F | $NO_2$ | H | Cl | O |
| $(CH_3)_2N-$ | $NH_2$ | H | F | CN | H | H | O |
| $(CH_3)_2N-$ | $NH_2$ | F | H | CN | H | H | O |
| $(CH_3)_2N-$ | $NH_2$ | Cl | H | $CF_3$ | H | Cl | O |
| $(CH_3)_2N-$ | $NH_2$ | Cl | H | Cl | H | H | O |
| $(CH_3)_2N-$ | $NH_2$ | $NO_2$ | H | $CF_3$ | H | $NO_2$ | O |
| $(CH_3)_2N-$ | $NH_2$ | Cl | F | $CF_3$ | H | Cl | O |
| $(CH_3)_2N-$ | $NH_2$ | H | Cl | CN | Cl | H | O |
| $(CH_3)_2N-$ | $NH_2$ | H | CN | CN | H | H | O |
| $(CH_3)_2N-$ | $NH_2$ | Cl | F | $NO_2$ | H | H | O |
| $(CH_3)_2N-$ | $NH_2$ | Cl | H | $NO_2$ | H | Cl | O |
| $(CH_3)_2N-$ | $NH_2$ | Cl | H | $NO_2$ | H | F | O |
| $(CH_3)_2N-$ | $NH_2$ | H | F | CN | H | F | O |

| $R^1$ | $R^2$ | $R^3$ | $R^4$ | $R^5$ | $R^6$ | $R^7$ | X |
|---|---|---|---|---|---|---|---|
| $(CH_3)_2N$- | $NH_2$ | H | F | CN | H | F | S |
| $(CH_3)_2N$- | $NH_2$ | H | F | CN | H | Cl | S |
| $(CH_3)_2N$- | $NH_2$ | H | F | CN | H | Cl | O |
| $(CH_3)_2N$- | $NH_2$ | H | $-NH_2$ | CN | H | Cl | O |
| $(CH_3)_2N$- | $NH_2$ | H | $-NH_2$ | CN | H | F | O |
| $(CH_3)_2N$- | $NH_2$ | H | $-NH_2$ | CN | H | F | S |
| $(CH_3)_2N$- | $NH_2$ | H | $-NH-CH_3$ | CN | H | F | O |
| $(CH_3)_2N$- | $NH_2$ | H | $-NH-CH_2-CH=CH_2$ | CN | H | F | O |
| $(CH_3)_2N$- | $NH_2$ | H | $-NH-s-C_4H_9$ | CN | H | F | O |
| $(CH_3)_2N$- | $NH_2$ | H | $-N(CH_3)_2$ | CN | H | F | O |
| $(CH_3)_2N$- | $NH_2$ | H | $-N(CH_2CN)_2$ | CN | H | F | O |
| $(CH_3)_2N$- | $NH_2$ | H | $-NH-SO_2-CH_3$ | CN | H | F | O |
| $(CH_3)_2N$- | $NH_2$ | H | $-NH-SO_2-CH_3$ | CN | H | Cl | O |
| $(CH_3)_2N$- | $NH_2$ | H | $-N(CH_3)-SO_2-CH_3$ | CN | H | F | O |
| $(CH_3)_2N$- | $NH_2$ | H | $-NH-SO_2-n-C_4H_9$ | CN | H | F | O |
| $(CH_3)_2N$- | $NH_2$ | H | $-O-SO_2-CH_3$ | CN | H | F | O |
| $(CH_3)_2N$- | $NH_2$ | H | $-NH-P(O)(CH_3)(OCH_3)$ | CN | H | F | O |
| $(CH_3)_2N$- | $NH_2$ | H | $-NH-P(O)(OC_2H_5)_2$ | CN | H | F | O |
| $(CH_3)_2N$- | $NH_2$ | H | -OH | CN | H | F | O |
| $(CH_3)_2N$- | $NH_2$ | H | $-O-CH_3$ | CN | H | F | O |
| $(CH_3)_2N$- | $NH_2$ | H | $-O-C_2H_5$ | CN | H | F | O |
| $(CH_3)_2N$- | $NH_2$ | H | $-O-CH_2-C\equiv CH$ | CN | H | F | O |
| $(CH_3)_2N$- | $NH_2$ | H | $-S-C_2H_5$ | CN | H | F | O |
| $(CH_3)_2N$- | $NH_2$ | H | $-O-CH(CH_2F)_2$ | CN | H | F | O |
| $(CH_3)_2N$- | $NH_2$ | H | $-(O-CH_2-CH_2)_2-OCH_3$ | CN | H | F | O |
| $(CH_3)_2N$- | $NH_2$ | H | $-O-CH(CH_3)-COOCH_3$ | CN | H | F | O |
| $(CH_3)_2N$- | $NH_2$ | H | $-O-CH(CH_3)-C\equiv CH$ | CN | H | F | O |
| $(CH_3)_2N$- | $NH_2$ | H | $-S-CH_2-COOCH_3$ | CN | H | F | O |
| $(CH_3)_2N$- | $NH_2$ | H | $-CH_2-CN$ | CN | H | F | O |

| $R^1$ | $R^2$ | $R^3$ | $R^4$ | $R^5$ | $R^6$ | $R^7$ | X |
|---|---|---|---|---|---|---|---|
| $(CH_3)_2N$- | $NH_2$ | H | 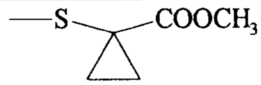 | CN | H | F | O |
| $(CH_3)_2N$- | $NH_2$ | H | 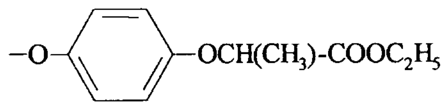 | CN | H | F | O |
| $(CH_3)_2N$- | $NH_2$ | H | $-O-CH_2-Si(CH_3)_3$ | CN | H | F | O |
| $(CH_3)_2N$- | $NH_2$ | H | 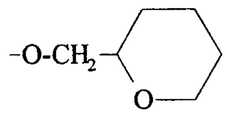 | CN | H | F | O |
| $(CH_3)_2N$- | $NH_2$ | H | 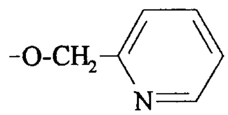 | CN | H | F | O |
| $(CH_3)_2N$- | $NH_2$ | H | $-O-CHF_2$ | CN | H | F | O |
| $(CH_3)_2N$- | $NH_2$ | H | $-COOCH_3$ | CN | H | Cl | O |
| $(CH_3)_2N$- | $NH_2$ | H | $-CO-NH-CH_3$ | CN | H | Cl | O |
| $(CH_3)_2N$- | $NH_2$ | H | $-CO-N(CH_3)_2$ | CN | H | F | O |
| $(CH_3)_2N$- | $NH_2$ | H | $-CH_3$ | CN | H | F | O |
| $(CH_3)_2N$- | $NH_2$ | H | Cl | Cl | H | Cl | O |
| $(CH_3)_2N$- | H | H | F | $NO_2$ | H | F | O |
| $(CH_3)_2N$- | H | H | F | $NO_2$ | H | Cl | O |
| $(CH_3)_2N$- | H | H | F | CN | H | H | O |
| $(CH_3)_2N$- | H | F | H | CN | H | H | O |
| $(CH_3)_2N$- | H | Cl | H | $CF_3$ | H | Cl | O |
| $(CH_3)_2N$- | H | Cl | H | Cl | H | H | O |
| $(CH_3)_2N$- | H | $NO_2$ | H | $CF_3$ | H | $NO_2$ | O |
| $(CH_3)_2N$- | H | Cl | F | $CF_3$ | H | Cl | O |
| $(CH_3)_2N$- | H | H | Cl | CN | Cl | H | O |
| $(CH_3)_2N$- | H | H | CN | CN | H | H | O |
| $(CH_3)_2N$- | H | Cl | F | $NO_2$ | H | H | O |

| $R^1$ | $R^2$ | $R^3$ | $R^4$ | $R^5$ | $R^6$ | $R^7$ | X |
|---|---|---|---|---|---|---|---|
| $(CH_3)_2N$- | H | Cl | H | $NO_2$ | H | Cl | O |
| $(CH_3)_2N$- | H | Cl | H | $NO_2$ | H | F | O |
| $(CH_3)_2N$- | H | H | F | CN | H | F | O |
| $(CH_3)_2N$- | H | H | F | CN | H | F | S |
| $(CH_3)_2N$- | H | H | F | CN | H | Cl | S |
| $(CH_3)_2N$- | H | H | F | CN | H | Cl | O |
| $(CH_3)_2N$- | H | H | $-NH_2$ | CN | H | Cl | O |
| $(CH_3)_2N$- | H | H | $-NH_2$ | CN | H | F | O |
| $(CH_3)_2N$- | H | H | $-NH_2$ | CN | H | F | S |
| $(CH_3)_2N$- | H | H | $-NH-CH_3$ | CN | H | F | O |
| $(CH_3)_2N$- | H | H | $-NH-CH_2-CH=CH_2$ | CN | H | F | O |
| $(CH_3)_2N$- | H | H | $-NH-s-C_4H_9$ | CN | H | F | O |
| $(CH_3)_2N$- | H | H | $-N(CH_3)_2$ | CN | H | F | O |
| $(CH_3)_2N$- | H | H | $-N(CH_2CN)_2$ | CN | H | F | O |
| $(CH_3)_2N$- | H | H | $-NH-SO_2-CH_3$ | CN | H | F | O |
| $(CH_3)_2N$- | H | H | $-NH-SO_2-CH_3$ | CN | H | Cl | O |
| $(CH_3)_2N$- | H | H | $-N(CH_3)-SO_2-CH_3$ | CN | H | F | O |
| $(CH_3)_2N$- | H | H | $-NH-SO_2-n-C_4H_9$ | CN | H | F | O |
| $(CH_3)_2N$- | H | H | $-O-SO_2-CH_3$ | CN | H | F | O |
| $(CH_3)_2N$- | H | H | $-NH-P(O)(CH_3)(OCH_3)$ | CN | H | F | O |
| $(CH_3)_2N$- | H | H | $-NH-P(O)(OC_2H_5)_2$ | CN | H | F | O |
| $(CH_3)_2N$- | H | H | -OH | CN | H | F | O |
| $(CH_3)_2N$- | H | H | $-O-CH_3$ | CN | H | F | O |
| $(CH_3)_2N$- | H | H | $-O-C_2H_5$ | CN | H | F | O |
| $(CH_3)_2N$- | H | H | $-O-CH_2-C\equiv CH$ | CN | H | F | O |
| $(CH_3)_2N$- | H | H | $-S-C_2H_5$ | CN | H | F | O |
| $(CH_3)_2N$- | H | H | $-O-CH(CH_2F)_2$ | CN | H | F | O |
| $(CH_3)_2N$- | H | H | $-(O-CH_2-CH_2)_2-OCH_3$ | CN | H | F | O |
| $(CH_3)_2N$- | H | H | $-O-CH(CH_3)-COOCH_3$ | CN | H | F | O |
| $(CH_3)_2N$- | H | H | $-O-CH(CH_3)-C\equiv CH$ | CN | H | F | O |

| $R^1$ | $R^2$ | $R^3$ | $R^4$ | $R^5$ | $R^6$ | $R^7$ | X |
|---|---|---|---|---|---|---|---|
| $(CH_3)_2N$- | H | H | $-S-CH_2-COOCH_3$ | CN | H | F | O |
| $(CH_3)_2N$- | H | H | $-CH_2-CN$ | CN | H | F | O |
| $(CH_3)_2N$- | H | H | 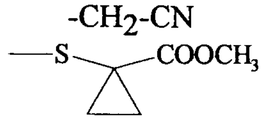 | CN | H | F | O |
| $(CH_3)_2N$- | H | H | 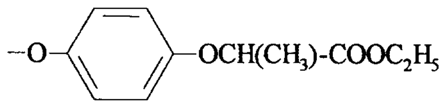 | CN | H | F | O |
| $(CH_3)_2N$- | H | H | $-O-CH_2-Si(CH_3)_3$ | CN | H | F | O |
| $(CH_3)_2N$- | H | H | 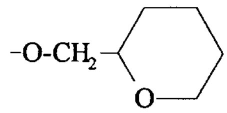 | CN | H | F | O |
| $(CH_3)_2N$- | H | H | 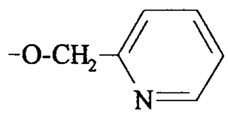 | CN | H | F | O |
| $(CH_3)_2N$- | H | H | $-O-CHF_2$ | CN | H | F | O |
| $(CH_3)_2N$- | H | H | $-COOCH_3$ | CN | H | Cl | O |
| $(CH_3)_2N$- | H | H | $-CO-NH-CH_3$ | CN | H | Cl | O |
| $(CH_3)_2N$- | H | H | $-CO-N(CH_3)_2$ | CN | H | F | O |
| $(CH_3)_2N$- | H | H | $-CH_3$ | CN | H | F | O |
| $(CH_3)_2N$- | H | H | Cl | Cl | H | Cl | O |
| $(CH_3)_2N$- | $(CH_3)_2N$- | H | F | $NO_2$ | H | F | O |
| $(CH_3)_2N$- | $(CH_3)_2N$- | H | F | $NO_2$ | H | Cl | O |
| $(CH_3)_2N$- | $(CH_3)_2N$- | H | F | CN | H | H | O |
| $(CH_3)_2N$- | $(CH_3)_2N$- | F | H | CN | H | H | O |
| $(CH_3)_2N$- | $(CH_3)_2N$- | Cl | H | $CF_3$ | H | Cl | O |
| $(CH_3)_2N$- | $(CH_3)_2N$- | Cl | H | Cl | H | H | O |
| $(CH_3)_2N$- | $(CH_3)_2N$- | $NO_2$ | H | $CF_3$ | H | $NO_2$ | O |
| $(CH_3)_2N$- | $(CH_3)_2N$- | Cl | F | $CF_3$ | H | Cl | O |
| $(CH_3)_2N$- | $(CH_3)_2N$- | H | Cl | CN | Cl | H | O |
| $(CH_3)_2N$- | $(CH_3)_2N$- | H | CN | CN | H | H | O |

| $R^1$ | $R^2$ | $R^3$ | $R^4$ | $R^5$ | $R^6$ | $R^7$ | X |
|---|---|---|---|---|---|---|---|
| $(CH_3)_2N$- | $(CH_3)_2N$- | Cl | F | $NO_2$ | H | H | O |
| $(CH_3)_2N$- | $(CH_3)_2N$- | Cl | H | $NO_2$ | H | Cl | O |
| $(CH_3)_2N$- | $(CH_3)_2N$- | Cl | H | $NO_2$ | H | F | O |
| $(CH_3)_2N$- | $(CH_3)_2N$- | H | F | CN | H | F | O |
| $(CH_3)_2N$- | $(CH_3)_2N$- | H | F | CN | H | F | S |
| $(CH_3)_2N$- | $(CH_3)_2N$- | H | F | CN | H | Cl | S |
| $(CH_3)_2N$- | $(CH_3)_2N$- | H | F | CN | H | Cl | O |
| $(CH_3)_2N$- | $(CH_3)_2N$- | H | $-NH_2$ | CN | H | Cl | O |
| $(CH_3)_2N$- | $(CH_3)_2N$- | H | $-NH_2$ | CN | H | F | O |
| $(CH_3)_2N$- | $(CH_3)_2N$- | H | $-NH_2$ | CN | H | F | S |
| $(CH_3)_2N$- | $(CH_3)_2N$- | H | $-NH-CH_3$ | CN | H | F | O |
| $(CH_3)_2N$- | $(CH_3)_2N$- | H | $-NH-CH_2-CH=CH_2$ | CN | H | F | O |
| $(CH_3)_2N$- | $(CH_3)_2N$- | H | $-NH-s-C_4H_9$ | CN | H | F | O |
| $(CH_3)_2N$- | $(CH_3)_2N$- | H | $-N(CH_3)_2$ | CN | H | F | O |
| $(CH_3)_2N$- | $(CH_3)_2N$- | H | $-N(CH_2CN)_2$ | CN | H | F | O |
| $(CH_3)_2N$- | $(CH_3)_2N$- | H | $-NH-SO_2-CH_3$ | CN | H | F | O |
| $(CH_3)_2N$- | $(CH_3)_2N$- | H | $-NH-SO_2-CH_3$ | CN | H | Cl | O |
| $(CH_3)_2N$- | $(CH_3)_2N$- | H | $-N(CH_3)-SO_2-CH_3$ | CN | H | F | O |
| $(CH_3)_2N$- | $(CH_3)_2N$- | H | $-NH-SO_2-n-C_4H_9$ | CN | H | F | O |
| $(CH_3)_2N$- | $(CH_3)_2N$- | H | $-O-SO_2-CH_3$ | CN | H | F | O |
| $(CH_3)_2N$- | $(CH_3)_2N$- | H | $-NH-P(O)(CH_3)(OCH_3)$ | CN | H | F | O |
| $(CH_3)_2N$- | $(CH_3)_2N$- | H | $-NH-P(O)(OC_2H_5)_2$ | CN | H | F | O |
| $(CH_3)_2N$- | $(CH_3)_2N$- | H | -OH | CN | H | F | O |
| $(CH_3)_2N$- | $(CH_3)_2N$- | H | $-O-CH_3$ | CN | H | F | O |
| $(CH_3)_2N$- | $(CH_3)_2N$- | H | $-O-C_2H_5$ | CN | H | F | O |
| $(CH_3)_2N$- | $(CH_3)_2N$- | H | $-O-CH_2-C\equiv CH$ | CN | H | F | O |
| $(CH_3)_2N$- | $(CH_3)_2N$- | H | $-S-C_2H_5$ | CN | H | F | O |
| $(CH_3)_2N$- | $(CH_3)_2N$- | H | $-O-CH(CH_2F)_2$ | CN | H | F | O |
| $(CH_3)_2N$- | $(CH_3)_2N$- | H | $-(O-CH_2-CH_2)_2-OCH_3$ | CN | H | F | O |
| $(CH_3)_2N$- | $(CH_3)_2N$- | H | $-O-CH(CH_3)-COOCH_3$ | CN | H | F | O |

| $R^1$ | $R^2$ | $R^3$ | $R^4$ | $R^5$ | $R^6$ | $R^7$ | X |
|---|---|---|---|---|---|---|---|
| $(CH_3)_2N$- | $(CH_3)_2N$- | H | $-O-CH(CH_3)-C\equiv CH$ | CN | H | F | O |
| $(CH_3)_2N$- | $(CH_3)_2N$- | H | $-S-CH_2-COOCH_3$ | CN | H | F | O |
| $(CH_3)_2N$- | $(CH_3)_2N$- | H | $-CH_2-CN$ | CN | H | F | O |
| $(CH_3)_2N$- | $(CH_3)_2N$- | H | 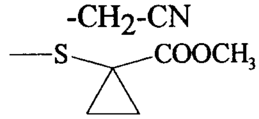 | CN | H | F | O |
| $(CH_3)_2N$- | $(CH_3)_2N$- | H | 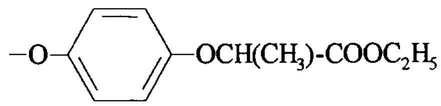 | CN | H | F | O |
| $(CH_3)_2N$- | $(CH_3)_2N$- | H | $-O-CH_2-Si(CH_3)_3$ | CN | H | F | O |
| $(CH_3)_2N$- | $(CH_3)_2N$- | H | 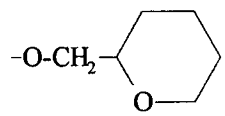 | CN | H | F | O |
| $(CH_3)_2N$- | $(CH_3)_2N$- | H | 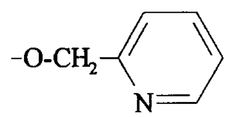 | CN | H | F | O |
| $(CH_3)_2N$- | $(CH_3)_2N$- | H | $-O-CHF_2$ | CN | H | F | O |
| $(CH_3)_2N$- | $(CH_3)_2N$- | H | $-COOCH_3$ | CN | H | Cl | O |
| $(CH_3)_2N$- | $(CH_3)_2N$- | H | $-CO-NH-CH_3$ | CN | H | Cl | O |
| $(CH_3)_2N$- | $(CH_3)_2N$- | H | $-CO-N(CH_3)_2$ | CN | H | F | O |
| $(CH_3)_2N$- | $(CH_3)_2N$- | H | $-CH_3$ | CN | H | F | O |
| $(CH_3)_2N$- | $(CH_3)_2N$- | H | Cl | Cl | H | Cl | O |
| $(CH_3)_2N$- | $CH_3-NH$- | H | F | $NO_2$ | H | F | O |
| $(CH_3)_2N$- | $CH_3-NH$- | H | F | $NO_2$ | H | Cl | O |
| $(CH_3)_2N$- | $CH_3-NH$- | H | F | CN | H | H | O |
| $(CH_3)_2N$- | $CH_3-NH$- | F | H | CN | H | H | O |
| $(CH_3)_2N$- | $CH_3-NH$- | Cl | H | $CF_3$ | H | Cl | O |
| $(CH_3)_2N$- | $CH_3-NH$- | Cl | H | Cl | H | H | O |
| $(CH_3)_2N$- | $CH_3-NH$- | $NO_2$ | H | $CF_3$ | H | $NO_2$ | O |
| $(CH_3)_2N$- | $CH_3-NH$- | Cl | F | $CF_3$ | H | Cl | O |
| $(CH_3)_2N$- | $CH_3-NH$- | H | Cl | CN | Cl | H | O |

| $R^1$ | $R^2$ | $R^3$ | $R^4$ | $R^5$ | $R^6$ | $R^7$ | X |
|---|---|---|---|---|---|---|---|
| $(CH_3)_2N$- | $CH_3$-NH- | H | CN | CN | H | H | O |
| $(CH_3)_2N$- | $CH_3$-NH- | Cl | F | $NO_2$ | H | H | O |
| $(CH_3)_2N$- | $CH_3$-NH- | Cl | H | $NO_2$ | H | Cl | O |
| $(CH_3)_2N$- | $CH_3$-NH- | Cl | H | $NO_2$ | H | F | O |
| $(CH_3)_2N$- | $CH_3$-NH- | H | F | CN | H | F | O |
| $(CH_3)_2N$- | $CH_3$-NH- | H | F | CN | H | F | S |
| $(CH_3)_2N$- | $CH_3$-NH- | H | F | CN | H | Cl | S |
| $(CH_3)_2N$- | $CH_3$-NH- | H | F | CN | H | Cl | O |
| $(CH_3)_2N$- | $CH_3$-NH- | H | $-NH_2$ | CN | H | Cl | O |
| $(CH_3)_2N$- | $CH_3$-NH- | H | $-NH_2$ | CN | H | F | O |
| $(CH_3)_2N$- | $CH_3$-NH- | H | $-NH_2$ | CN | H | F | S |
| $(CH_3)_2N$- | $CH_3$-NH- | H | $-NH-CH_3$ | CN | H | F | O |
| $(CH_3)_2N$- | $CH_3$-NH- | H | $-NH-CH_2-CH=CH_2$ | CN | H | F | O |
| $(CH_3)_2N$- | $CH_3$-NH- | H | -NH-s-$C_4H_9$ | CN | H | F | O |
| $(CH_3)_2N$- | $CH_3$-NH- | H | $-N(CH_3)_2$ | CN | H | F | O |
| $(CH_3)_2N$- | $CH_3$-NH- | H | $-N(CH_2CN)_2$ | CN | H | F | O |
| $(CH_3)_2N$- | $CH_3$-NH- | H | $-NH-SO_2-CH_3$ | CN | H | F | O |
| $(CH_3)_2N$- | $CH_3$-NH- | H | $-NH-SO_2-CH_3$ | CN | H | Cl | O |
| $(CH_3)_2N$- | $CH_3$-NH- | H | $-N(CH_3)-SO_2-CH_3$ | CN | H | F | O |
| $(CH_3)_2N$- | $CH_3$-NH- | H | $-NH-SO_2$-n-$C_4H_9$ | CN | H | F | O |
| $(CH_3)_2N$- | $CH_3$-NH- | H | $-O-SO_2-CH_3$ | CN | H | F | O |
| $(CH_3)_2N$- | $CH_3$-NH- | H | $-NH-P(O)(CH_3)(OCH_3)$ | CN | H | F | O |
| $(CH_3)_2N$- | $CH_3$-NH- | H | $-NH-P(O)(OC_2H_5)_2$ | CN | H | F | O |
| $(CH_3)_2N$- | $CH_3$-NH- | H | -OH | CN | H | F | O |
| $(CH_3)_2N$- | $CH_3$-NH- | H | $-O-CH_3$ | CN | H | F | O |
| $(CH_3)_2N$- | $CH_3$-NH- | H | $-O-C_2H_5$ | CN | H | F | O |
| $(CH_3)_2N$- | $CH_3$-NH- | H | $-O-CH_2-C\equiv CH$ | CN | H | F | O |
| $(CH_3)_2N$- | $CH_3$-NH- | H | $-S-C_2H_5$ | CN | H | F | O |
| $(CH_3)_2N$- | $CH_3$-NH- | H | $-O-CH(CH_2F)_2$ | CN | H | F | O |
| $(CH_3)_2N$- | $CH_3$-NH- | H | $-(O-CH_2-CH_2)_2-OCH_3$ | CN | H | F | O |

| $R^1$ | $R^2$ | $R^3$ | $R^4$ | $R^5$ | $R^6$ | $R^7$ | X |
|---|---|---|---|---|---|---|---|
| $(CH_3)_2N$- | $CH_3$-NH- | H | -O-CH($CH_3$)-$COOCH_3$ | CN | H | F | O |
| $(CH_3)_2N$- | $CH_3$-NH- | H | -O-CH($CH_3$)-C≡CH | CN | H | F | O |
| $(CH_3)_2N$- | $CH_3$-NH- | H | -S-$CH_2$-$COOCH_3$ | CN | H | F | O |
| $(CH_3)_2N$- | $CH_3$-NH- | H | -$CH_2$-CN | CN | H | F | O |
| $(CH_3)_2N$- | $CH_3$-NH- | H | 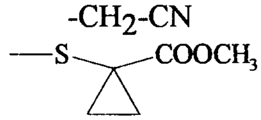 | CN | H | F | O |
| $(CH_3)_2N$- | $CH_3$-NH- | H | 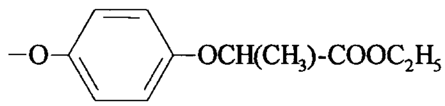 | CN | H | F | O |
| $(CH_3)_2N$- | $CH_3$-NH- | H | -O-$CH_2$-Si($CH_3$)$_3$ | CN | H | F | O |
| $(CH_3)_2N$- | $CH_3$-NH- | H | 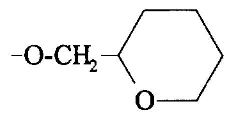 | CN | H | F | O |
| $(CH_3)_2N$- | $CH_3$-NH- | H | 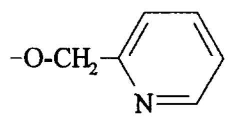 | CN | H | F | O |
| $(CH_3)_2N$- | $CH_3$-NH- | H | -O-$CHF_2$ | CN | H | F | O |
| $(CH_3)_2N$- | $CH_3$-NH- | H | -$COOCH_3$ | CN | H | Cl | O |
| $(CH_3)_2N$- | $CH_3$-NH- | H | -CO-NH-$CH_3$ | CN | H | Cl | O |
| $(CH_3)_2N$- | $CH_3$-NH- | H | -CO-N($CH_3$)$_2$ | CN | H | F | O |
| $(CH_3)_2N$- | $CH_3$-NH- | H | -$CH_3$ | CN | H | F | O |
| $(CH_3)_2N$- | $CH_3$-NH- | H | Cl | Cl | H | Cl | O |
| $(CH_3)_2N$- | $F_2CH$- | H | F | $NO_2$ | H | F | O |
| $(CH_3)_2N$- | $F_2CH$- | H | F | $NO_2$ | H | Cl | O |
| $(CH_3)_2N$- | $F_2CH$- | H | F | CN | H | H | O |
| $(CH_3)_2N$- | $F_2CH$- | F | H | CN | H | H | O |
| $(CH_3)_2N$- | $F_2CH$- | Cl | H | $CF_3$ | H | Cl | O |
| $(CH_3)_2N$- | $F_2CH$- | Cl | H | Cl | H | H | O |
| $(CH_3)_2N$- | $F_2CH$- | $NO_2$ | H | $CF_3$ | H | $NO_2$ | O |
| $(CH_3)_2N$- | $F_2CH$- | Cl | F | $CF_3$ | H | Cl | O |

| $R^1$ | $R^2$ | $R^3$ | $R^4$ | $R^5$ | $R^6$ | $R^7$ | X |
|---|---|---|---|---|---|---|---|
| $(CH_3)_2N$- | $F_2CH$- | H | Cl | CN | Cl | H | O |
| $(CH_3)_2N$- | $F_2CH$- | H | CN | CN | H | H | O |
| $(CH_3)_2N$- | $F_2CH$- | Cl | F | $NO_2$ | H | H | O |
| $(CH_3)_2N$- | $F_2CH$- | Cl | H | $NO_2$ | H | Cl | O |
| $(CH_3)_2N$- | $F_2CH$- | Cl | H | $NO_2$ | H | F | O |
| $(CH_3)_2N$- | $F_2CH$- | H | F | CN | H | F | O |
| $(CH_3)_2N$- | $F_2CH$- | H | F | CN | H | F | S |
| $(CH_3)_2N$- | $F_2CH$- | H | F | CN | H | Cl | S |
| $(CH_3)_2N$- | $F_2CH$- | H | F | CN | H | Cl | O |
| $(CH_3)_2N$- | $F_2CH$- | H | $-NH_2$ | CN | H | Cl | O |
| $(CH_3)_2N$- | $F_2CH$- | H | $-NH_2$ | CN | H | F | O |
| $(CH_3)_2N$- | $F_2CH$- | H | $-NH_2$ | CN | H | F | S |
| $(CH_3)_2N$- | $F_2CH$- | H | $-NH-CH_3$ | CN | H | F | O |
| $(CH_3)_2N$- | $F_2CH$- | H | $-NH-CH_2-CH=CH_2$ | CN | H | F | O |
| $(CH_3)_2N$- | $F_2CH$- | H | $-NH-s-C_4H_9$ | CN | H | F | O |
| $(CH_3)_2N$- | $F_2CH$- | H | $-N(CH_3)_2$ | CN | H | F | O |
| $(CH_3)_2N$- | $F_2CH$- | H | $-N(CH_2CN)_2$ | CN | H | F | O |
| $(CH_3)_2N$- | $F_2CH$- | H | $-NH-SO_2-CH_3$ | CN | H | F | O |
| $(CH_3)_2N$- | $F_2CH$- | H | $-NH-SO_2-CH_3$ | CN | H | Cl | O |
| $(CH_3)_2N$- | $F_2CH$- | H | $-N(CH_3)-SO_2-CH_3$ | CN | H | F | O |
| $(CH_3)_2N$- | $F_2CH$- | H | $-NH-SO_2-n-C_4H_9$ | CN | H | F | O |
| $(CH_3)_2N$- | $F_2CH$- | H | $-O-SO_2-CH_3$ | CN | H | F | O |
| $(CH_3)_2N$- | $F_2CH$- | H | $-NH-P(O)(CH_3)(OCH_3)$ | CN | H | F | O |
| $(CH_3)_2N$- | $F_2CH$- | H | $-NH-P(O)(OC_2H_5)_2$ | CN | H | F | O |
| $(CH_3)_2N$- | $F_2CH$- | H | -OH | CN | H | F | O |
| $(CH_3)_2N$- | $F_2CH$- | H | $-O-CH_3$ | CN | H | F | O |
| $(CH_3)_2N$- | $F_2CH$- | H | $-O-C_2H_5$ | CN | H | F | O |
| $(CH_3)_2N$- | $F_2CH$- | H | $-O-CH_2-C\equiv CH$ | CN | H | F | O |
| $(CH_3)_2N$- | $F_2CH$- | H | $-S-C_2H_5$ | CN | H | F | O |
| $(CH_3)_2N$- | $F_2CH$- | H | $-O-CH(CH_2F)_2$ | CN | H | F | O |

| $R^1$ | $R^2$ | $R^3$ | $R^4$ | $R^5$ | $R^6$ | $R^7$ | X |
|---|---|---|---|---|---|---|---|
| $(CH_3)_2N$- | $F_2CH$- | H | $-(O-CH_2-CH_2)_2-OCH_3$ | CN | H | F | O |
| $(CH_3)_2N$- | $F_2CH$- | H | $-O-CH(CH_3)-COOCH_3$ | CN | H | F | O |
| $(CH_3)_2N$- | $F_2CH$- | H | $-O-CH(CH_3)-C{\equiv}CH$ | CN | H | F | O |
| $(CH_3)_2N$- | $F_2CH$- | H | $-S-CH_2-COOCH_3$ | CN | H | F | O |
| $(CH_3)_2N$- | $F_2CH$- | H | $-CH_2-CN$ | CN | H | F | O |
| $(CH_3)_2N$- | $F_2CH$- | H | 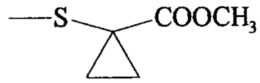 | CN | H | F | O |
| $(CH_3)_2N$- | $F_2CH$- | H | 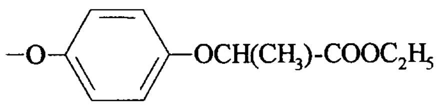 | CN | H | F | O |
| $(CH_3)_2N$- | $F_2CH$- | H | $-O-CH_2-Si(CH_3)_3$ | CN | H | F | O |
| $(CH_3)_2N$- | $F_2CH$- | H | 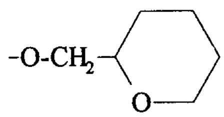 | CN | H | F | O |
| $(CH_3)_2N$- | $F_2CH$- | H | 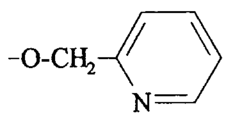 | CN | H | F | O |
| $(CH_3)_2N$- | $F_2CH$- | H | $-O-CHF_2$ | CN | H | F | O |
| $(CH_3)_2N$- | $F_2CH$- | H | $-COOCH_3$ | CN | H | Cl | O |
| $(CH_3)_2N$- | $F_2CH$- | H | $-CO-NH-CH_3$ | CN | H | Cl | O |
| $(CH_3)_2N$- | $F_2CH$- | H | $-CO-N(CH_3)_2$ | CN | H | F | O |
| $(CH_3)_2N$- | $F_2CH$- | H | $-CH_3$ | CN | H | F | O |
| $(CH_3)_2N$- | $F_2CH$- | H | Cl | Cl | H | Cl | O |

Verwendet man beispielsweise 4-Methyl-3-dimethylamino-1,2,4-triazolin-5-on und 2,4,5-Trifluorbenzonitril als Ausgangsstoffe, so läßt sich der Reaktionsablauf des erfindungsgemäßen Verfahrens (a) durch das folgende Formelschema darstellen:

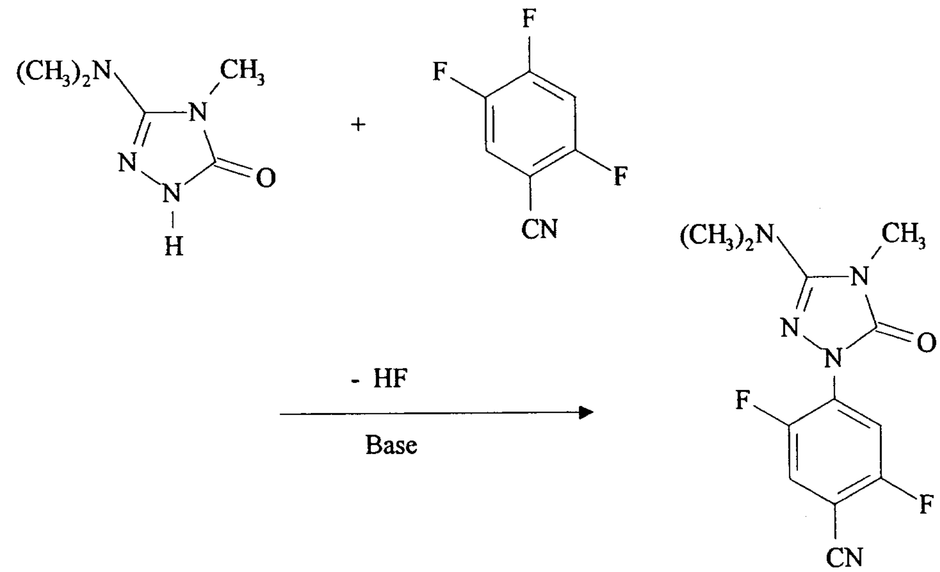


Verwendet man beispielsweise 1-(4-Cyano-2,5-difluorphenyl)-4-methyl-3-dimethylamino-1,2,4-triazolin-5-on und 1-Butin-3-ol als Ausgangsstoffe, so läßt sich der Reaktionsablauf des erfindungsgemäßen Verfahrens (b) durch das folgende Formelschema darstellen:

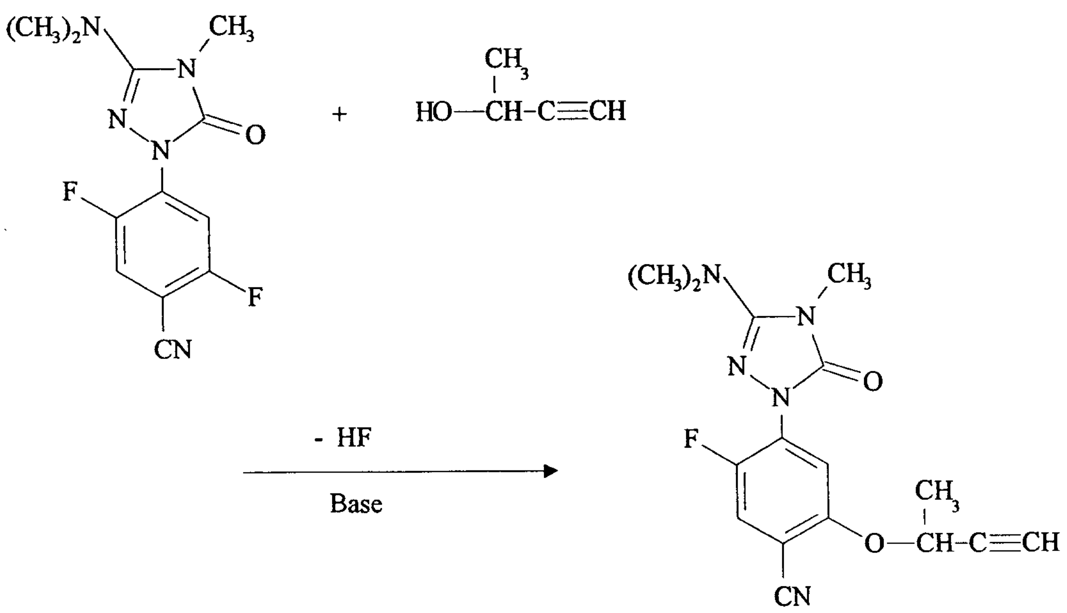


Verwendet man beispielsweise 1-(4-Cyano-2-fluor-5-methoxy-phenyl)-4-amino-3-dimethyamino-1,2,4-triazolin-5-on und Natriumnitrit als Ausgangsstoffe, so läßt sich der Reaktionsablauf des erfindungsgemäßen Verfahrens (c) durch das folgende Formelschema darstellen:

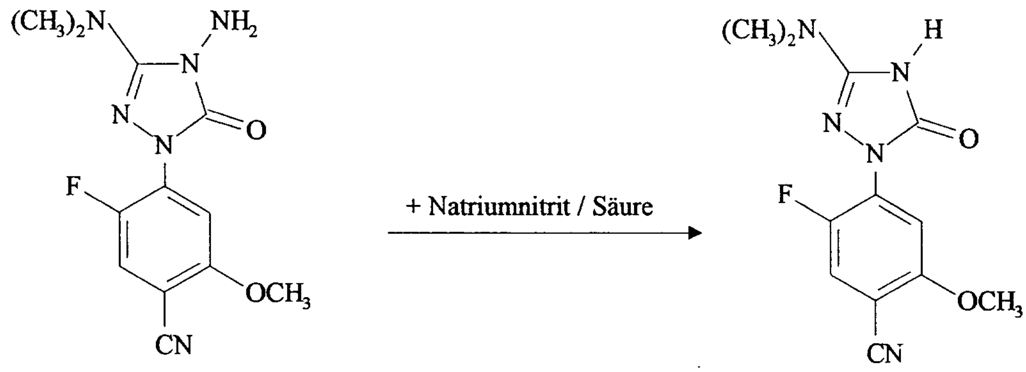


Verwendet man beispielsweise 1-(4-Cyano-2-fluor-5-methoxy-phenyl)-3-dimethylamino-(4H)-1,2,4-triazolin-5-on und Chlordifluormethan als Ausgangsstoffe, so läßt sich der Reaktionsablauf des erfindungsgemäßen Verfahrens (d) durch das folgende Formelschema darstellen:

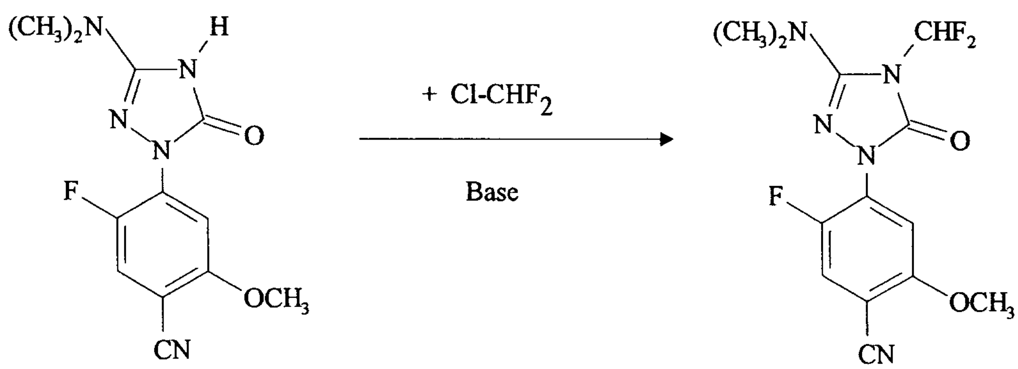


Verwendet man beispielsweise 2,6-Dichlor-4-trifluormethylphenylhydrazin und Chlortrimethylformamidiniumhydrochlorid als Ausgangsstoffe, so läßt sich der Reaktionsablauf des erfindungsgemäßen Verfahrens (e) durch das folgende Formelschema darstellen:

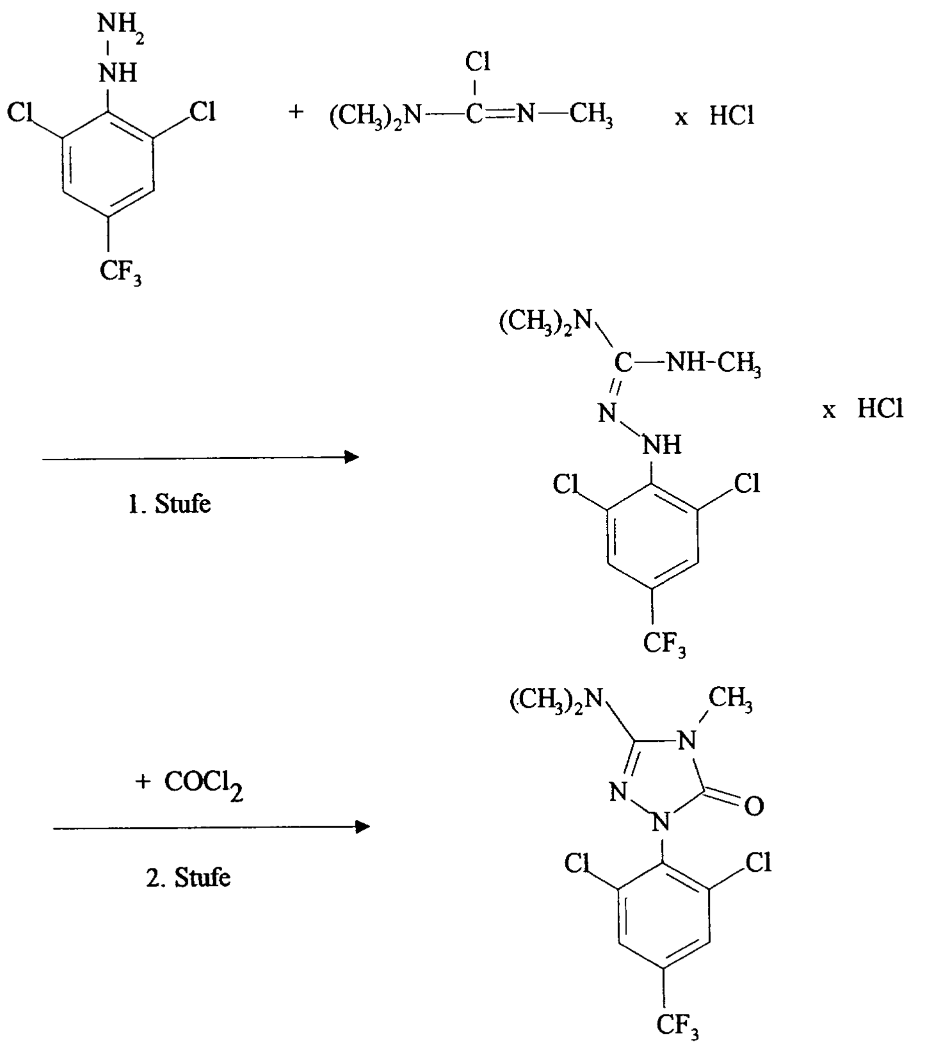


Die zur Durchführung des erfindungsgemäßen Verfahrens (a) als Ausgangsstoffe benötigten 1H-Triazolinone sind durch die Formel (II) allgemein definiert. In dieser Formel (II) stehen $R^1$, $R^2$ und X vorzugsweise und besonders bevorzugt für diejenigen Reste, die bereits im Zusammenhang mit der Beschreibung der erfindungsgemäßen Verbindungen der Formel (I) als bevorzugt und besonders bevorzugt für diese Substituenten genannt wurden.

Die 1H-Triazolinone der Formel (II) sind bekannt oder erhältlich in Analogie zu bekannten Verfahren (vergl. z.B. EP 283 876; EP 305 844; EP 513 621; EP 415 196; Chem. Ber. 102, 755-766 [1969]; Liebigs Ann. Chem. 343, 24 [1905]). Außerdem erhält man 1H-Triazolinone der Formel (II) nach bisher nicht bekanntem Vefahren, wenn man Chlor-formamidinium-Hydrochloride der Formel (VI),

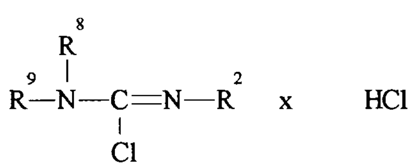
(VI)

in welcher

$R^2$, $R^8$ und $R^9$ die oben angegebenen Bedeutungen haben,

mit Carbazaten der Formel (IX),

$H_2N\text{-}NH\text{-}CO\text{-}O\text{-}R^1$ (IX)

in welcher

$R^1$ die oben angegebene Bedeutung hat,

gegebenenfalls in Gegenwart eines Verdünnungsmittels wie beispielsweise Acetonitril und gegebenenfalls in Gegenwart eines Reaktionshilfsmittels wie beispielsweise Natriumhydrogencarbonat bei Temperaturen zwischen -20 °C und +100 °C umsetzt (vergleiche hierzu auch die Herstellungsbeispiele).

Die zur Durchführung des erfindungsgemäßen Verfahrens (a) weiterhin als Ausgangsstoffe benötigten Halogenbenzol-Derivate sind durch die Formel (III) allgemein definiert. In dieser Formel (III) stehen $R^3$, $R^4$, $R^5$, $R^6$ und $R^7$ vorzugsweise und besonders bevorzugt für diejenigen Reste, die bereits im Zusammenhang mit der Beschreibung der erfindungsgemäßen Verbindungen der Formel (I) als bevorzugt und besonders bevorzugt für diese Substituenten genannt wurden. $Hal^1$ steht vorzugsweise für Fluor, Chlor oder Brom, insbesondere für Fluor oder Chlor.

Die Halogenbenzol-Derivate der Formel (III) sind allgemein bekannt oder erhältlich in Analogie zu bekannten Verfahren (vergl. z.B. EP 191 181; EP 441 004; EP 431 373). Noch nicht bekannt ist die Verbindung 5-Chlor-2,4-difluorbenzonitril. Man erhält sie, wenn man die bekannte Verbindung 2,4,5-Trichlorbenzonitril (vergl. z.B. EP 441 004) mit Kaliumfluorid gegebenenfalls in Gegenwart eines Verdünnungsmittels wie beispielsweise Tetramethylensulfon bei Temperaturen zwischen 100 °C und 200 °C umsetzt (vergleiche hierzu auch die Herstellungsbeispiele).

Die zur Durchführung des erfindungsgemäßen Verfahren (b) als Edukt benötigten substituierten Triazolinone sind durch die Formel (Ia) allgemein definiert. In dieser Formel (Ia) stehen $R^1$, $R^2$, $R^3$, $R^5$, $R^6$, $R^7$ und X vorzugsweise und besonders bevorzugt für diejenigen Reste, die bereits im Zusammenhang mit der Beschreibung der erfindungsgemäßen Stoffe der Formel (I) als bevorzugt und besonders bevorzugt für diese Substituenten genannt wurden. $Hal^2$ steht vorzugsweise für Fluor, Chlor oder Brom, insbesondere für Fluor oder Chlor.

Die substituierten Triazolinone der Formel (Ia) sind erfindungsgemäße Verbindungen und erhältlich mit Hilfe der erfindungsgemäßen Verfahren (a), (c), (d) und/oder (e).

Die zur Durchführung des erfindungsgemäßen Verfahren (b) weiterhin als Edukte benötigten Nukleophile sind durch die Formel (IV) allgemein definiert. In dieser Formel (IV) steht $R^{13}$ vorzugsweise für einen Rest der Formel $-O-R^{12}$, $-S-R^{12}$ oder $-NR^{11}R^{12}$, wobei $R^{11}$ und $R^{12}$ vorzugsweise für diejenigen Reste stehen, die bereits im Zusammenhang mit der Beschreibung der erfindungsgemäßen Stoffe der Formel (I) als bevorzugt und besonders bevorzugt für diese Substituenten genannt wurden. Die Nukleophile der Formel (IV) sind allgemein bekannte Verbindungen der organischen Chemie.

Die zur Durchführung des erfindungsgemäßen Verfahren (c) als Edukte benötigten substituierten Triazolinone sind durch die Formel (Ib) allgemein definiert. In dieser Formel (Ib) stehen $R^1$, $R^3$, $R^4$, $R^5$, $R^6$, $R^7$ und X vorzugsweise und besonders bevorzugt für diejenigen Reste, die bereits im Zusammenhang mit der Beschreibung der erfindungsgemäßen Stoffe der Formel (I) als bevorzugt und besonders bevorzugt für diese Substituenten genannt wurden.

Die substituierten Triazolinone der Formel (Ib) sind erfindungsgemäße Verbindungen und erhältlich mit Hilfe der erfindungsgemäßen Verfahren (a), (b) und/oder (e).

Die zur Durchführung des erfindungsgemäßen Verfahren (d) als Edukte benötigten substituierten Triazolinone sind durch die Formel (Ic) allgemein definiert. In dieser Formel (Ic) stehen $R^1$, $R^3$, $R^4$, $R^5$, $R^6$, $R^7$ und X vorzugsweise und besonders bevorzugt für diejenigen Reste, die bereits im Zusammenhang mit der Beschreibung der erfindungsgemäßen Stoffe der Formel (I) als bevorzugt und besonders bevorzugt für diese Substituenten genannt wurden.

Die substituierten Triazolinone der Formel (Ic) sind erfindungsgemäße Verbindungen und erhältlich mit Hilfe der erfindungsgemäßen Verfahren (a), (b), (c) und/oder (e).

Die zur Durchführung des erfindungsgemäßen Verfahren (d) weiterhin als Edukte benötigten Alkylierungsmittel sind durch die Formel (V) allgemein definiert. In dieser Formel (V) steht $R^{2-1}$ vorzugsweise und besonders bevorzugt für diejenigen Reste, die bereits im Zusammenhang mit der Beschreibung der erfindungsgemäßen Stoffe der Formel (I) als bevorzugt und besonders bevorzugt für den Substituenten $R^{10}$ genannt wurden, mit Ausnahme der gegebenenfalls substituierten Aryl- bzw. Heterocyclylreste. $E^1$ steht vorzugsweise für einen bei Alkylierungsmitteln üblichen Abgangsrest, wie beispielsweise Halogen, insbesondere für Chlor, Brom oder Iod oder für jeweils gegebenenfalls substituiertes Alkylsulfonyloxy, Alkoxysulfonyloxy oder Arylsulfonyloxy, wie insbesondere Methansulfonyloxy, Trifluormethansulfonyloxy, Methoxysulfonyloxy, Ethoxysulfonyloxy oder p-Toluolsulfonyloxy.

Die Alkylierungsmittel der Formel (V) sind allgemein bekannte Verbindungen der organischen Chemie.

Die zur Durchführung des erfindungsgemäßen Verfahren (e) als Edukte benötigten Chlor-formamidiniumhydrochloride sind durch die Formel (VI) allgemein definiert. In dieser Formel (VI) stehen $R^2$, $R^8$ und $R^9$ vorzugsweise und besonders bevorzugt für diejenigen Reste, die bereits im Zusammenhang mit der

Beschreibung der erfindungsgemäßen Stoffe der Formel (I) als bevorzugt und besonders bevorzugt für diese Substituenten genannt wurden. Die Chlor-formamidiniumhydrochloride der Formel (VI) sind bekannt oder erhältlich in Analogie zu bekannten Verfahren (vergl. z.B. EP 283 876; EP 398 097; Chem. Ber. 97, 1232 [1964]).

Die zur Durchführung des erfindungsgemäßen Verfahren (e) weiterhin als Edukte benötigten Arylhydrazine sind durch die Formel (VII) allgemein definiert. In dieser Formel (VII) stehen $R^3$, $R^4$, $R^5$, $R^6$ und $R^7$ vorzugsweise und besonders bevorzugt für diejenigen Reste, die bereits im Zusammenhang mit der Beschreibung der erfindungsgemäßen Stoffe der Formel (I) als bevorzugt und besonders bevorzugt für diese Substituenten genannt wurden

Die Arylhydrazine der Formel (VII) sind allgemein bekannte Verbindungen der organischen Chemie, oder erhältlich in Analogie zu bekannten Verfahren (vergl. z.B. Houben-Weyl "Methoden der organischen Chemie", Thieme Verlag Stuttgart, Band X/2, S. 180, 201, 245; Angew. Chem. 80, 284 [1968]; Chem. Ber. 102, 3088 [1969]; DE 33 37 543; DE 34 02 308; DE 34 47 211; DE 39 03 799).

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid oder Ester, wie Essigsäuremethylester oder Essigsäureethylester.

Das erfindungsgemäße Verfahren (a) wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydroxide, wie Natriumhydroxid, Calciumhydroxid, Kaliumhydroxid oder auch Ammoniumhydroxid, Alkalimetallcarbonate, wie Natriumcarbonat, Kaliumcarbonat, Kaliumhydrogencarbonat, Natriumhydrogencarbonat oder Ammoniumcarbonat, Alkali- oder Erdalkalimetallacetate, wie Natriumacetat, Kaliumacetat, Calciumacetat oder Ammoniumacetat, sowie tertiäre Amine, wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen 0 °C und +180 °C, vorzugsweise bei Temperaturen zwischen +20 °C und +120 °C.

Das erfindungsgemäße Verfahren (a) wird üblicherweise unter Normaldruck durchgeführt. Es ist jedoch auch möglich unter erhöhtem oder vermindertem Druck zu arbeiten.

Zur Durchführung des erfindungsgemäßen Verfahrens (a) setzt man pro Mol 1H-Triazolinon der Formel (II) im allgemeinen 1,0 bis 3,0 Mol, vorzugsweise 1,0 bis 1,5 Mol Halogenbenzol-Derivat der Formel (III) und gegebenenfalls 1,0 bis 3,0 Mol, vorzugsweise 1,0 bis 1,5 Mol Base als Reaktionshilfsmittel ein. Die Reaktionsdurchführung, Aufarbeitung und Isolierung der Reaktionsprodukte erfolgt nach allgemein üblichen, bekannten Verfahren (vergleiche auch die Herstellungsbeispiele).

Als Verdünnungsmittel zur Durchführung des erfindungsgemäßen Verfahrens (b) kommen inerte organische Lösungsmittel infrage. Vorzugsweise verwendet man die bei der Beschreibung der Durchführung des erfindungsgemäßen Verfahrens (a) aufgezählten Lösungsmittel.

Das erfindungsgemäße Verfahren (b) wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man bei Temperaturen zwischen -20 °C und +150 °C, vorzugsweise bei Temperaturen zwischen 0 °C und +120 °C.

Das erfindungsgemäße Verfahren (b) wird üblicherweise unter Normaldruck durchgeführt. Es ist jedoch auch möglich unter erhöhtem oder vermindertem Druck zu arbeiten.

Zur Durchführung des erfindungsgemäßen Verfahrens (b) setzt man pro Mol substituiertem Triazolinon der Formel (Ia) im allgemeinen 1,0 bis 3,0 Mol, vorzugsweise 1,0 bis 1,5 Mol Nukleophil der Formel (IV) und gegebenenfalls 0,1 bis 3,0 Mol, vorzugsweise 1,0 bis 1,5 Mol Base als Reaktionshilfsmittel ein.
Die Reaktionsdurchführung, Aufarbeitung und Isolierung der Reaktionsprodukte erfolgt nach allgemein üblichen, bekannten Verfahren (vergl. auch die Herstellungsbeispiele).

Das erfindungsgemäße Verfahren (c) wird üblicherweise in Gegenwart einer geeigneten Säure durchgeführt. Als solche kommen insbesondere wässrige Mineralsäuren infrage. Mit besonderem Vorzug verwendet man verdünnte Salzsäure.

Als Verdünnungsmittel zur Durchführung des erfindungsgemäßen Verfahrens (c) kommen alle für derartige Diazotierungsreaktionen üblichen Verdünnungsmittel in Betracht. Mit besonderem Vorzug verwendet man die als Reagenzien eingesetzten wässrigen Mineralsäuren, wie beispielsweise Salzsäure in entsprechendem Überschuß gleichzeitig als Verdünnungsmittel.

Die Reaktionstemperaturen können bei der Durchführung des erfindungsgemäßen Verfahrens (c) in einem größeren Bereich variiert werden. Im allgemeinen arbeitet man bei Temperaturen zwischen -20 °C und +100 °C, vorzugsweise bei Temperaturen zwischen -10 °C und +80 °C.

Das erfindungsgemäße Verfahren (c) wird üblicherweise unter Normaldruck durchgeführt. Es ist jedoch auch möglich unter erhöhtem oder vermindertem Druck zu arbeiten.

Zur Durchführung des erfindungsgemäßen Verfahrens (c) setzt man pro Mol substituiertem Triazolinon der Formel (Ib) im allgemeinen 1,0 bis 3,0 Mol, vorzugsweise 1,0 bis 2,0 Mol Natriumnitrit und 1,0 bis 10,0 Mol, vorzugsweise 1,0 bis 5,0 Mol Säure ein.
Die Reaktionsdurchführung Aufarbeitung und Isolierung der Reaktionsprodukte erfolgt nach allgemein üblichen, bekannten Verfahren (vergleiche auch die Herstellungsbeispiele).

Als Verdünnungsmittel zur Durchführung des erfindungsgemäßen Verfahrens (d) kommen inerte organische Lösungsmittel infrage. Hierzu gehören insbesondere aliphatische, alicyclische oder aromatische gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutylketon; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester oder Sulfoxide, wie Dimethylsulfoxid.

Das erfindungsgemäße Verfahren (d) kann gegebenenfalls auch in einem Zweiphasensystem, wie beispielsweise Wasser/Toluol oder Wasser/Dichlormethan, gegebenenfalls in Gegenwart eines geeigneten Phasentransferkatalysators, durchgeführt werden. Als Beispiele für solche Katalysatoren seien genannt: Tetrabutylammoniumiodid, Tetrabutylammoniumbromid, Tetrabutylammoniumchlorid, Tributyl-methylphosphoniumbromid, Trimethyl-$C_{13}$/$C_{15}$-alkylammoniumchlorid, Trimethyl-$C_{13}$/$C_{15}$-alkylammoniumbromid, Dibenzyl-dimethyl-ammoniummethylsulfat, Dimethyl-$C_{12}$/$C_{14}$-alkyl-benzylammoniumchlorid, Dimethyl-$C_{12}$/$C_{14}$-alkyl-benzylammoniumbromid, Tetrabutylammoniumhydroxid, Triethylbenzylammoniumchlorid, Methyltrioctylammoniumchlo rid, Trimethylbenzylammoniumchlorid, 15-Krone-5, 18-Krone-6 oder Tris-[2-(2-methoxyethoxy)-ethyl]-amin.

Das erfindungsgemäße Verfahren (d) wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (d) in einem größeren Bereich variiert werden. Im allgemeinen arbeitet man bei Temperaturen zwischen -20 °C und +150 °C, vorzugsweise bei Temperaturen zwischen 0 °C und +120 °C.

Das erfindungsgemäße Verfahren (d) wird üblicherweise unter Normaldruck durchgeführt. Es ist jedoch auch möglich unter erhöhtem oder vermindertem Druck zu arbeiten.

Zur Durchführung des erfindungsgemäßen Verfahrens (d) setzt man pro Mol substituiertem Triazolinon der Formel (Ic) im allgemeinen 1,0 bis 3,0 Mol, vorzugsweise 1,0 bis 2,0 Mol Alkylierungsmittel der Formel (V) und gegebenenfalls 1,0 bis 3,0 Mol, vorzugsweise 1,0 bis 2,0 Mol Base als Reaktionshilfsmittel ein.
Die Reaktionsdurchführung, Aufarbeitung und Isolierung der Reaktionsprodukte erfolgt in beiden Fällen

nach allgemein üblichen, bekannten Verfahren (vergleiche auch die Herstellungsbeispiele).

Als Verdünnungsmittel zur Durchführung der 1. Stufe des erfindungsgemäßen Verfahrens (e) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid, Ester, wie Essigsäuremethylester oder Essigsäureethylester oder Alkohole, wie Methanol, Ethanol, Propanol oder Butanol.

Die Reaktionstemperaturen können bei der Durchführung der 1. Stufe des erfindungsgemäßen Verfahrens (e) in einem größeren Bereich variiert werden. Im allgemeinen arbeitet man bei Temperaturen zwischen -20 °C und +100 °C, vorzugsweise bei Temperaturen zwischen 0 °C und +80 °C.

Die 1. Stufe des erfindungsgemäßen Verfahrens (e) wird üblicherweise unter Normaldruck durchgeführt. Es ist jedoch auch möglich unter erhöhtem oder vermindertem Druck zu arbeiten.

Zur Durchführung der 1. Stufe des erfindungsgemäßen Verfahrens (e) setzt man pro Mol Chlorformamidinium-Hydrochlorid der Formel (VI) im allgemeinen 0,1 bis 1,0 Mol, vorzugsweise 0,4 bis 0,6 Mol Arylhydrazin der Formel (VIII) ein. Die Reaktionsdurchführung, Aufarbeitung und Isolierung der Reaktionsprodukte erfolgt nach allgemein üblichen, bekannten Verfahren (vergleiche hierzu auch die Herstellungsbeispiele).

Als Verdünnungsmittel zur Durchführung der 2. Stufe des erfindungsgemäßen Verfahrens (e) kommen ebenfalls inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid oder Ester, wie Essigsäuremethylester oder Essigsäureethylester.

Die Reaktionstemperaturen können bei der Durchführung der 2. Stufe des erfindungsgemäßen Verfahrens (e) in einem größeren Bereich variiert werden. Im allgemeinen arbeitet man bei Temperaturen zwischen -20 °C und +100 °C, vorzugsweise bei Temperaturen zwischen 0 °C und +80 °C.

Die 2. Stufe des erfindungsgemäßen Verfahrens (e) wird üblicherweise unter Normaldruck durchgeführt. Es ist jedoch auch möglich unter erhöhtem oder vermindertem Druck zu arbeiten.

Zur Durchführung der 2. Stufe des erfindungsgemäßen Verfahrens (e) setzt man pro Mol Zwischenprodukt der Formel (VIII) im allgemeinen 2,0 bis 20,0 Mol vorzugsweise 10,0 bis 15,0 Mol Phosgen ein. Die Reaktionsdurchführung, Aufarbeitung und Isolierung der Reaktionsprodukte erfolgt nach allgemein üblichen, bekannten Verfahren (vergleiche hierzu auch die Herstellungsbeispiele).

Die Reinigung der Endprodukte der Formel (I) erfolgt mit Hilfe üblicher Verfahren, beispielsweise durch Säulenchromatographie, oder durch Umkristallisieren.

Die Charakterisierung erfolgt mit Hilfe des Schmelzpunktes oder bei nicht kristallisierenden Verbindungen mit Hilfe der Protonen-Kernresonanzspektroskopie ($^1$H-NMR).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera. Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Zitrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung von dikotylen Unkräutern in monokotylen Kulturen wie beispielsweise Weizen oder Mais einsetzen. Daneben besitzen die erfindungsgemäßen Wirstoffe in entsprechenden Aufwandmengen auch blattinsektizide und akarizide Wirksamkeit.

Die Wirkstoffe können in Abhängigkeit von ihren jeweiligen physikalischen und/oder chemischen Eigenschaften in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen. Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infragen: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zinn verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba oder Picloram; Aryloxyalkansäuren, wie z.B. 2,4-D, 2,4-DB, 2,4-DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofopethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin, Diphenylether, wie z.B.

Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazolsulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können dabei als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen; Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro Hektar.

Die Herstellung und die Verwendung der erfindungsgemäßen Wirkstoffe geht aus den nachfolgenden Beispielen hervor.

Herstellungsbeispiele:

Beispiel 1:

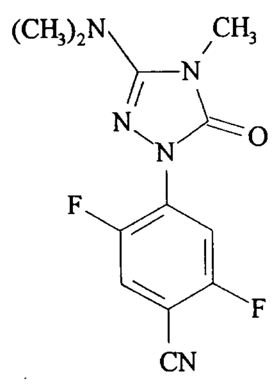

(Verfahren a)

7,1 g (0,05 Mol) 3-Dimethylamino-4-methyl-1H-1,2,4-triazolin-5-on (vergl. z.B. EP 283 876) und 7,85 g (0,05 Mol) 2,4,5-Trifluorbenzonitril (vergl. z.B. EP 191 181) werden in 100 ml Dimethylsulfoxid bei Raumtemperatur mit 8,3 g (0,06 Mol) Kaliumcarbonat versetzt und anschließend eine Stunde bei 80 °C bis 90 °C gerührt. Zur Aufarbeitung wird die abgekühlte Reaktionsmischung in 300 ml Wasser gegeben, der ausgefallenene Feststoff abfiltriert, mit Wasser gewaschen und getrocknet.

Man erhält 9,3 g (67 % der Theorie) 1-(4-Cyano-2,5-difluorphenyl)-4-methyl-3-dimethylamino-1,2,4-triazolin-5-on vom Schmelzpunkt 131-133 °C.

Beispiel 2:

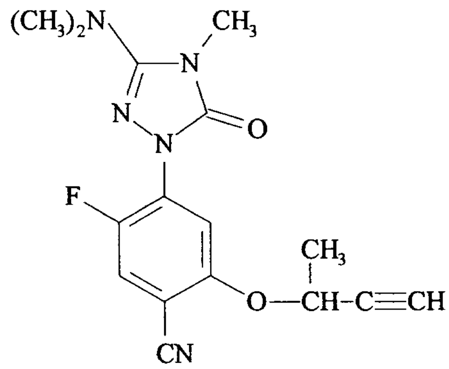

(Verfahren b)

Zu 0,42 g (0,006 Mol) 3-Butin-1-ol in 50 ml Acetonitril gibt man bei Raumtemperatur 0,24 g (0,006 Mol) Natriumhydrid (60% in Paraffinöl), rührt 10 Minuten bei Raumtemperatur, gibt anschließend 1,2 g (0,0043 Mol) 1-(4-Cyano-2,5-difluorphenyl)-4-methyl-3-dimethylamino-1,2,4-triazolin-5-on zu und rührt weitere 2 Stunden bei Raumtemperatur. Zur Aufarbeitung wird die Reaktionsmischung filtriert, das Filtrat im Vakuum eingeengt, der Rückstand zwischen Dichlormethan und Wasser verteilt, die organische Phase abgetrennt, über Natriumsulfat getrocknet, im Vakuum eingeengt und durch Verrühren mit wenig Methanol zur Kristallisation gebracht.

Man erhält 0,7 g (50 % der Theorie) 1-(4-Cyano-2-fluor-5-but-1-in-3-yl-oxy-phenyl)-4-methyl-3-dimethylamino-1,2,4-triazolin-5-on vom Schmelzpunkt 112-114 °C.

Beispiel 3:

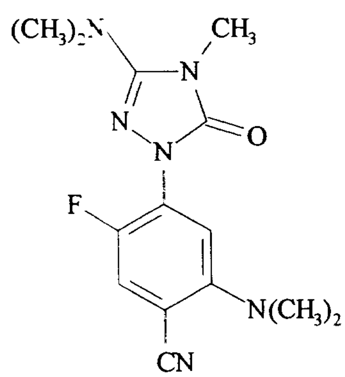

(Verfahren b)

2,2 g (0,008 Mol) 1-(4-Cyano-2,5-difluorphenyl)-4-methyl-3-dimethylamino-1,2,4-triazolin-5-on und 0,65 g (0,008 Mol) Dimethylamin-Hydrochlorid werden in 80 ml Dimethylsulfoxid mit 2,2 g (0,016 Mol) Kaliumcarbonat versetzt und anschließend 20 Stunden bei 80 °C gerührt. Zur Aufarbeitung wird die abgekühlte Reaktionsmischung filtriert, das Filtrat im Vakuum eingeengt, der Rückstand zwischen Dichlormethan und Wasser verteilt, die organische Phase abgetrennt, über Natriumsulfat getrocknet und im Vakuum eingeengt.

Man erhält 0,9 g (37 % der Theorie) 1-(4-Cyano-2-fluor-5-dimethylamino-phenyl)-4-methyl-3-dimethylamino-1,2,4-triazolin-5-on vom Schmelzpunkt 109-110 °C.

Beispiel 4:

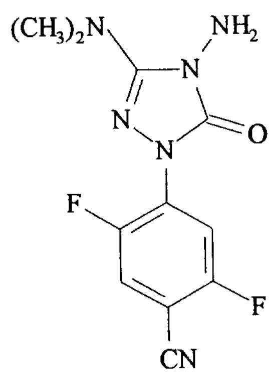

(Verfahren a)

28,6 g (0,2 Mol) 3-Dimethylamino-4-amino-1H-1,2,4-triazolin-5-on (vergl. z.B. EP 415 196) und 31,4 g (0,05 Mol) 2,4,5-Trifluorbenzonitril (vergl. z.B. EP 191 181) werden in 200 ml Dimethylsulfoxid bei Raumtemperatur mit 27,6 g (0,2 Mol) Kaliumcarbonat versetzt und anschließend eine Stunde bei 50 °C und weitere zwei Stunden bei 120 °C gerührt. Zur Aufarbeitung wird die abgekühlte Reaktionsmischung in Wasser gegeben, der ausgefallene Feststoff abgesaugt, nacheinander mit Wasser und Isopropanol gewaschen und getrocknet.

Man erhält 49 g (87,5 % der Theorie) 1-(4-Cyano-2,5-difluorphenyl)-4-amino-3-dimethylamino-1,2,4-triazolin-5-on vom Schmelzpunkt 154-155 °C.

Beispiel 5:

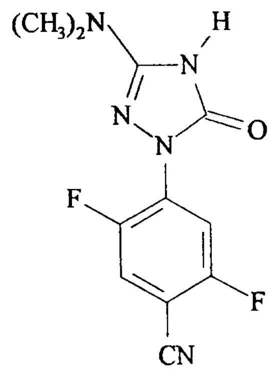

(Verfahren c)

Zu 14 g (0,05 Mol) 1-(4-Cyano-2,5-difluorphenyl)-4-amino-3-dimethylamino-1,2,4-triazolin-5-on in 200 ml Essigsäure gibt man bei Raumtemperatur tropfenweise unter Rühren eine Lösung von 3,5 g (0,05 Mol) Natriumnitrit in 200 ml Wasser rührt nach beendeter Zugabe 10 weitere Minuten bei Raumtemperatur und erwärmt dann langsam unter Rühren bis auf 80 °C. Zur Aufarbeitung wird die abgekühlte Reaktionsmischung abfiltriert, der Rückstand nacheinander mit Wasser und Ethanol gewaschen und getrocknet.

Man erhält 11 g (83 % der Theorie) 1-(4-Cyano-2,5-difluorphenyl)-3-dimethylamino-4H-1,2,4-triazolin-5-on vom Schmelzpunkt >250 °C.

Beispiel 6:

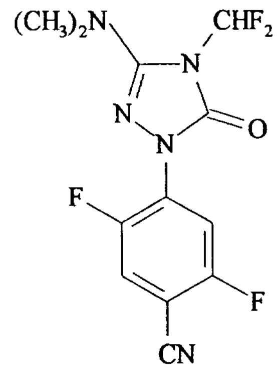

(Verfahren d)

In eine Suspension aus 8,75 g (0,033 Mol) 1-(4-Cyano-2,5-difluorphenyl)-3-dimethylamino-4H-1,2,4-triazolin-5-on, 3,4 g (0,06 Mol) Kaliumcarbonat und 0,6 g Tetrabutylammmoniumbromid in 200 ml Tetrahydrofuran leitet man bei 0 °C bis 10 °C innerhalb von 5 Stunden 80 g (0,9 Mol) Chlordifluormethan ein und ergänzt nach zwei Stunden den Basenverbrauch durch Zugabe von 1,7 g (0,03 Mol) Kaliumhydroxid. Zur Aufarbeitung wird die Reaktionsmischung in Wasser gegeben, mehrfach mit Dichlormethan extrahiert, die vereinigten organischen Phasen über Natriumsulfat getrocknet, im Vakuum eingeengt und der Rückstand aus Isopropanol umkristallisiert.

Man erhält 6,8 g (65 % der Theorie) 1-(4-Cyano-2,5-difluorphenyl)-3-dimethylamino-4-difluormethyl-1,2,4-triazolin-5-on vom Schmelzpunkt 188-190 °C.

Beispiel 7:

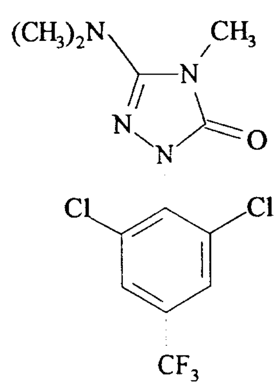

(Verfahren e / 2.Stufe)

18,3 g (0,05 Mol) 1-(2,6-Dichlor-4-trifluormethylphenylamino)-2,2,3-trimethylguanidinium-Hydrochlorid werden in 200 ml Acetonitril unter Einleiten von Phosgen langsam auf Rückflußtemperatur erwärmt, wobei ab ca 60 °C eine Chlorwasserstoffentwicklung einsetzt. Man leitet insgesamt 80 g (0,8 Mol) Phosgen ein und rührt nach beendeter Einleitung bis zur Beendigung der Chlorwasserstoffentwicklung bei Rückflußtemperatur. Zur Aufarbeitung wird die abgekühlte Reaktionsmischung im Vakuum eingeengt, der Rückstand mit Essigester verrührt, filtriert, das Filtrat abermals im Vakuum eingeengt und der Rückstand aus Cyclohexan umkristallisiert.

Man erhält 13 g (73 % der Theorie) 1-(2,6-Dichlor-4-trifluormethylphenyl)-3-dimethylamino-4-methyl-1,2,4-triazolin-5-on vom Schmelzpunkt 105-107 °C.

Herstellung der Ausgangsverbindungen:

Beispiel VIII-1:

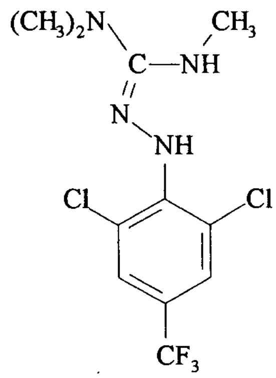

x HCl

(Verfahren e / 1.Stufe)

Zu 49 g (0,2 Mol) 2,6-Dichlor-4-trifluormethylphenylhydrazin (vergl. z.B. EP 187 285) in 300 ml Isopropanol gibt man bei Raumtemperatur (maximal 30 °C) tropfenweise unter Rühren eine Lösung von 15,7 g (0,1 Mol) Chlortrimethylformamidinium-Hydrochlorid (vergl. z.B. EP 283 876) in 100 ml Isopropanol, rührt nach beendeter Zugabe 30 weitere Minuten bei Raumtemperatur. Zur Aufarbeitung wird die Reaktionsmischung filtriert, das Filtrat im Vakuum eingeengt, der Rückstand durch Verrühren mit Essigester zur Kristallisation gebracht, abfiltriert und getrocknet.

Man erhält 29 g (80 % der Theorie) 1-(2,6-Dichlor-4-trifluormethylphenylamino)-2,2,3-trimethylguanidinium-Hydrochlorid vom Schmelzpunkt 236-238 °C.

Beispiel II-1:

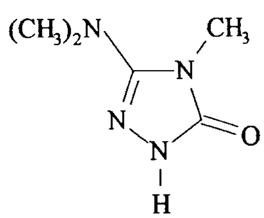

Zu 411 g (3,95 Mol) Ethylcarbazat und 996 g (11,85 Mol) Natriumhydrogencarbonat in 2.000 ml Acetonitril gibt man unter Rühren bei Raumtemperatur (bis maximal 30 °C) tropfenweise eine Lösung von 620 g (3,95 Mol) Chlortrimethylformamidinium-Hydrochlorid (vergl. z.B. EP 283 876) in 1.600 ml Acetonitril, erwärmt nach beendeter Zugabe langsam auf Rückflußtemperatur und rührt weitere 10 Stunden bei Rückflußtemperatur. Zur Aufarbeitung wird die abgekühlte Reaktionsmischung filtriert, das Filtrat unter vermindertem Druck eingeengt und im Hochvakuum destilliert.

Man erhält 480 g (85,5 % der Theorie) 3-Dimethylamino-4-methyl-1H-1,2,4-triazolin-5-on vom Siedepunkt 178-180 °C bei 4 mbar und vom Schmelzpunkt 78-80 °C.

In entsprechender Weise und gemäß den allgemeinen Angaben zur Herstellung erhält man die folgenden 1H-Triazolinone der allgemeinen Formel (II):

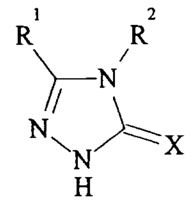

(II)

| Bsp.-Nr. | $R^1$ | $R^2$ | X | physikalische Eigenschaften |
|---|---|---|---|---|
| II-2 | $(CH_3)_2N$- | i-$C_3H_7$ | O | Fp. 125-126°C |
| II-3 | 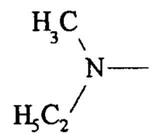 | $CH_3$ | O | Fp. 69-70°C |
| II-4 | $(CH_3)_2N$- | $C_2H_5$ | O | Fp. 58-59°C |
| II-5 | $(C_2H_5)_2N$- | $CH_3$ | O | Fp. 84-85°C |
| II-6 | 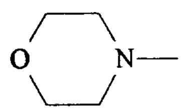 | $CH_3$ | O | Fp. 172-173°C |

*) Die $^1H$-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Beispiel III-1:

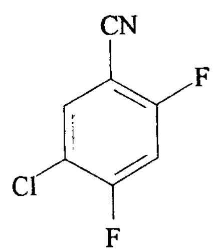

Zu 250 g (4,31 Mol) Kaliumfluorid in 400 ml destilliertem Tetramethylensulfon gibt man unter Rühren bei Raumtemperatur 220 g (1,06 Mol) 2,4,5-Trichlorbenzonitril (vergl. z.B. EP 441 004) und rührt anschließend 10 Stunden bei 195 °C bis 200 °C. Zur Aufarbeitung wird abgekühlt, 500 ml Wasser zugegeben und die Mischung einer Wasserdampfdestillation unterzogen. Der organische Anteil wird in Dichlormethan aufgenommen, über Natriumsulfat getrocknet, im Vakuum eingeengt und destilliert.

Man erhält 108 g (58 % der Theorie) an 2,4-Difluor-5-chlorbenzonitril vom Siedepunkt 105-107 °C bei 30 mbar und vom Schmelzpunkt 48-50 °C.

In entsprechender Weise und gemäß den allgemeinen Angaben zur Herstellung erhält man die folgenden substituierten Triazolinone der allgemeinen Formel (I):

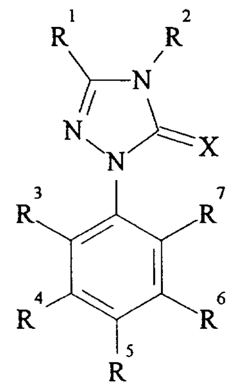

(I)

Tabelle II

| **Bsp. Nr.** | 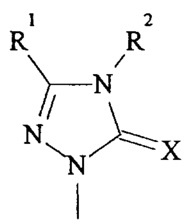 | 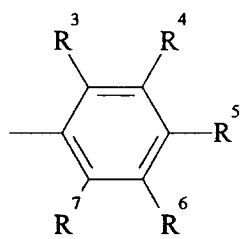 | **physikalische Eigenschaften** |
|---|---|---|---|
| 8 | 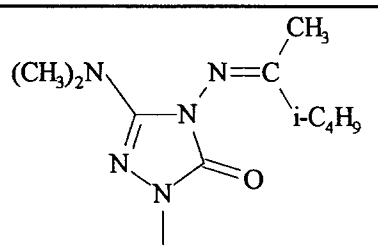 | 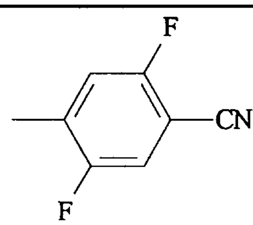 | Fp. 133-134°C |
| 9 | 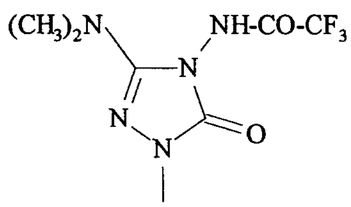 | 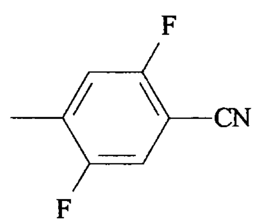 | Fp. 165-166°C |

| Bsp. Nr. | 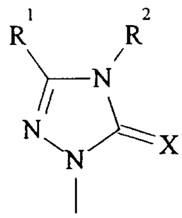 | 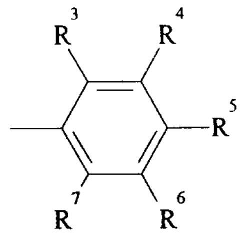 | **physikalische Eigenschaften** |
|---|---|---|---|
| 10 | 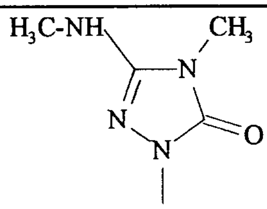 | 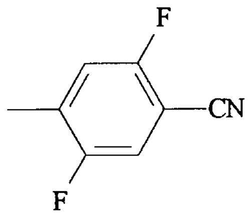 | Fp. >250°C |
| 11 | 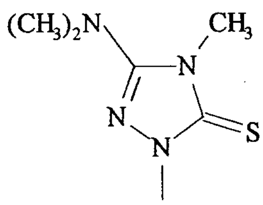 | 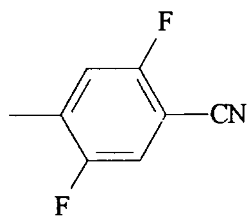 | Fp. 138°C |
| 12 | 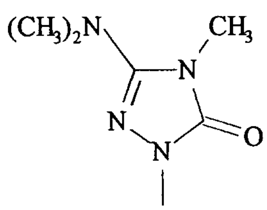 | 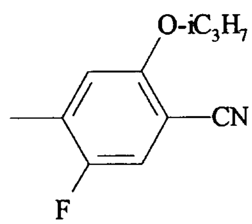 | Fp. 230°C |
| 13 | 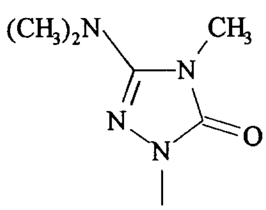 | 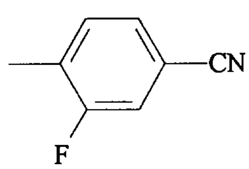 | Fp. 110°C |
| 14 | 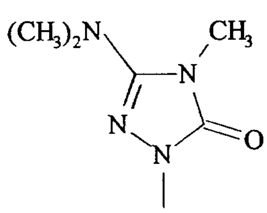 | 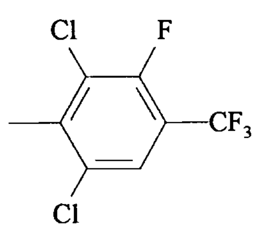 | ¹H-NMR*): 2,77; 3,35; 7,67-7,69 |

| Bsp. Nr. | 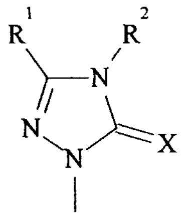 | 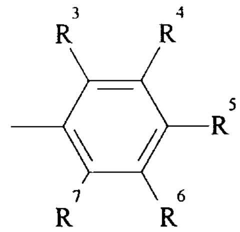 | physikalische Eigenschaften |
|---|---|---|---|
| 15 | 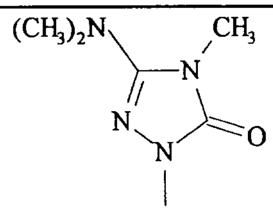 | 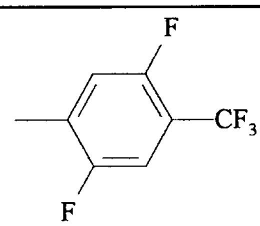 | $^1$H-NMR*): 2,78; 3,35; 7,4-7,55 |
| 16 | 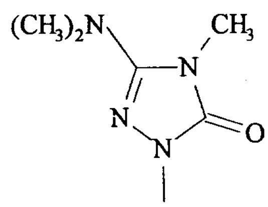 | 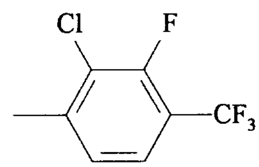 | Fp. 95-96°C |
| 17 | 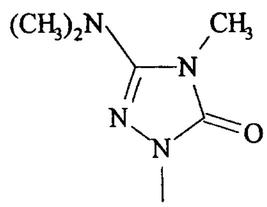 | 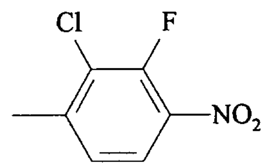 | Fp. 230°C |
| 18 | 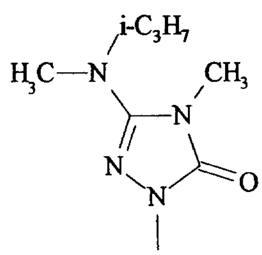 | 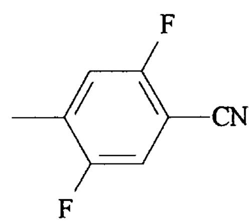 | Fp. 107-109°C |
| 19 | 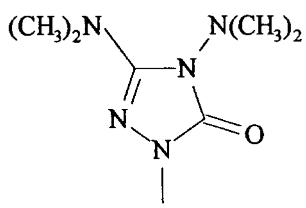 | 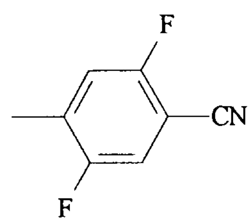 | Fp. 84-88°C |

| Bsp. Nr. | 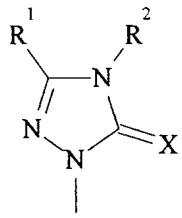 | 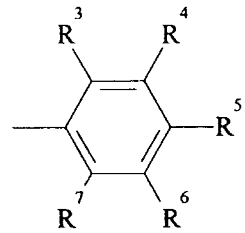 | physikalische Eigenschaften |
|---|---|---|---|
| 20 | 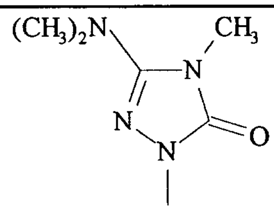 | 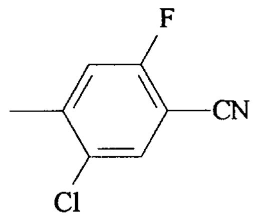 | Fp. 125-126°C |
| 21 | 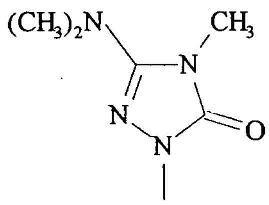 | 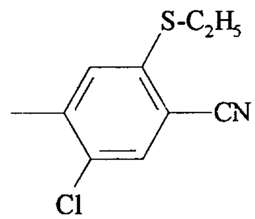 | Fp. 142-143°C |
| 22 | 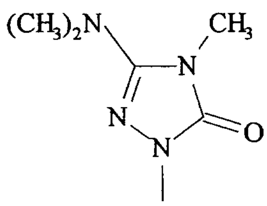 | 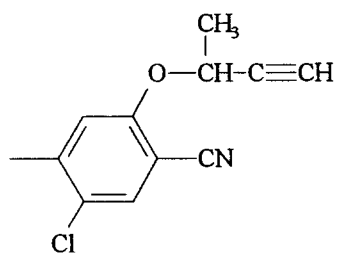 | Fp. 147-148°C |
| 23 | 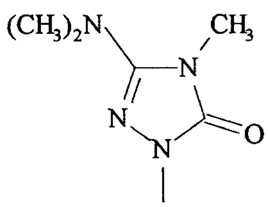 | 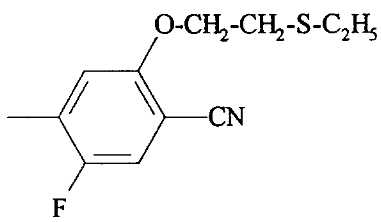 | ¹H-NMR*): 1,28-1,35; 2,78; 4,22-4,25 |
| 24 | 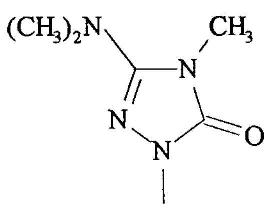 | 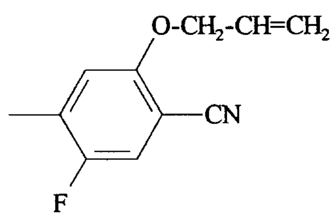 | Fp. 122-123°C |

| Bsp. Nr. | 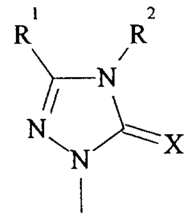 | 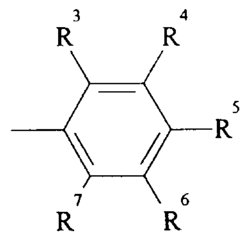 | physikalische Eigenschaften |
|---|---|---|---|
| 25 | 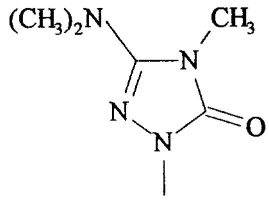 | 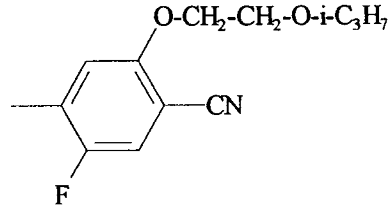 | Fp. 64-65°C |
| 26 | 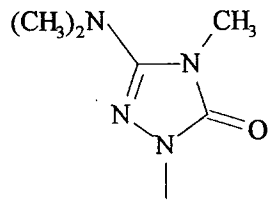 | 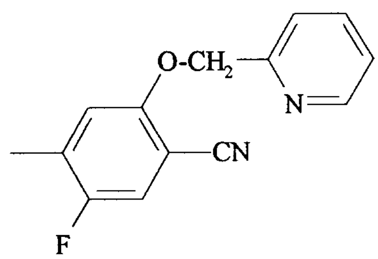 | Fp. 142-143°C |
| 27 | 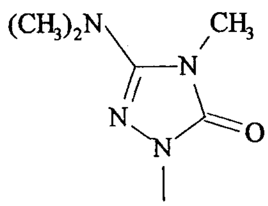 | 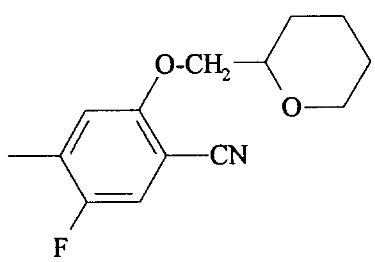 | 1H-NMR*): 2,78; 3,48-3,53; 3,72-3,78 |
| 28 | 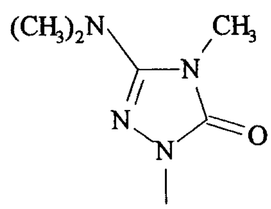 | 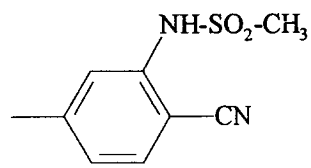 | Fp. 236-237°C |
| 29 | 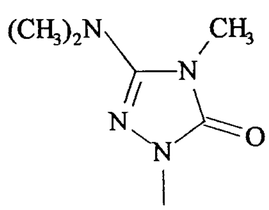 | 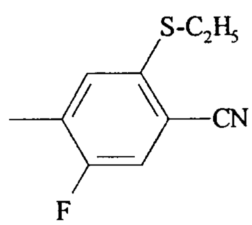 | Fp. 123-124°C |

| **Bsp. Nr.** | 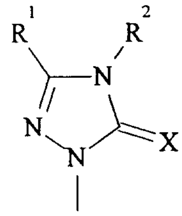 | 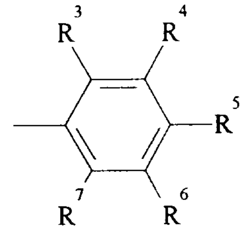 | **physikalische Eigenschaften** |
|---|---|---|---|
| 30 | 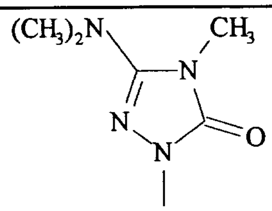 | 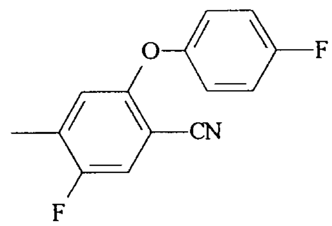 | Fp. 163-164°C |
| 31 | 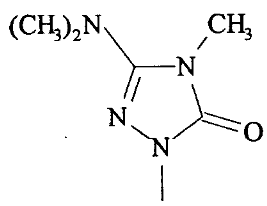 | 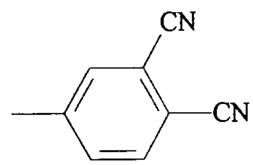 | Fp. 188-189°C |
| 32 | 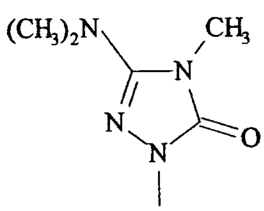 | 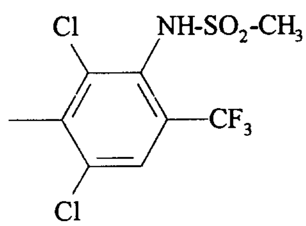 | Fp. >250°C |
| 33 | 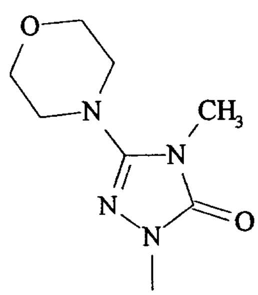 | 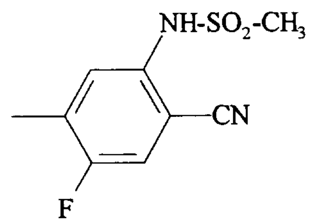 | $^1$H-NMR*): 3,12; 3,20-3,25; 3,30; 3,85-3,90 |
| 34 | 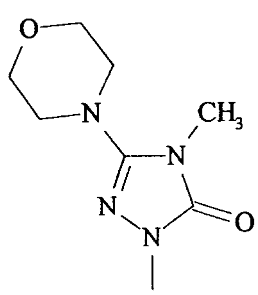 | 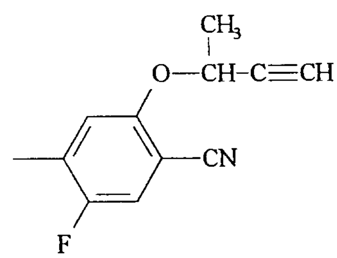 | Fp. 148-150°C |

| Bsp. Nr. | 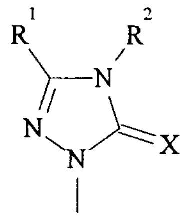 | 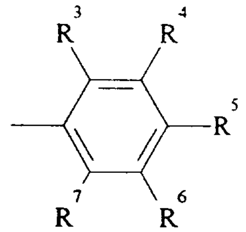 | physikalische Eigenschaften |
|---|---|---|---|
| 35 | 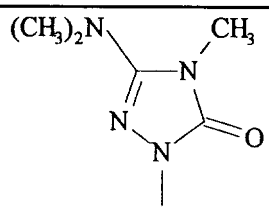 | 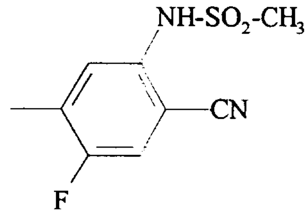 | Fp. 200-202°C |
| 36 | 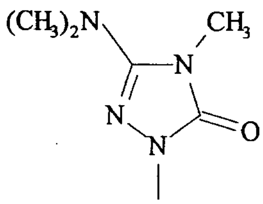 | 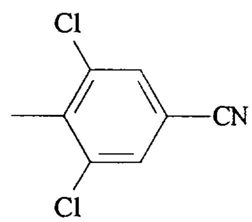 | Fp. 157-158°C |
| 37 | 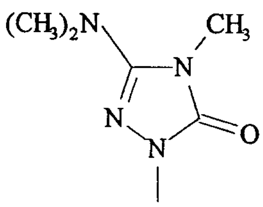 | 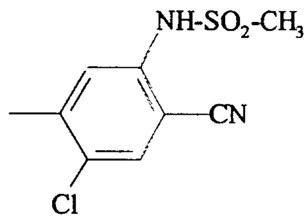 | Fp. 210-211°C |
| 38 | 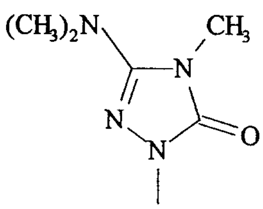 | 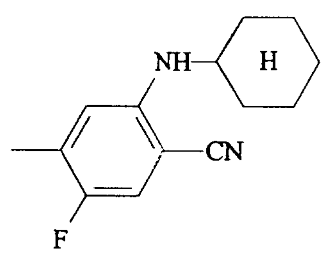 | Fp. 153-154°C |
| 39 | 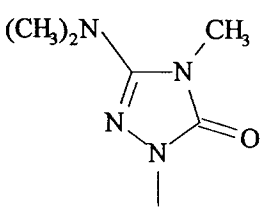 | 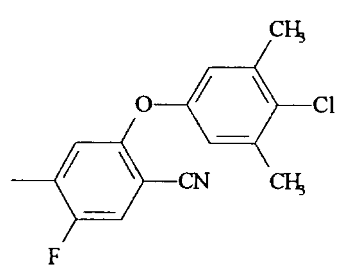 | Fp. 172-173°C |

| Bsp. Nr. | 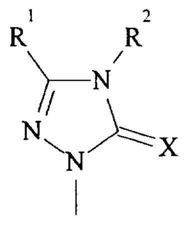 | 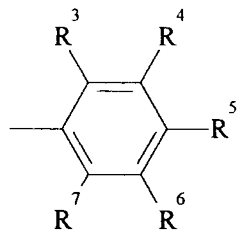 | **physikalische Eigenschaften** |
|---|---|---|---|
| 40 | 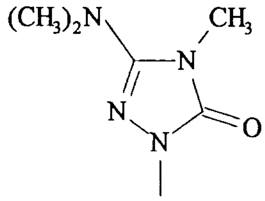 | 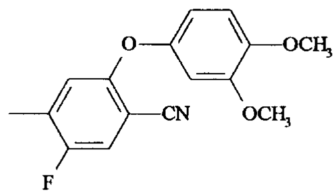 | Fp. 135-136°C |
| 41 | 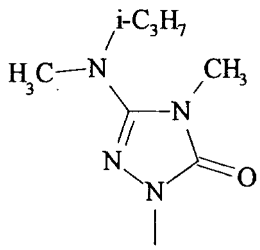 | 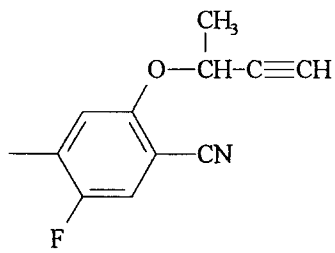 | Fp. 250°C |
| 42 | 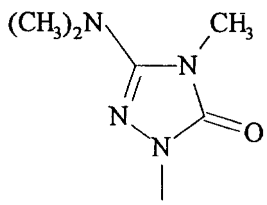 | 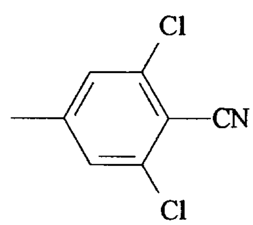 | Fp. 157-158°C |
| 43 | 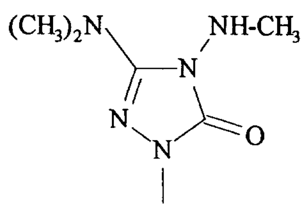 | 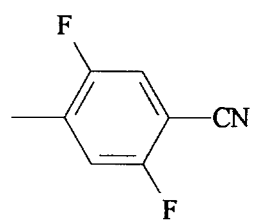 | Fp. 157-158°C |
| 44 | 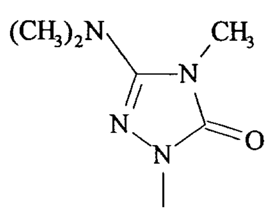 | 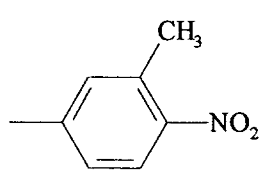 | Fp. 135-136°C |

| **Bsp. Nr.** | 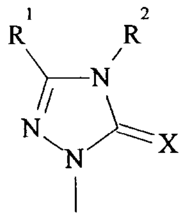 | 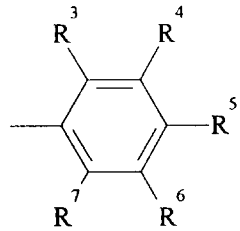 | **physikalische Eigenschaften** |
|---|---|---|---|
| 45 | 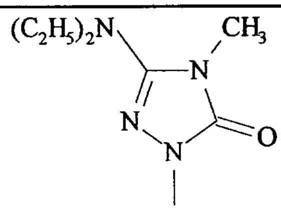 | 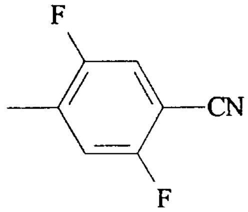 | Fp. 87-88°C |
| 46 | 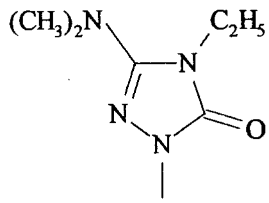 | 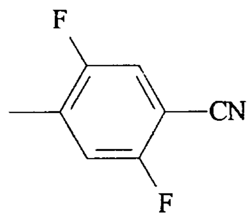 | Fp. 108-109°C |
| 47 | 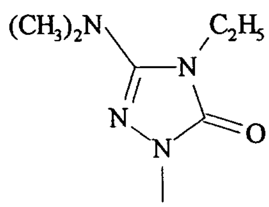 | 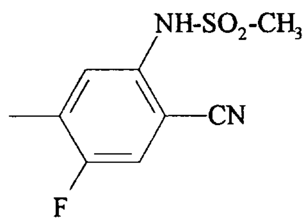 | Fp. 182-183°C |
| 48 | 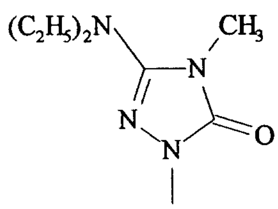 | 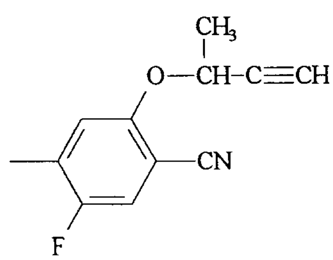 | Fp. 99-101°C |
| 49 | 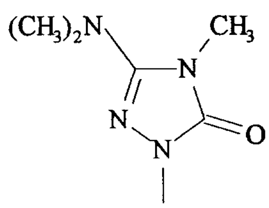 | 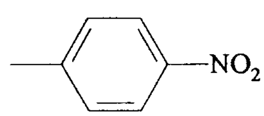 | Fp. 130-131°C |

| Bsp. Nr. | 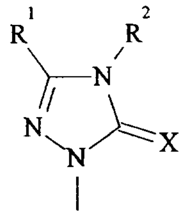 | 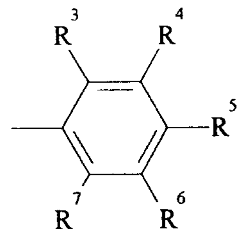 | physikalische Eigenschaften |
|---|---|---|---|
| 50 | 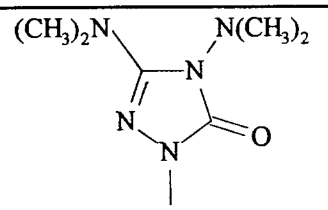 | 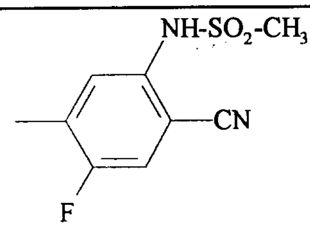 | Fp. 167-168°C |
| 51 | 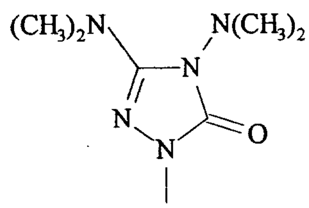 | 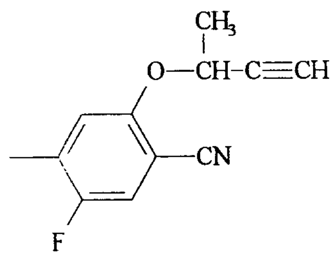 | Fp. 108-109°C |
| 52 | 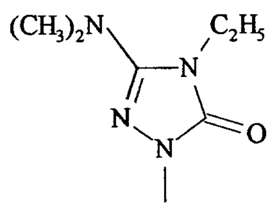 | 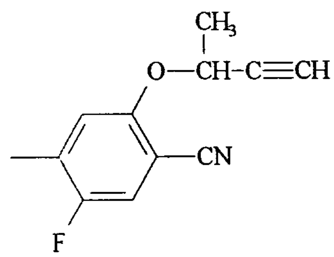 | Fp. 103-104°C |
| 53 | 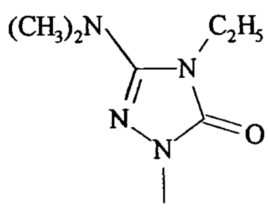 | 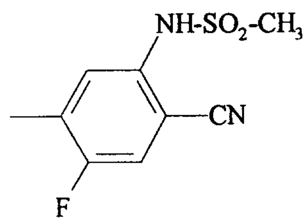 | Fp. 234-235°C |
| 54 | 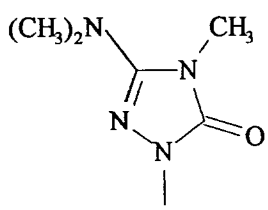 | 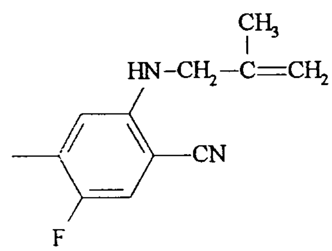 | Fp. 168-169°C |

| Bsp. Nr. | 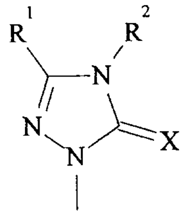 | 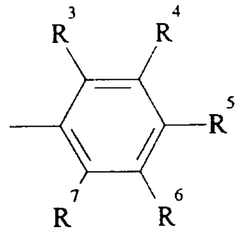 | physikalische Eigenschaften |
|---|---|---|---|
| 55 | 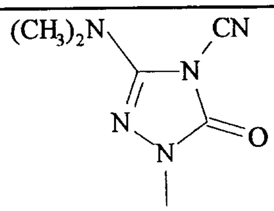 | 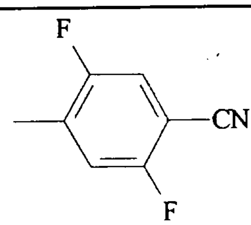 | Fp. 151-152°C |
| 56 | 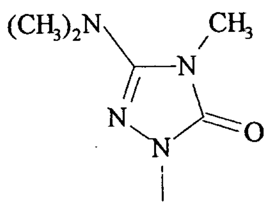 | 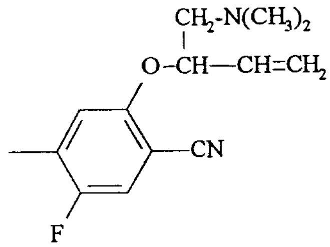 | $^{1}$H-NMR*): 2,37; 2,88; 3,30 |
| 57 | 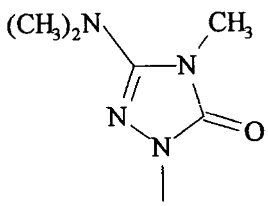 | 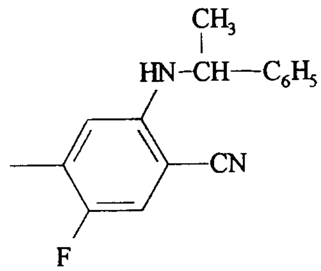 | Fp. 191-192°C |
| 58 | 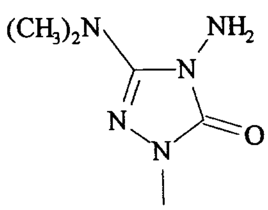 | 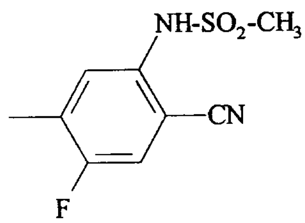 | Fp. 259-261°C |
| 59 | 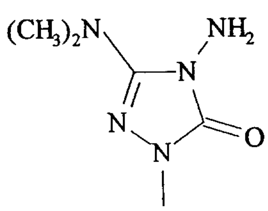 | 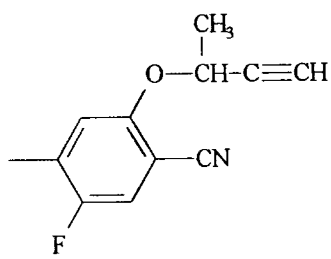 | Fp. 240-241°C |

| **Bsp. Nr.** | 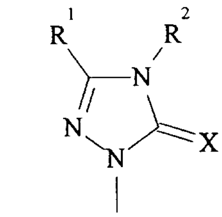 | 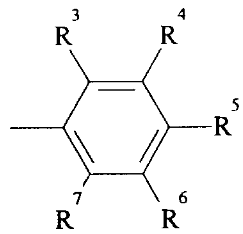 | **physikalische Eigenschaften** |
|---|---|---|---|
| 60 | 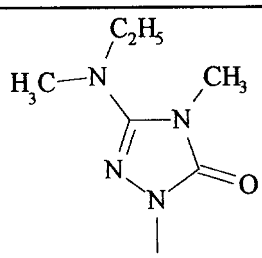 | 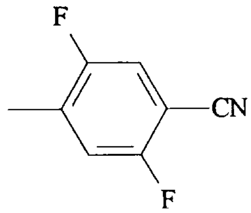 | Fp. 77-78°C |
| 61 | 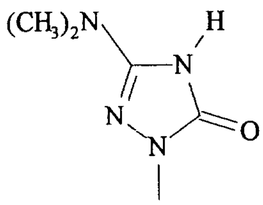 | 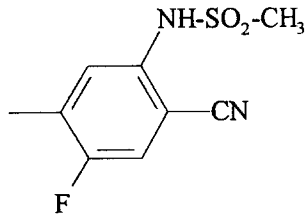 | Fp. >250°C |
| 62 | 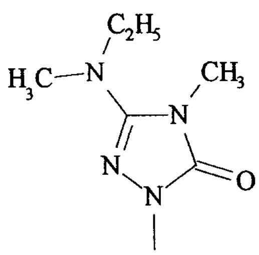 | 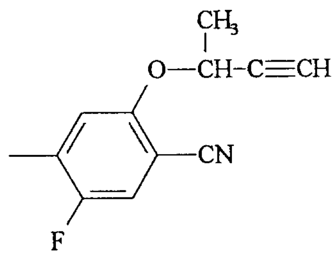 | Fp. 107-108°C |
| 63 | 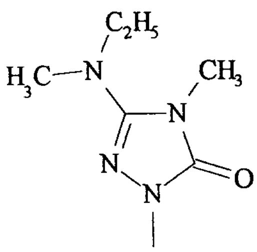 | 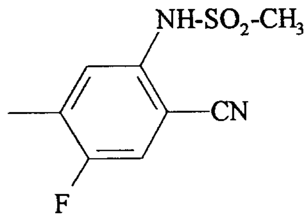 | Fp. 180-181°C |
| 64 | 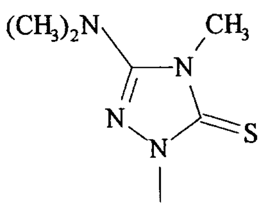 | 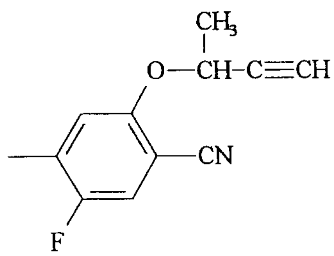 | $^1$H-NMR*): 1,75-178; 2,60; 2,92; 3,60 |

| Bsp. Nr. | 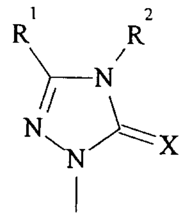 | 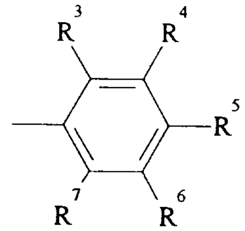 | physikalische Eigenschaften |
|---|---|---|---|
| 65 | 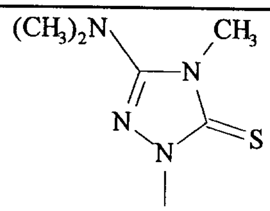 | 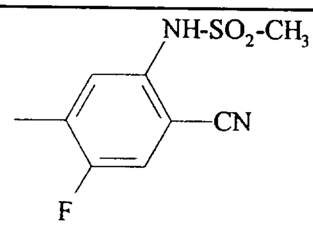 | Fp. 195-196°C |
| 66 | 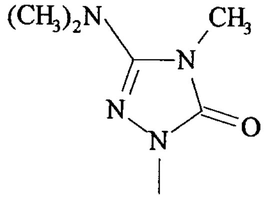 | 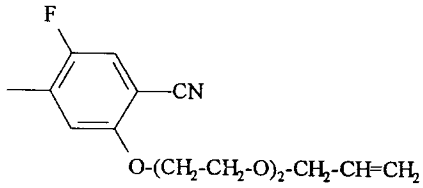 | $^1$H-NMR*): 1,21-1,25; 2,85; 5,85-5.98 |
| 67 | 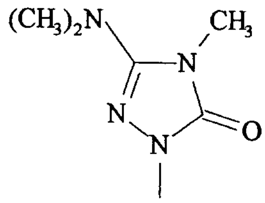 | 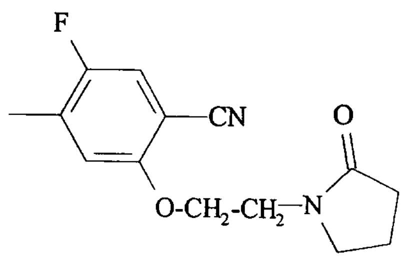 | Fp. 146-147°C |
| 68 | 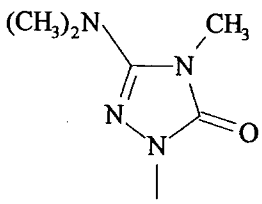 | 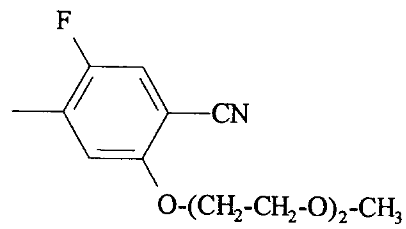 | Fp. 78-80°C |
| 69 | 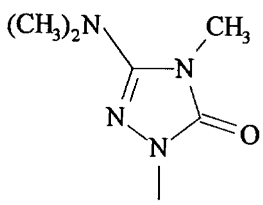 | 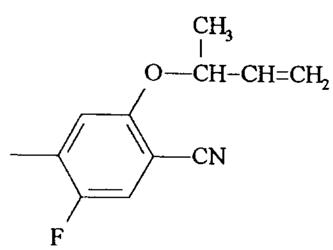 | Fp. 84-85°C |

| Bsp. Nr. | 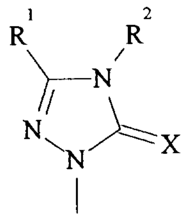 | 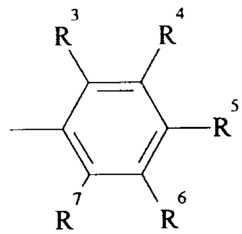 | physikalische Eigenschaften |
|---|---|---|---|
| 70 | 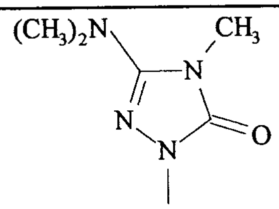 | 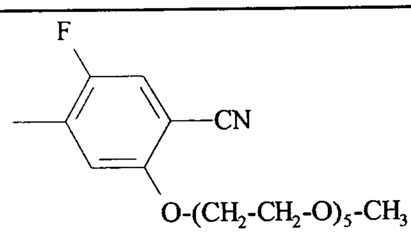 | 1H-NMR*): 1,22-1,26; 2,88; 4,22-4,25 |
| 71 | 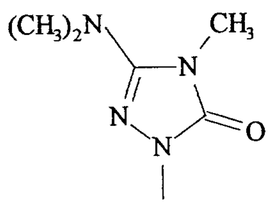 | 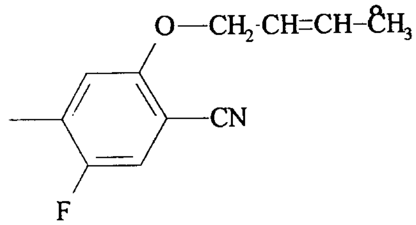 | Fp. 94-95°C |
| 72 | 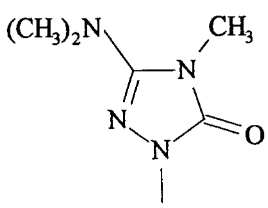 | 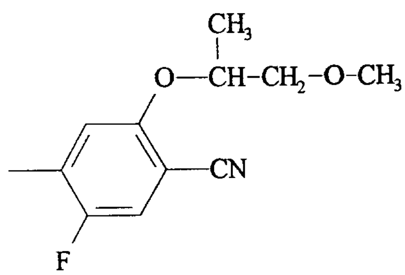 | Fp. 88-90°C |
| 73 | 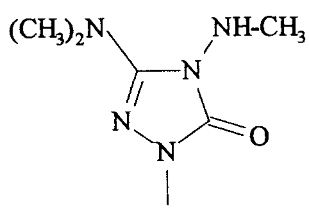 | 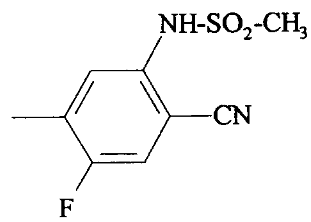 | Fp. 171-173°C |
| 74 | 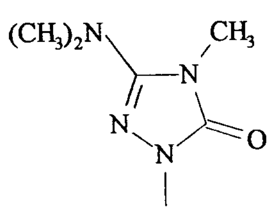 | 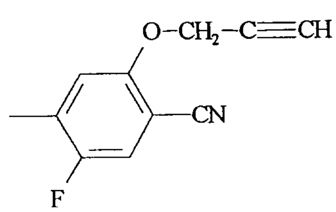 | Fp. 187-188°C |

| Bsp. Nr. | 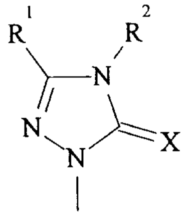 | 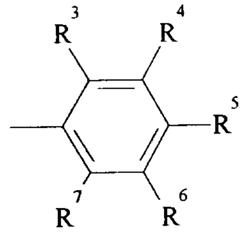 | physikalische Eigenschaften |
|---|---|---|---|
| 75 | 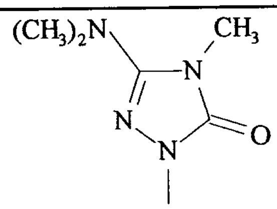 | 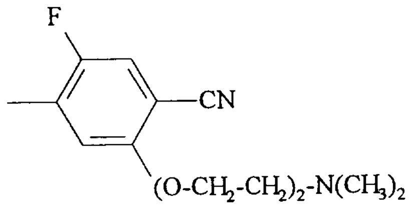 | $^1$H-NMR*): 2,29; 2,90; 4,33-4,36 |
| 76 | 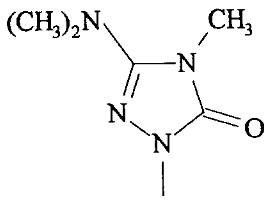 | 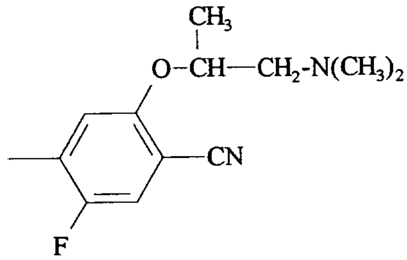 | $^1$H-NMR*): 1,35-1,38; 2,33; 2,90; 4,50-4,60 |
| 77 | 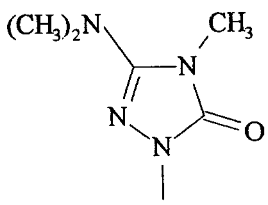 | 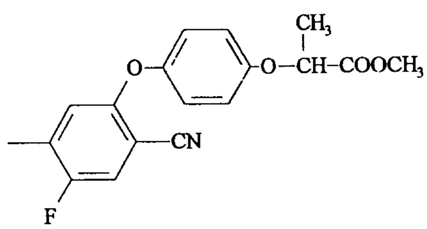 | $^1$H-NMR*): 2,80; 3,25; 4,20-4,30; 4,70-4,76 |
| 78 | 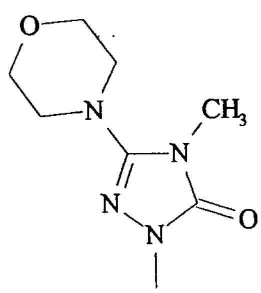 | 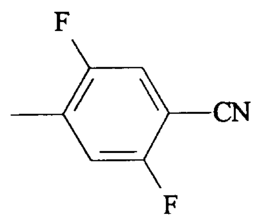 | Fp. 164-165°C |
| 79 | 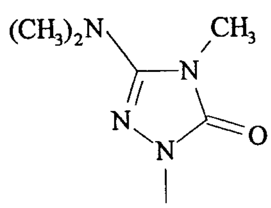 | 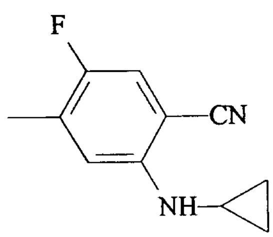 | Fp. 214-215°C |

| Bsp. Nr. | 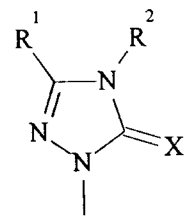 | 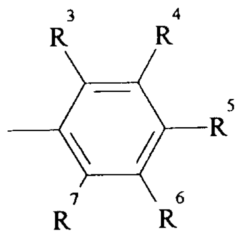 | physikalische Eigenschaften |
|---|---|---|---|
| 80 | 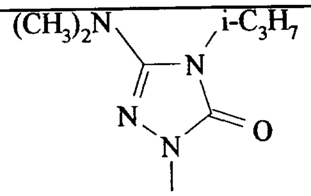 | 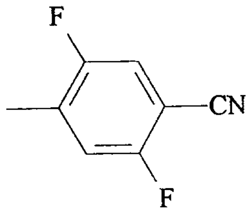 | Fp. 123°C |
| 81 | 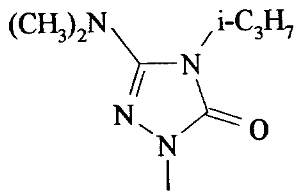 | 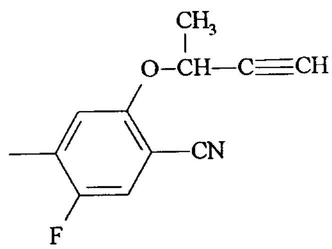 | Fp. 84°C |
| 82 | 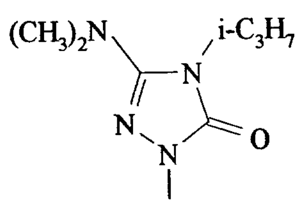 | 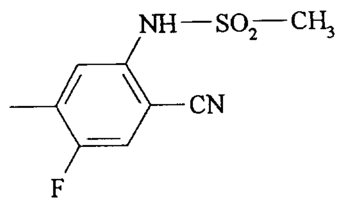 | Fp. 84°C |

*) Die $^{1}$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

## Anwendungsbeispiele:

In dem folgenden Anwendungsbeispiel wurde die nachstehend aufgeführte Verbindung als Vergleichssubstanz eingesetzt:

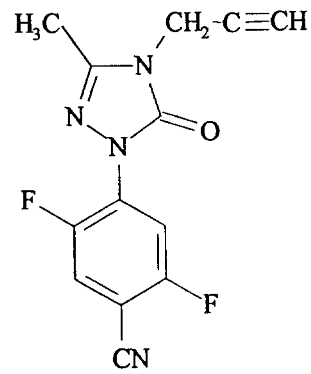

(A)

3-Methyl-4-propargyl-1-(2,5-difluor-4-cyano-phenyl)-1,2,4-triazolin-5-on
(bekannt aus DE 38 39 480)

Beispiel A:

**Pre-emergence-Test**

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man ein Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffes pro Flächeneinheit. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

Eine deutliche Überlegenheit in der Wirksamkeit gegenüber dem Stand der Technik zeigen in diesem Test z.B. die Verbindungen gemäß den Herstellungsbeispielen 1 und 2.

**Patentansprüche**

**1.** Neue 1-Aryltriazolin(thi)one der allgemeinen Formel (I),

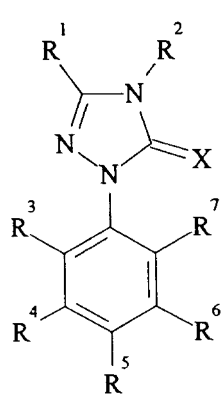

(I)

in welcher
$R^1$ für Nitro oder für einen Rest $-NR^8R^9$ steht,

$R^2$ für Wasserstoff, Amino, Cyano oder für einen der Reste $-R^{10}$, $-O-R^{10}$, $-S-R^{10}$, $-NR^{10}R^{11}$, $-N(R^{11})-CO-R^{10}$ oder $-N=CR^{10}R^{11}$ steht,

$R^3$, $R^6$ und $R^7$ unabhängig voneinander jeweils für Wasserstoff, Halogen oder Nitro stehen,

$R^4$ für Wasserstoff, Halogen, Cyano, Nitro, oder für einen der Reste $-R^{12}$, $-O-R^{12}$, $-S-R^{12}$, $-S(O)-R^{12}$, $-SO_2-R^{12}$, $-SO_2-OR^{12}$, $-SO_2-NR^{11}R^{12}$, $-CO-OR^{12}$, $-CO-NR^{11}R^{12}$, $-O-SO_2-R^{12}$, $-N(R^{11})-SO_2-R^{12}$, $-NR^{11}R^{12}$, $-NH-P(O)(R^{11})(OR^{12})$ oder $-NH-P(O)(OR^{11})-(OR^{12})$ steht,

$R^5$ für Nitro, Cyano, Halogen oder Halogenalkyl steht und

X für Sauerstoff oder Schwefel steht, wobei

$R^8$ und $R^9$ unabhängig voneinander jeweils für Wasserstoff oder für jeweils gegebenenfalls substituiertes Alkyl, Alkenyl, Alkinyl, Alkoxy, Cycloalkyl, Aryl oder Heterocyclyl stehen oder gemeinsam mit dem Stickstoffatom, an welches sie gebunden sind, für einen gegebenenfalls substituierten Heterocyclus stehen,

$R^{10}$ für jeweils gegebenenfalls substituiertes Alkyl, Alkenyl, Alkinyl, Cycloalkyl, Aryl, Arylalkyl oder Heterocyclyl steht,

$R^{11}$ für Wasserstoff oder für jeweils gegebenenfalls substituiertes Alkyl, Alkenyl, Alkinyl, Cycloalkyl, Arylalkyl oder Aryl steht und

$R^{12}$ für Wasserstoff oder für jeweils gegebenenfalls substituiertes Alkyl, Alkenyl, Alkinyl, Cycloalkyl, Aryl oder Heterocyclyl steht.

**2.** Neue 1-Aryltriazolin(thi)one der allgemeinen Formel (I) gemäß Anspruch 1, dadurch gekennzeichnet daß

$R^1$ für Nitro oder für einen Rest $-NR^8R^9$ steht,

$R^2$ für Wasserstoff, Amino, Cyano oder für einen der Reste $-R^{10}$, $-O-R^{10}$, $-S-R^{10}$, $-NR^{10}R^{11}$, $-N(R^{11})-CO-R^{10}$ oder $-N=CR^{10}R^{11}$ steht,

$R^3$, $R^6$ und $R^7$ unabhängig voneinander jeweils für Wasserstoff, Fluor, Chlor, Brom, Iod oder Nitro stehen,

$R^4$ für Wasserstoff, Fluor, Chlor, Brom, Iod, Cyano, Nitro, oder für einen der Reste $-R^{12}$, $-O-R^{12}$, $-S-R^{12}$, $-S(O)-R^{12}$, $-SO_2-R^{12}$, $-SO_2-OR^{12}$, $-SO_2-NR^{11}R^{12}$, $-CO-OR^{12}$, $-CO-NR^{11}R^{12}$, $-O-SO_2-R^{12}$, $-N(R^{11})-SO_2-R^{12}$, $-NR^{11}R^{12}$, $-NH-P(O)(R^{11})(OR^{12})$ oder $-NH-P(O)(OR^{11})(OR^{12})$ steht,

$R^5$ für Nitro, Cyano, Fluor, Chlor, Brom, Iod oder für geradkettiges oder verzweigtes Halogenalkyl mit 1 bis 6 Kohlenstoffatomen und 1 bis 13 gleichen oder verschiedenen Halogenatomen steht und

X für Sauerstoff oder Schwefel steht, wobei

$R^8$ und $R^9$ unabhängig voneinander jeweils für Wasserstoff stehen;

$R^8$ und $R^9$ außerdem für jeweils gegebenenfalls einfach oder mehrfach, gleich oder verschieden substituiertes, geradkettiges oder verzweigtes Alkyl oder Alkoxy mit jeweils 1 bis 14 Kohlenstoffatomen stehen, wobei als Substituenten jeweils infrage kommen:

Halogen - insbesondere Fluor, Chlor, Brom und/oder Iod -, Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl oder Alkylsulfonylaminocarbonyl mit jeweils 1 bis 8 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff Sauerstoff und/ oder Schwefel - steht;

$R^8$ und $R^9$ außerdem für jeweils gegebenenfalls einfach oder mehrfach, gleich oder verschieden durch Halogen - insbesondere Fluor, Chlor, Brom und/oder Iod - substituiertes Alkenyl oder Alkinyl mit jeweils 2 bis 8 Kohlenstoffatomen stehen;

$R^8$ und $R^9$ außerdem für gegebenenfalls einfach oder mehrfach, gleich oder verschieden durch Halogen - insbesondere Fluor, Chlor, Brom und/oder Iod - und/oder geradkettiges oder verzweigtes Alkyl mit 1 bis 4 Kohlenstoffatomen substituiertes Cycloalkyl mit 3 bis 7 Kohlenstoffatomen stehen;

$R^8$ und $R^9$ außerdem für jeweils gegebenenfalls im Arylteil einfach oder mehrfach, gleich oder verschieden substituiertes Arylalkyl oder Aryl mit jeweils 6 bis 10 Kohlenstoffatomen im Arylteil und gegebenenfalls 1 bis 4 Kohlenstoffatomen im geradkettigen oder verzweigten Alkylteil stehen, oder für einen gegebenenfalls einfach oder mehrfach, gleich oder verschieden substituierten und/oder benzannellierten, gesättigten oder ungesättigten, fünf- bis siebengliedrigen Heterocyclylrest mit 1 bis 3 gleichen oder verschiedenen Heteroatomen - insbesondere Stickstoff, Sauerstoff und/oder Schwefel - stehen, wobei als Aryl- bzw. Heterocyclylsubstituenten jeweils infrage kommen:

Halogen, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Alkoxycarbonyl oder Alkoximinoalkyl mit jeweils 1 bis 6 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen substituiertes Phenyl;

$R^8$ und $R^9$ außerdem gemeinsam mit dem Stickstoffatom, an welches sie gebunden sind, für einen gegebenenfalls einfach oder mehrfach, gleich oder verschieden substituierten und/oder benzannellierten, gesättigten oder ungesättigten, fünf- bis siebengliedrigen Heterocyclylrest mit 1 bis 3 gleichen oder verschiedenen Heteroatomen - insbesondere Stickstoff, Sauerstoff und/oder Schwefel - stehen, wobei als Heterocyclylsubstituenten infrage kommen:

Halogen, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Alkoxycarbonyl oder Alkoximinoalkyl mit jeweils 1 bis 6 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen substituiertes Phenyl;

$R^{10}$ für gegebenenfalls einfach oder mehrfach, gleich oder verschieden substituiertes, geradkettiges oder verzweigtes Alkyl mit 1 bis 14 Kohlenstoffatomen steht, wobei als Substituenten infrage kommen:

Halogen - insbesondere Fluor, Chlor, Brom und/oder Iod -, Cyano, Carboxyl, Carbomoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl oder Alkylsulfonylaminocarbonyl mit jeweils 1 bis 8 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/ oder Schwefel - steht;

$R^{10}$ außerdem für jeweils gegebenenfalls einfach oder mehrfach, gleich oder verschieden durch Halogen - insbesondere Fluor, Chlor, Brom und/oder Iod - substituiertes Alkenyl oder Alkinyl mit jeweils 2 bis 8 Kohlenstoffatomen steht;

$R^{10}$ außerdem für gegebenenfalls einfach oder mehrfach, gleich oder verschieden durch Halogen - insbesondere Fluor, Chlor, Brom und/oder Iod - und/oder geradkettiges oder verzweigtes Alkyl mit 1 bis 4 Kohlenstoffatomen substituiertes Cycloalkyl mit 3 bis 7 Kohlenstoffatomen steht;

$R^{10}$ außerdem für jeweils gegebenenfalls im Arylteil einfach oder mehrfach, gleich oder verschieden substituiertes Arylalkyl oder Aryl mit jeweils 6 bis 10 Kohlenstoffatomen im Arylteil und gegebenenfalls 1 bis 4 Kohlenstoffatomen im geradkettigen oder verzweigten Alkylteil steht, oder für einen gegebenenfalls einfach

oder mehrfach, gleich oder verschieden substituierten und/oder benzannellierten, gesättigten oder ungesättigten, fünf- bis siebengliedrigen Heterocyclylrest mit 1 bis 3 gleichen oder verschiedenen Heteroatomen - insbesondere Stickstoff, Sauerstoff und/oder Schwefel - steht, wobei als Aryl- bzw. Heterocyclylsubstituenten jeweils infrage kommen:

Halogen, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Alkoxycarbonyl oder Alkoximinoalkyl mit jeweils 1 bis 6 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen substituiertes Phenyl;

$R^{11}$ für Wasserstoff steht;

$R^{11}$ außerdem für gegebenenfalls einfach oder mehrfach, gleich oder verschieden substituiertes, geradkettiges oder verzweigtes Alkyl mit 1 bis 14 Kohlenstoffatomen steht, wobei als Substituenten infrage kommen:

Halogen - insbesondere Fluor, Chlor, Brom und/oder Iod -, Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl oder Alkylsulfonylaminocarbonyl mit jeweils 1 bis 8 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/ oder Schwefel - steht;

$R^{11}$ außerdem für jeweils gegebenenfalls einfach oder mehrfach, gleich oder verschieden durch Halogen - insbesondere Fluor, Chlor, Brom und/oder Iod - substituiertes Alkenyl oder Alkinyl mit jeweils 2 bis 8 Kohlenstoffatomen steht;

$R^{11}$ außerdem für gegebenenfalls einfach oder mehrfach, gleich oder verschieden durch Halogen - insbesondere Fluor, Chlor, Brom und/oder Iod - und/oder geradkettiges oder verzweigtes Alkyl mit 1 bis 4 Kohlenstoffatomen substituiertes Cycloalkyl mit 3 bis 7 Kohlenstoffatomen steht;

$R^{11}$ außerdem für jeweils gegebenenfalls im Arylteil einfach oder mehrfach, gleich oder verschieden substituiertes Arylalkyl oder Aryl mit jeweils 6 bis 10 Kohlenstoffatomen im Arylteil und gegebenenfalls 1 bis 4 Kohlenstoffatomen im geradkettigen oder verzweigten Alkylteil steht, wobei als Arylsubstituenten jeweils infrage kommen:

Halogen, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Alkoxycarbonyl oder Alkoximinoalkyl mit jeweils 1 bis 6 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen substituiertes Phenyl;

$R^{12}$ für Wasserstoff steht;

$R^{12}$ außerdem für gegebenenfalls einfach oder mehrfach, gleich oder verschieden substituiertes, geradkettiges oder verzweigtes Alkyl mit 1 bis 14 Kohlenstoffatomen steht, wobei als Substituenten infrage kommen:

Halogen - insbesondere Fluor, Chlor, Brom und/oder Iod -, Cyano, Carboxyl,

Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl oder Alkylsulfonylaminocarbonyl mit jeweils 1 bis 8 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/ oder Schwefel - steht;

$R^{12}$ außerdem für jeweils gegebenenfalls einfach oder mehrfach, gleich oder verschieden durch Halogen - insbesondere Fluor, Chlor, Brom und/oder Iod - substituiertes Alkenyl oder Alkinyl mit jeweils 2 bis 8 Kohlenstoffatomen steht;

$R^{12}$ außerdem für gegebenenfalls einfach oder mehrfach, gleich oder verschieden durch Halogen - insbesondere Fluor, Chlor, Brom und/oder Iod - und/oder geradkettiges oder verzweigtes Alkyl mit 1 bis 4 Kohlenstoffatomen substituiertes Cycloalkyl mit 3 bis 7 Kohlenstoffatomen steht;

$R^{12}$ außerdem für jeweils gegebenenfalls im Arylteil einfach oder mehrfach, gleich oder verschieden substituiertes Arylalkyl oder Aryl mit jeweils 6 bis 10 Kohlenstoffatomen im Arylteil und gegebenenfalls 1 bis 4 Kohlenstoffatomen im geradkettigen oder verzweigten Alkylteil steht, oder für einen gegebenenfalls einfach oder mehrfach, gleich oder verschieden substituierten und/oder benzannellierten, gesättigten oder ungesättigten, fünf- bis siebengliedrigen Heterocyclylrest mit 1 bis 3 gleichen oder verschiedenen Heteroatomen - insbesondere Stickstoff Sauerstoff und/oder Schwefel - steht, wobei als Aryl- bzw. Heterocyclylsubstituenten jeweils infrage kommen:
Halogen, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Alkoxycarbonyl oder Alkoximinoalkyl mit jeweils 1 bis 6 Kohlenstoffatomen in den einzelnen Alkylteilen, sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen substituiertes Phenyl.

**3.** Verfahren zur Herstellung neuer 1-Aryltriazolin(thi)one der allgemeinen Formel (I),

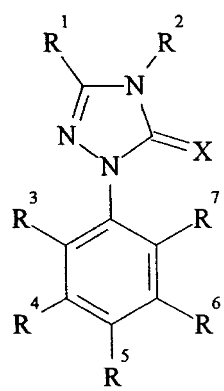

(I)

in welcher

$R^1$ für Nitro oder für einen Rest $-NR^8R^9$ steht,

$R^2$ für Wasserstoff, Amino, Cyano oder für einen der Reste $-R^{10}$, $-O-R^{10}$, $-S-R^{10}$, $-NR^{10}R^{11}$, $-N(R^{11})-CO-R^{10}$ oder $-N=CR^{10}R^{11}$ steht,

$R^3$, $R^6$ und $R^7$ unabhängig voneinander jeweils für Wasserstoff, Halogen oder Nitro stehen,

| | |
|---|---|
| $R^4$ | für Wasserstoff, Halogen, Cyano, Nitro, oder für einen der Reste $-R^{12}$, $-O-R^{12}$, $-S-R^{12}$, $-S(O)-R^{12}$, $-SO_2-R^{12}$, $-SO_2-OR^{12}$, $-SO_2-NR^{11}R^{12}$, $-CO-OR^{12}$, $-CO-NR^{11}R^{12}$, $-O-SO_2-R^{12}$, $-N(R^{11})-SO_2-R^{12}$, $-NR^{11}R^{12}$, $-NH-P(O)(R^{11})(OR^{12})$ oder $-NH-P(O)(OR^{11})-(OR^{12})$ steht, |
| $R^5$ | für Nitro, Cyano, Halogen oder Halogenalkyl steht und |
| X | für Sauerstoff oder Schwefel steht, wobei |
| $R^8$ und $R^9$ | unabhängig voneinander jeweils für Wasserstoff oder für jeweils gegebenenfalls substituiertes Alkyl, Alkenyl, Alkinyl, Alkoxy, Cycloalkyl, Aryl oder Heterocyclyl stehen oder gemeinsam mit dem Stickstoffatom, an welches sie gebunden sind, für einen gegebenenfalls substituierten Heterocyclus stehen, |
| $R^{10}$ | für jeweils gegebenenfalls substituiertes Alkyl, Alkenyl, Alkinyl, Cycloalkyl, Aryl oder Heterocyclyl steht, |
| $R^{11}$ | für Wasserstoff oder für jeweils gegebenenfalls substituiertes Alkyl, Alkenyl, Alkinyl, Cycloalkyl, Arylalkyl oder Aryl steht und |
| $R^{12}$ | für Wasserstoff oder für jeweils gegebenenfalls substituiertes Alkyl, Alkenyl, Alkinyl, Cycloalkyl,Arylalkyl, Aryl oder Heterocyclyl steht, |

dadurch gekennzeichnet, daß man

a) 1H-Triazolinone der Formel (II),

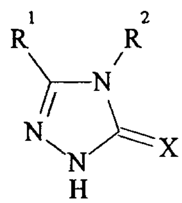

(II)

in welcher

$R^1$, $R^2$ und X die oben angegebenen Bedeutungen haben,

mit Halogenbenzol-Derivaten der Formel (III),

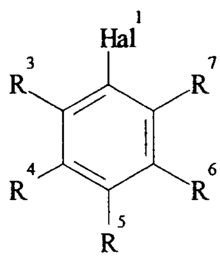

(III)

in welcher

$R^3$, $R^4$, $R^5$, $R^6$ und $R^7$ die oben angegebenen Bedeutungen haben und

$Hal^1$ für Halogen steht,

gegebenenfalls in Gegenwart eines Verdünnungsmittels und gegebenenfalls in Gegenwart eines Reaktionshilfsmittels umsetzt, oder daß man

b) substituierte Triazolinone der Formel (Ia),

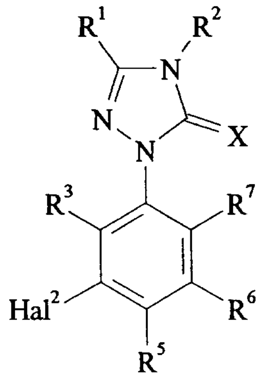

(Ia)

in welcher
$R^1$, $R^2$, $R^3$, $R^5$, $R^6$, $R^7$ und X die oben angegebenen Bedeutungen haben und
$Hal^2$ für Halogen steht,
mit Nukleophilen der Formel (IV),

$H\text{-}R^{13}$ (IV)

in welcher

| | |
|---|---|
| $R^{13}$ | für einen Rest der Formel $-O-R^{12}$, $-S-R^{12}$ oder $-NR^{11}R^{12}$ steht, wobei |
| $R^{11}$ und $R^{12}$ | die oben angegebenen Bedeutungen haben, |

gegebenenfalls in Gegenwart eines Verdünnungsmittels und gegebenenfalls in Gegenwart eines Reaktionshilfsmittels umsetzt, oder daß man
c) substituierte Triazolinone der Formel (Ib),

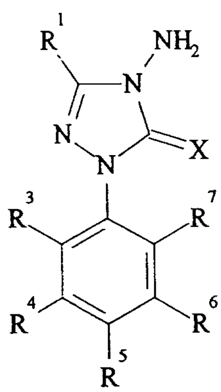

(Ib)

in welcher
$R^1$, $R^3$, $R^4$, $R^5$, $R^6$, $R^7$ und X die oben angegebenen Bedeutungen haben,
mit Natriumnitrit in Gegenwart einer Säure und gegebenenfalls in Gegenwart eines Verdünnungsmittels umsetzt, oder daß man
d) substituierte Triazolinone der Formel (Ic),

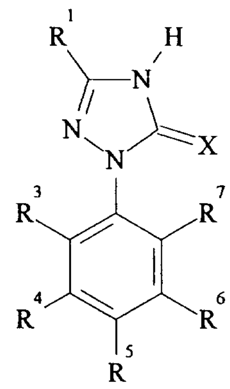

(Ic)

in welcher
$R^1$, $R^3$, $R^4$, $R^5$, $R^6$, $R^7$ und X die oben angegebenen Bedeutungen haben,
mit Alkylierungsmitteln der Formel (V),

$R^{2-1}$-$E^1$ (V)

in welcher

$R^{2-1}$ für jeweils gegebenenfalls substituiertes Alkyl, Alkenyl, Alkinyl oder Cycloalkyl steht und

$E^1$ für eine elektronenanziehende Abgangsgruppe steht,

gegebenenfalls in Gegenwart eines Verdünnungsmittels und gegebenenfalls in Gegenwart eines Reaktionshilfsmittels umsetzt, oder daß man

d) Chlor-formamidinium-Hydrochloride der Formel (VI),

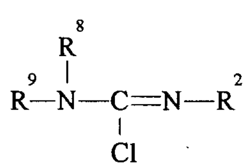 x HCl (VI)

in welcher
$R^2$, $R^8$ und $R^9$ die oben angegebenen Bedeutungen haben,
zunächst in einer ersten Stufe mit Arylhydrazinen der Formel (VII),

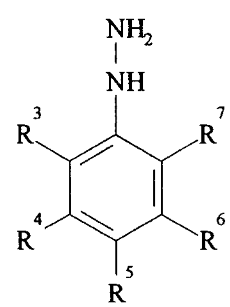

(VII)

in welcher
$R^3$, $R^4$, $R^5$, $R^6$ und $R^7$ die oben angegebenen Bedeutungen haben,
gegebenenfalls in Gegenwart eines Verdünnungsmittels umsetzt und in einer anschließenden zweiten Stufe die so erhältlichen Zwischenprodukte der Formel (VIII),

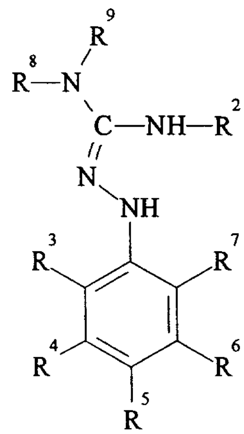

(VIII)

in welcher
$R^2$, $R^3$, $R^4$, $R^5$, $R^6$, $R^7$, $R^8$ und $R^9$ die oben angegebenen Bedeutungen haben,
mit Phosgen oder Thiophosgen gegebenenfalls in Gegenwart eines Verdünnungsmittels und gegebenenfalls in Gegenwart eines Reaktionshilfsmittels cyclisiert.

**4.** Herbizide Mittel, gekennzeichnet durch einen Gehalt an mindestens einem 1-Aryltriazolin(thi)on der Formel (I) gemäß den Ansprüchen 1 bis 3.

**5.** Verfahren zur Bekämpfung von unerwünschten Pflanzen, dadurch gekennzeichnet, daß man 1-Aryltriazolin(thi)one der allgemeinen Formel (I) gemäß den Ansprüchen 1 bis 3 auf die Pflanzen und/oder ihren Lebensraum einwirken läßt.

**6.** Verwendung von 1-Aryltriazolin(thi)onen der allgemeinen Formel (I) gemäß der Ansprüche 1 bis 3 zur Bekämpfung von unerwünschten Pflanzen.

**7.** Verfahren zur Herstellung von herbiziden Mitteln, dadurch gekennzeichnet, daß man 1-Aryltriazolin(thi)one der allgemeinen Formel (I) gemäß der Ansprüche 1 bis 3 mit Streckmitteln und/oder oberflächenaktiven Substanzen vermischt.

**8.** Substituierte Triazolinone der Formel (Ia)

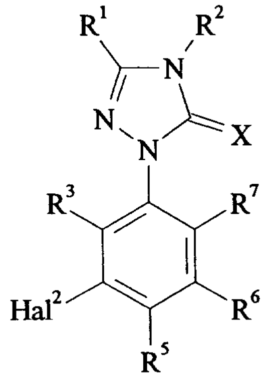

(Ia)

dadurch gekennzeichnet, daß

| | |
|---|---|
| $R^1$ | für Nitro oder für einen Rest $-NR^8R^9$ steht, |
| $R^2$ | für Wasserstoff, Amino, Cyano oder für einen der Reste $-R^{10}$, $-O-R^{10}$, $-S-R^{10}$, $-NR^{10}R^{11}$, $-N(R^{11})-CO-R^{10}$ oder $-N=CR^{10}R^{11}$ steht, |
| $R^3$, $R^6$ und $R^7$ | unabhängig voneinander jeweils für Wasserstoff, Halogen oder Nitro stehen, |
| $R^5$ | für Nitro, Cyano, Halogen oder Halogenalkyl steht und |
| X | für Sauerstoff oder Schwefel steht, wobei |

| | |
|---|---|
| $R^8$ und $R^9$ | unabhängig voneinander jeweils für Wasserstoff oder für jeweils gegebenenfalls substituiertes Alkyl Alkenyl, Alkinyl, Alkoxy, Cycloalkyl, Aryl oder Heterocyclyl stehen oder gemeinsam mit dem Stickstoffatom, an welches sie gebunden sind, für einen gegebenenfalls substituierten Heterocyclus stehen, |
| $R^{10}$ | für jeweils gegebenenfalls substituiertes Alkyl, Alkenyl, Alkinyl, Cycloalkyl, Aryl, Arylalkyl oder Heterocyclyl steht, |
| $R^{11}$ | für Wasserstoff oder für jeweils gegebenenfalls substituiertes Alkyl, Alkenyl, Alkinyl, Cycloalkyl, Arylalkyl oder Aryl steht und |
| $Hal^2$ | für Halogen steht. |

**9.** Substituierte Triazolinone der allgemeinen Formel (Ib)

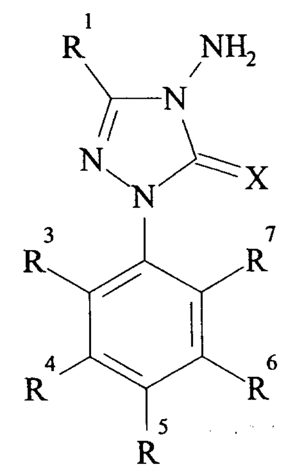

(I b)

dadurch gekennzeichnet, daß

| | |
|---|---|
| $R^1$ | für Nitro oder für einen Rest $-NR^8R^9$ steht, |
| $R^3$, $R^6$ und $R^7$ | unabhängig voneinander jeweils für Wasserstoff, Halogen oder Nitro stehen, |
| $R^4$ | für Wasserstoff, Halogen, Cyano, Nitro, oder für einen der Reste $-R^{12}$, $-O-R^{12}$, $-S-R^{12}$, $-S(O)-R^{12}$, $-SO_2-R^{12}$, $-SO_2-OR^{12}$, $-SO_2-NR^{11}R^{12}$, $-CO-OR^{12}$, $-CO-NR^{11}R^{12}$, $-O-SO_2-R^{12}$, $-N(R^{11})-SO_2-R^{12}$, $-NR^{11}R^{12}$, $-NH-P(O)(R^{11})(OR^{12})$ oder $-NH-P(O)(OR^{11})-(OR^{12})$ steht, |
| $R^5$ | für Nitro, Cyano, Halogen oder Halogenalkyl steht und |
| X | für Sauerstoff oder Schwefel steht, wobei |
| $R^8$ und $R^9$ | unabhängig voneinander jeweils für Wasserstoff oder für jeweils gegebenenfalls substituiertes Alkyl, Alkenyl, Alkinyl, Alkoxy, Cycloalkyl, Aryl oder Heterocyclyl stehen oder gemeinsam mit dem Stickstoffatom, an welches sie gebunden sind, für einen gegebenenfalls substituierten Heterocyclus stehen, |
| $R^{11}$ | für Wasserstoff oder für jeweils gegebenenfalls substituiertes Alkyl, Alkenyl, Alkinyl, Cycloalkyl, Arylalkyl oder Aryl steht und |
| $R^{12}$ | für Wasserstoff oder für jeweils gegebenenfalls substituiertes Alkyl, Alkenyl, Alkinyl, Cycloalkyl, Aryl oder Heterocyclyl steht. |

**10.** Substituierte Triazolinone der Formel (Ic)

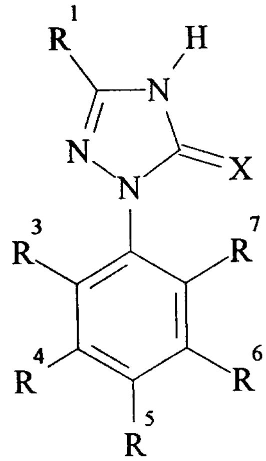

(I c)

dadurch gekennzeichnet, daß

| | |
|---|---|
| $R^1$ | für Nitro oder für einen Rest $-NR^8R^9$ steht, |
| $R^3$, $R^6$ und $R^7$ | unabhängig voneinander jeweils für Wasserstoff Halogen oder Nitro stehen, |
| $R^4$ | für Wasserstoff, Halogen, Cyano, Nitro, oder für einen der Reste $-R^{12}$, $-O-R^{12}$, $-S-R^{12}$, $-S(O)-R^{12}$, $-SO_2-R^{12}$, $-SO_2-OR^{12}$, $-SO_2-NR^{11}R^{12}$, $-CO-OR^{12}$, $-CO-NR^{11}R^{12}$, $-O-SO_2-R^{12}$, $-N(R^{11})-SO_2-R^{12}$, $-NR^{11}R^{12}$, $-NH-P(O)(R^{11})(OR^{12})$ oder $-NH-P(O)(OR^{11})-(OR^{12})$ steht, |
| $R^5$ | für Nitro, Cyano, Halogen oder Halogenalkyl steht und |
| X | für Sauerstoff oder Schwefel steht, wobei |
| $R^8$ und $R^9$ | unabhängig voneinander jeweils für Wasserstoff oder für jeweils gegebenenfalls substituiertes Alkyl, Alkenyl, Alkinyl, Alkoxy, Cycloalkyl, Aryl oder Heterocyclyl stehen oder gemeinsam mit dem Stickstoffatom, an welches sie gebunden sind, für einen gegebenenfalls substituierten Heterocyclus stehen, |
| $R^{11}$ | für Wasserstoff oder für jeweils gegebenenfalls substituiertes Alkyl, Alkenyl, Alkinyl, Cycloalkyl, Arylalkyl oder Aryl steht und |
| $R^{12}$ | für Wasserstoff oder für jeweils gegebenenfalls substituiertes Alkyl, Alkenyl, Alkinyl, Cycloalkyl, Aryl oder Heterocyclyl steht. |

Europäisches Patentamt

# EUROPÄISCHER RECHERCHENBERICHT

Nummer der Anmeldung
EP 94 10 1222

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| X | DE-A-29 10 330 (HOECHST AG) 2. Oktober 1980<br>*siehe Seite 6 und Beispiele 1b und 1j*<br>--- | 1-3,10 | C07D249/14<br>C07D401/12<br>C07D403/12<br>A01N43/653 |
| X | J. HETEROCYCLIC CHEM.<br>Bd. 27 , 1990<br>Seiten 575 - 577<br>M.D.COBURN AND K.LEE 'Picryl derivatives of 5-Nitro-2,4-dihydro-3H-1,2,4-triazol-3-one.'<br>*siehe 2 und 4, Seite 576*<br>--- | 1-3,10 | |
| X | CHEM. BER.<br>Bd. 102 , 1969<br>Seiten 755 - 766<br>C.KRÖGER ET AL 'Die Nitrierung und Bromierung von 1,2,4-Triazolonen'<br>*siehe Verbindung Nr 3a*<br>--- | 1,2,10 | |
| D,Y | DE-A-38 39 480 (BAYER AG) 31. Mai 1990<br>* das ganze Dokument *<br>--- | 1-10 | RECHERCHIERTE SACHGEBIETE (Int.Cl.5) |
| Y | US-A-4 906 284 (G. THEODORIDIS) 6. März 1990<br>*Siehe Spalte 4, Zeilen 17-21 und Table 1, Spalte 7, Verbindung Nr 15 und Zeilen 58-65*<br>--- | 1-10 | C07D<br>A01N |
| Y | WO-A-86 04481 (FMC CORPORATION) 14. August 1986<br>* das ganze Dokument *<br>--- | 1-10 | |
| Y | EP-A-0 055 105 (SUMITOMO CHEMICAL COMPANY LIMITED) 30. Juni 1982<br>* das ganze Dokument *<br>----- | 1-10 | |

Der vorliegende Recherchenbericht wurde für alle Patentansprüche erstellt

| Recherchenort | Abschlußdatum der Recherche | Prüfer |
|---|---|---|
| MÜNCHEN | 16. Mai 1994 | Scruton-Evans, I |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
.......................................................
& : Mitglied der gleichen Patentfamilie, übereinstimmendes Dokument

EPO FORM 1503 03.82 (P04C03)